# EUROPEAN PATENT APPLICATION

(11) **EP 4 696 240 A1**
(43) Date of publication of application: **18.02.2026**
(21) Application number: 24788824.1
(22) Date of filing: 11.04.2024
(51) Int. Cl.: A61B 6/00, A61B 6/50

(54) **DIAGNOSIS ASSISTANCE PROGRAM AND DIAGNOSIS ASSISTANCE SYSTEM**

(30) Priority: 11.04.2023 JP 2023064526
(71) Applicant: Radwisp Pte. Ltd., The Central 059819 (SG); Mediott Co., Ltd., Tokyo 162-0803 (JP); Abe, Takehiko, The Central Singapore 059819 (SG)
(72) Inventor: YOSHIDA Norifumi, Singapore 059819 (SG)
(74) Representative: SSM Sandmair
(86) International application number: PCT/JP2024/014754
(87) International publication number: WO 2024/214802

(57) **Abstract**

[Problem] To analyze a dynamic image of a living body and display an analysis result.

[Solution] A diagnosis assistance program for analyzing an image of a living body and displaying an analysis result, the diagnosis assistance program causing a computer to execute a process of acquiring a plurality of frame images, a process of acquiring a periodic image signal changing at a fixed period from the plurality of frame images, a process of extracting the waveform of all or a part of the periodic image signal, and a process of collating the extracted waveform with a model waveform acquired in advance and detecting a correlation therebetween. Further included is a process of complementing discrete periodic image signals to form a continuous waveform. Further, the correlation is detected based on at least one of the phase, the amplitude, or the frequency of the waveform of all or a part of the periodic image signal.

## Description

### TECHNICAL FIELD

The present invention relates to a technique for analyzing images of a human body and displaying an analysis result.

### BACKGROUND ART

When a physician performs a pulmonary diagnosis based on dynamic images of a chest, observation of a time-series dynamic chest image captured while a subject is in a natural breathing state is important. A spirometer, which facilitates acquisition of physiological data, an RI (Radio Isotope) examination, a plain X-ray image from which morphological data may be obtained, and a CT (Computed Tomography) scan are known as methods for evaluating pulmonary function. However, acquiring both physiological data and morphological data efficiently is not easy.

In recent years, methods for capturing dynamic images of a human chest using a semiconductor image sensor such as an FPD (Flat Panel Detector) have been attempted and used for diagnosis. For example, Non-Patent Document 1 ("Basic Imaging Properties of a Large Image Intensifier-TV Digital Chest Radiographic System" Investigative Radiology: April 1987; 22: 328-335) discloses a technique for generating a difference image indicating a difference in signal values between a plurality of frame images constituting a dynamic image, determining a maximum value of each signal value from the difference image, and displaying the maximum value.

Moreover, Patent Document 1 (Japanese Patent Publication No. 5874636) discloses a technique in which a lung field region is extracted from each frame image among a plurality of frame images indicating a dynamic state of a human chest, the lung field region is divided into a plurality of small regions, and the divided small regions are associated with one another and analyzed among the plurality of frame images. According to this technique, a feature amount indicating movement of the divided small regions is displayed.

### PRIOR ART

### PATENT DOCUMENT

[Patent Document 1] Japanese Patent Publication No. 5874636

### NON-PATENT DOCUMENT

[Non-Patent Document 1] "Basic Imaging Properties of a Large Image Intensifier-TV Digital Chest Radiographic System" Investigative Radiology: April 1987; 22: 328-335

### SUMMARY OF THE INVENTION

### PROBLEM TO BE SOLVED

However, as in the technique described in Non-Patent Document 1, merely displaying a maximum inter-frame difference value for each pixel of a dynamic image does not allow a physician to easily grasp a pathological condition. Moreover, as in the technique described in Patent Document 1, merely displaying a feature amount is still not sufficient for grasping the pathological condition. In particular, conventionally, respiration and blood pressure were measured after a subject was placed in a resting state, and therefore measurement may have been performed only under this condition; however, acquiring biological information such as respiration and blood pressure under a dynamic condition is desirable. That is, it is desirable that an entire dynamic respiratory state and vascular dynamics of a human body being a subject are grasped, and an image indicating an actual movement is displayed based on a waveform or a frequency, or a waveform and a frequency, of respiration, heart, or blood vessels or blood flow in a pulmonary hilum region, or based on a change tendency of an image.

The present invention has been made in view of such circumstances and aims to provide a diagnosis assistance program capable of analyzing an image of a human body and displaying an analysis result. More specifically, the present invention aims to calculate a numerical value useful for diagnosis by specifying a waveform of newly measured data of a dynamic target or by quantifying a degree of match and other degrees of mismatch with respect to an already acquired waveform and frequency (Hz), and to generate an image useful for diagnosis by visualizing these numerical values.

### MEANS FOR SOLVING THE PROBLEM

(1) In order to achieve the above object, the present application has taken the following means. That is, according to an aspect of the present disclosure, a diagnosis assistance program for analyzing an image of a living body and displaying an analysis result is configured to cause a computer to execute a process for acquiring a plurality of frame images, a process for acquiring a periodic image signal that varies at a predetermined cycle from the plurality of frame images, a process for extracting all or a part of a waveform of the periodic image signal, and a process for matching the extracted waveform with a model waveform acquired in advance and detecting a correlation therebetween.
(2) Optionally, the diagnosis assistance program according to the present disclosure may further include a process for complementing the periodic image signal, which is discrete, and forming a continuous waveform.
(3) Optionally, in the diagnosis assistance program according to the present disclosure, the correlation may be detected based on at least one of a phase, an amplitude, or a frequency of all or a part of the waveform of the periodic image signal.
(4) Optionally, the diagnosis assistance program according to the present disclosure may be configured to cause the computer to execute a process for visualizing the extracted waveform based on the detected correlation.
(5) According to another aspect of the present disclosure, a diagnosis assistance program for analyzing an image of a living body and displaying an analysis result is configured to cause a computer to execute a process for acquiring a plurality of frame images, a process for acquiring a periodic image signal that varies at a predetermined cycle from the plurality of frame images, a process for extracting all or a part of a frequency of the periodic image signal, and a process for matching the extracted frequency with a model frequency acquired in advance and detecting a correlation therebetween.
(6) Optionally, the diagnosis assistance program according to the present disclosure may further include a process for identifying a waveform based on a phase, an amplitude, and the frequency of all or a part of the periodic image signal.
(7) Optionally, the diagnosis assistance program according to the present disclosure may be configured to cause the computer to execute a process for visualizing the identified waveform.
(8) According to another aspect of the present disclosure, a diagnosis assistance program for analyzing an image of a living body and displaying an analysis result is configured to cause a computer to execute a process for acquiring a plurality of frame images, a process for acquiring a periodic image signal that varies at a predetermined cycle from the plurality of frame images, and a process for creating a model by training an AI (Artificial Intelligence) to learn all or a part of the periodic image signal.
(9) Optionally, the diagnosis assistance program according to the present disclosure may further include a process for detecting a correlation between the model and the periodic image signal acquired from the plurality of frame images.
(10) Optionally, the diagnosis assistance program according to the present disclosure may further include a process for extracting a signal having a maximum amplitude among the extracted waveform, and the signal having the maximum amplitude may be visualized based on the detected correlation.
(11) Optionally, the diagnosis assistance program according to the present disclosure may further include a process for applying logarithmic transformation to the periodic image signal acquired from the plurality of frame images, and each of the processes may be executed with the periodic image signal after the logarithmic transformation.
(12) Optionally, the diagnosis assistance program according to the present disclosure may further include a process for applying Fourier transformation to the periodic image signal, a process for extracting a signal having the correlation from the signal after the Fourier transformation, and a process for applying inverse Fourier transformation to the extracted signal.
(13) Optionally, the diagnosis assistance program according to the present disclosure may further include a process for interpolating the waveform using a waveform representing a movement of a standard model created by training an AI (Artificial Intelligence) to learn an image of a standard organ.
(14) Optionally, the diagnosis assistance program according to the present disclosure may further include a process, using a frequency of a movement of a standard model created by training an AI (Artificial Intelligence) to learn an image of a standard organ, for extracting pixels, of which pixel values vary over time according to the frequency of the movement of the standard model.
(15) According to another aspect of the present disclosure, a diagnosis assistance program for analyzing an image of a living body and displaying an analysis result is configured to cause a computer to execute a process for acquiring a video composed of a plurality of frame images, a process for creating a model by training an AI (Artificial Intelligence) to learn the video, and a process for predicting a measurement value for the living body using the model.
(16) According to another aspect of the present disclosure, a diagnosis assistance program for analyzing an image of a living body and displaying an analysis result is configured to cause a computer to execute a process for acquiring a video composed of a plurality of frame images, a process for acquiring a periodic image signal that varies at a predetermined cycle from the video, a process for creating a model by training an AI (Artificial Intelligence) to learn the periodic image signal, and a process for predicting a phenomenon occurring in the living body using the model.
(17) According to another aspect of the present disclosure, a diagnosis assistance system configured to analyze an image of a living body and display an analysis result includes an input interface configured to acquire a plurality of frame images; and a basic module configured to acquire a periodic signal image that varies at a predetermined cycle from the plurality of frame images, extract all or a part of a waveform of the periodic image signal, and match the extracted waveform with a model waveform acquired in advance and detect a correlation therebetween.

### EFFECTS OF THE INVENTION

According to one aspect of the present invention, an entire respiratory state and vascular dynamics of a human body being a subject may be grasped, and an image indicating an actual movement may be displayed based on a waveform or a frequency, or a waveform and a frequency, of respiration, heart, or blood vessels or blood flow in a pulmonary hilum region, or based on a change tendency of an image.

### BRIEF DESCRIPTION OF THE DRAWINGS

[FIG. 1A] FIG. 1A is a diagram showing an outline configuration of a diagnosis assistance system according to an embodiment.
[FIG. 1B] FIG. 1B is a diagram showing an example of a method for dividing a lung region.
[FIG. 1C] FIG. 1C is a diagram showing how a morphology of a lung changes with lapse of time.
[FIG. 1D] FIG. 1D is a diagram showing how a morphology of a lung changes with lapse of time.
[FIG. 2A] FIG. 2A is a diagram showing an "intensity" change of a specific block and a result of Fourier analysis thereof.
[FIG. 2B] FIG. 2B is a diagram showing a Fourier transform result with a frequency component close to a heartbeat extracted, and an "intensity" change of the frequency component close to the heartbeat obtained by inverse Fourier transformation thereof.
[FIG. 2C] FIG. 2C is a diagram showing an example of extracting a certain band among spectra obtained after Fourier transformation.
[FIG. 2D] FIG. 2D is a diagram schematically representing a rate of change of lungs.
[FIG. 2E] FIG. 2E is a diagram showing an example of a pattern image of a lung field region.
[FIG. 2F] FIG. 2F is a diagram showing an example of a pattern image of a lung field region.
[FIG. 2G] FIG. 2G is a diagram showing an example of a pattern image of a lung field region.
[FIG. 2H] FIG. 2H is a diagram showing an example of a pattern image of a lung field region.
[FIG. 3A] FIG. 3A is a diagram showing an example in which a contour of a lung field is drawn using both a Bezier curve and a straight line, showing a state where the lung field is maximum.
[FIG. 3B] FIG. 3B is a diagram showing an example in which a contour of a lung field is drawn using both a Bezier curve and a straight line, showing a state where the lung field is minimum.
[FIG. 4A] FIG. 4A is a diagram in which front and back images of a lung field are superimposed between a previous frame and a next frame.
[FIG. 4B] FIG. 4B is a diagram showing a state in which a "strong-gap line" occurs as a result of taking a difference between two original images in FIG. 4A.
[FIG. 4C] FIG. 4C is a diagram showing a difference value of a total "density" of "intensity" values at respective positions in an up-down direction of an image in FIG. 4B.
[FIG. 5] FIG. 5 is a diagram showing a result of performing curve regression and approximating a relative position of a diaphragm.
[FIG. 6A] FIG. 6A is a flowchart showing an outline of respiratory function analysis according to the embodiment.
[FIG. 6B] FIG. 6B is a diagram showing an example of an image displayed on a display.
[FIG. 6C] FIG. 6C is a diagram showing an example of an image displayed on a display.
[FIG. 7] FIG. 7 is a flowchart showing an outline of pulmonary blood flow analysis according to the embodiment.
[FIG. 8] FIG. 8 is a flowchart showing an outline of other blood flow analysis according to the embodiment.
[FIG. 9] FIG. 9 is a diagram showing an example of multiplying a coefficient by a certain spectrum among spectra obtained after Fourier transformation.
[FIG. 10] FIG. 10 is a diagram showing an example in which a lung field is drawn using a Bezier curve.
[FIG. 11] FIG. 11 is a diagram showing an example in which a lung field is divided using a Bezier curve.
[FIG. 12] FIG. 12 is a diagram showing an example in which a lung field is divided using a Bezier curve.
[FIG. 13A] FIG. 13 is a diagram showing an example in which a waveform of aortic blood flow rate is compared with a waveform of ventricular volume.
[FIG. 13B] FIG. 13B is a diagram showing a state in which a waveform of a respiratory element is separated from a waveform of left atrial pressure obtained by a catheter examination to obtain an original waveform of the left atrial pressure.
[FIG. 13C] FIG. 13C is a diagram showing a state in which a waveform of a respiratory fluctuation component is separated from electrocardiographic data obtained by measurement to obtain an original waveform of an electrocardiogram.
[FIG. 13D] FIG. 13D is a diagram showing a waveform of an electrocardiogram and a waveform of respiration.
[FIG. 13E] FIG. 13E is a diagram showing a concept of "Wave form tunable imaging."
[FIG. 13F] FIG. 13F is a diagram showing a state in which pixel values by their own are plotted on a graph.
[FIG. 13G] FIG. 13G is a diagram in which dots indicating the pixel values are connected.
[FIG. 13H] FIG. 13H is a diagram in which a waveform is drawn based on a distribution of dots indicating pixel values.
[FIG. 13I] FIG. 13I is a diagram showing a state in which dots of an identical or article phase within one cycle are collected.
[FIG. 13J] FIG. 13J is a diagram showing a state in which a certain width is provided from an assumed waveform and dots within the width range are included in the waveform.
[FIG. 13K] FIG. 13K is a diagram showing a distribution of the pixel values.
[FIG. 13L] FIG. 13L is a diagram showing a distribution of the pixel values.
[FIG. 13M] FIG. 13M is a graph showing a state of inflow in a main pulmonary artery (MPA).
[FIG. 13N] FIG. 13N is a diagram showing a waveform acquired in a single heartbeat of a heart.
[FIG. 14] FIG. 14 is a diagram showing an example of pixel values of a lung and near the lung.
[FIG. 15] FIG. 15 is a diagram schematically illustrating a schematic configuration of blood vessels of a human body.
[FIG. 16] FIG. 16 is a diagram showing an example of characteristics of a PTE patient.
[FIG. 17] FIG. 17 is a diagram showing an example of characteristics of a PTE patient.
[FIG. 18] FIG. 18 is a diagram showing an example of a low-resolution difference image.
[FIG. 19] FIG. 19 is a diagram showing an example of a high-resolution difference image.
[FIG. 20] FIG. 20 is a diagram showing a change in pulmonary blood flow within a single heartbeat in normal lungs.
[FIG. 21] FIG. 21 is a diagram showing a change in pulmonary blood flow within a single heartbeat in abnormal lungs.
[FIG. 22] FIG. 22 is a diagram showing normal lungs and lungs with PAH (pulmonary arterial hypertension (including pulmonary arteriosclerosis)).
[FIG. 23] FIG. 23 is a diagram showing example images of MBDP (Maximum Blood-flow-related Differential Projection) and ABDP (Accumulated Blood-flow-related Differential Projection).
[FIG. 24] FIG. 24 is a diagram representing pulmonary blood flow for each frame within a single heartbeat of a heart.
[FIG. 25A] FIG. 25A is a diagram showing example measurement results of an MPA (Main Pulmonary Artery), a DPA (Distal Pulmonary Artery), and a DPV (Distal Pulmonary Vein).
[FIG. 25B] FIG. 25B is a diagram showing processing of a waveform.
[FIG. 26] FIG. 26 is a diagram showing a signal intensity in an ultrasonic image of an atrium.
[FIG. 27] FIG. 27 is a diagram showing a signal intensity in an ultrasonic image of an atrium.
[FIG. 28] FIG. 28 is a diagram showing an image of normal lungs.
[FIG. 29] FIG. 29 is a diagram displaying a normal lung and a lung with COPD.
[FIG. 30A] FIG. 30A is a diagram showing a three-dimensional structure at an apex-diaphragm side.
[FIG. 30B] FIG. 30B is a diagram showing positions of subapical areas.
[FIG. 30C] FIG. 30C is a diagram showing positions of outer layers.
[FIG. 30D] FIG. 30D is a diagram showing positions of subapical areas.
[FIG. 31] FIG. 31 is a diagram showing a state of a peripheral lung field.
[FIG. 32] FIG. 32 is a diagram showing shallow respiration and deep respiration of a normal lung.
[FIG. 33] FIG. 33 is a diagram showing a movement of a respiratory wave.
[FIG. 34] FIG. 34 is a diagram showing a three-dimensional structure and a cross-section based on a chest XP image.
[FIG. 35] FIG. 35 is a diagram representing normal lungs and lungs of a patient.
[FIG. 36] FIG. 36 is a diagram showing lungs of a patient.
[FIG. 37] FIG. 37 is a diagram showing lungs of a patient.
[FIG. 38] FIG. 38 is a diagram showing a contrast between deep respiration and shallow respiration.
[FIG. 39] FIG. 39 is a diagram showing waves of a diaphragm and the like being shifted relative to an original wave.
[FIG. 40] FIG. 40 is a diagram showing an image of a pneumonia patient.
[FIG. 41] FIG. 41 is a diagram showing an example of an electrocardiogram.
[FIG. 42] FIG. 42 is a diagram showing lungs of a patient with heart failure.
[FIG. 43] FIG. 43 is a diagram showing lungs of a patient with heart failure.
[FIG. 44] FIG. 44 is a diagram in which an analysis range is divided into four in an image of each lung.
[FIG. 45] FIG. 45 is a diagram showing a waveform of a lung.
[FIG. 46] FIG. 46 is a diagram showing a waveform of a lung.
[FIG. 47] FIG. 47 is a diagram showing a phase shift of a waveform of a lung.
[FIG. 48] FIG. 48 is a diagram showing a phase shift of a waveform of a lung.
[FIG. 49] FIG. 49 is a diagram showing a graph indicating a change of density in a static image of a lung and a dynamic image of the lung.
[FIG. 50A] FIG. 50A is a diagram showing a state in which each lung field region is divided into a plurality of regions.
[FIG. 50B] FIG. 50B is a diagram showing a variation range of a signal value relating to a movement of lungs.
[FIG. 51A] FIG. 51A is a diagram showing a state in which a region of pulmonary blood flow is divided.
[FIG. 51B] FIG. 51B is a diagram showing an outline of a waveform of a signal value of pulmonary blood flow.
[FIG. 52A] FIG. 52A is a diagram showing a state of pulmonary blood flow of a normal person (a healthy subject).
[FIG. 52B] FIG. 52B is a diagram showing an outline of a waveform of a signal value of pulmonary blood flow.
[FIG. 53A] FIG. 53A is a diagram showing a state of pulmonary blood flow of a patient with heart failure.
[FIG. 53B] FIG. 53B is a diagram showing an outline of a waveform of a signal value of pulmonary blood flow.
[FIG. 54A] FIG. 54A is a diagram showing an example of a waveform of aortic stenosis.
[FIG. 54B] FIG. 54B is a diagram showing an example of a waveform of mitral regurgitation.
[FIG. 54C] FIG. 54C is a diagram showing an example of a waveform of mitral regurgitation.
[FIG. 54D] FIG. 54D is a diagram showing an example of a waveform in aortic regurgitation.
[FIG. 55A] FIG. 55A is a diagram showing an outline of BPP (Blood flow-related Peak Projection).
[FIG. 55B] FIG. 55B is a diagram showing an image of BDP (Blood flow-related Differential Projection) and an image of BPP (Blood flow-related Peak Projection).
[FIG. 56A] FIG. 56A is a diagram showing sites considered to be defects in an image of lungs.
[FIG. 56B] FIG. 56B is a diagram showing an example in which the defect sites are explicitly indicated in the image of the lungs.
[FIG. 57A] FIG. 57A is a diagram schematically showing an outline of BPTP.
[FIG. 57B] FIG. 57B is a diagram schematically showing a mapping example of BPTP.
[FIG. 57C] FIG. 57C is a diagram schematically showing a mapping example of BPTP.

### DESCRIPTION OF THE EMBODIMENTS

First, basic concepts of the present invention will be described. In the present invention, for movements in human body that are perceived as repeating at a regular cycle, such as respiration and other biological movements in blood vessels, an area and a volume of a lung field, a certain repetition or a certain routine on a time axis for an entire range or for a partial range is regarded as a wave and is measured. A measurement result of the wave is represented in either (a) wave form itself or (b) an interval of the wave (frequency: Hz). Optionally, it may be represented in (c) both the wave form and the frequency of the wave may be used (even when frequencies of two waves are identical, waveforms may differ). These concepts are collectively referred to as "base data."

There may be waves that are linked in the same way at the same time.
For example, in a case of respiration, the following approximation may be understood: (an average of "density" change in a certain rough range) ≈ (a change in rib cage) ≈ (a diaphragm movement) ≈ (a pulmonary function test) ≈ (a thoracic and abdominal respiratory sensor).

For the above item "(a) wave form itself," a concept of "waveform tunability" is used, and an image is displayed based on this waveform tunability ("wave form tunable imaging"). For the above item "(b) interval of the wave (frequency: Hz)," a concept of "frequency tunability" is used, and an image is displayed based on frequency tunability ("frequency tunable imaging"). These concepts may be generalized as "biological waveform tunability" and "biological frequency tunability," respectively, and may be displayed as "biological waveform-tunability image" and "biological frequency-tunability image." That is, these "images of living body" may be applied to "data of living body (such as electrocardiogram)." Namely, information may overlap on a waveform itself acquired from a living body. For example, because a waveform obtained from an electrocardiogram reflects a movement of a ventricle and a movement of an atrium, a disease may be predicted from the movement (such as fibrillation).

Further, "density" at respective locations of a human body (inside and outside a heart, blood vessels (an upstream and a supply portion, including downstream, a pulmonary hilum, peripheral pulmonary vessels, and the like)) may be acquired in units of "phase," and a waveform may be obtained by superimposition according to real time or according to a ratio of the "phase" (a % axis). From each "density," including a height (likely attributable to an amplitude) and a reach length, a change of the "density," and from relational changes of "density" of surrounding organs, a method may be adopted to estimate functional information of respective organs. For example, by a reach-time phase from a pulmonary hilum to a periphery, a peripheral pulmonary pressure and a biodynamic quantity for each phase may be measured.

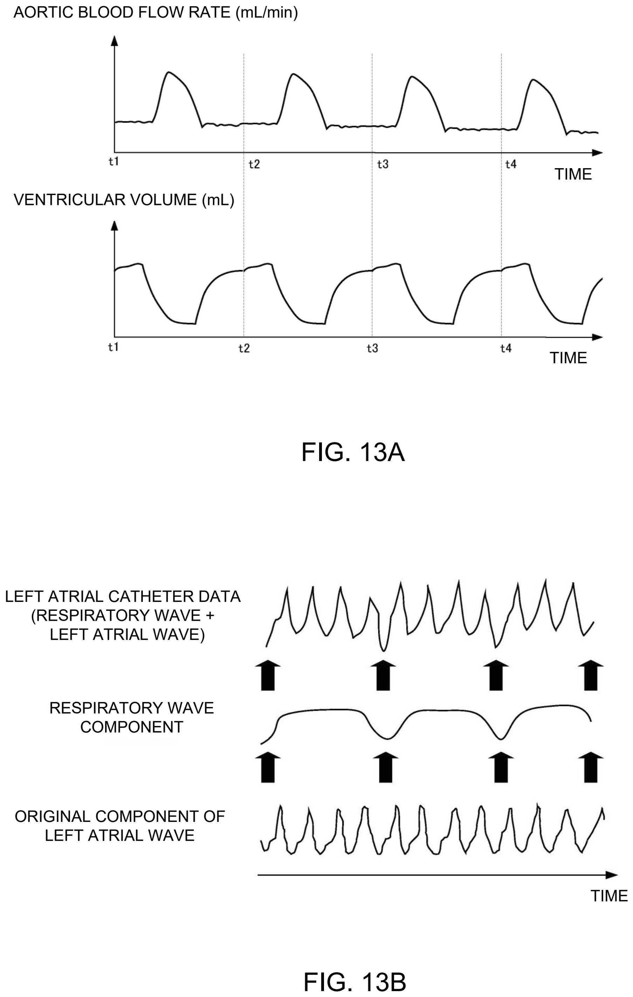

For example, in a heart, as in "an example comparing a waveform of aortic blood flow rate with a waveform of ventricular volume" shown in FIG. 13A, a peak of aortic blood flow rate does not match a peak or a waveform of ventricular volume. However, in FIG. 13A, when an equal temporal width such as from time t1 to t2, from time t2 to t3, from time t3 to t4, and so on is defined as one cycle, one cycle of aortic blood flow rate and one cycle of ventricular volume are repeated many times, and it may be considered that frequencies of respective waveforms are tuned. Focusing on this waveform, one cycle may be identified from measurement values as shown in FIG. 13A, and a shape of a wave (Wave form) may be predicted by using a model waveform. That is, as methods of creating "a waveform as base data," a waveform may be measured, may be generated from a frequency (cycle), may be created by using a model waveform, or may be created by averaging waveforms among individuals. If a cycle (period) of an organ having a frequency, such as a heart, is known, a shape of a wave (Wave form) may be predicted; therefore, waveforms such as aortic blood flow rate, ventricular volume, aortic blood flow velocity, and ventricular velocity may be grasped, and a dynamic image of the organ may be displayed based on this waveform.

Note that, when acquiring changes of "density" such as respiration, heart, and pulmonary hilum, a digital filter may be applied in advance so that other elements do not mix.

Further, it has been known that there is a correlation between respiration and blood flow, and that when they lose the correlation, some abnormality may be predicted. That is, these correlations are understood pathologically and ecologically; however, conventionally, verification has not been enabled for each patient in real time. For example, in electromyography, even if the same frequency (Frequency) is detected, correlations among a plurality of waveforms have not been verified. When a certain waveform appears, with the concept of "Wave Form Tunable," a frequency (Frequency) may be detected. Further, even if a waveform (Wave form) is grasped, the frequency (Frequency) may be unknown; in this case, by applying a periodic pulse signal and verifying its response, the frequency (Frequency) may be identified.

FIG. 13B is a diagram showing a state in which a waveform of a respiratory element is separated from a waveform of left atrial pressure obtained by a catheter examination to obtain an original waveform of the left atrial pressure. Since left atrial pressure is influenced by respiration and blood flow, it is known that a bright line fluctuates. In the present invention, the waveform of this left atrial pressure is processed as "Frequency Tunable Data." For example, in FIG. 13B, a portion where the bright line of left atrial pressure decreases corresponds to a portion where respiration decreases. Accordingly, by drawing a waveform of left atrial pressure using "Frequency Tunable Data" of respiration and a heartbeat, a bright line of left atrial pressure with reduced influence of respiration and blood flow may be obtained. That is, although influences of respiration and blood flow on left atrial pressure are included in calculation, by using "Frequency Tunable Data" of respiration and a heartbeat, an original waveform of left atrial pressure is obtained. Further, insofar as an influence of respiration exists, data of respiration may be extracted from "Frequency Tunable Data" of left atrial pressure and a heartbeat, and data of a heart may be extracted from "Frequency Tunable Data" of left atrial pressure and respiration to obtain a waveform close to an electrocardiogram. As such, various organ waveforms are mixed in biological data; however, by using a method of "Frequency Tunable Data," they may be separated, or may be combined.

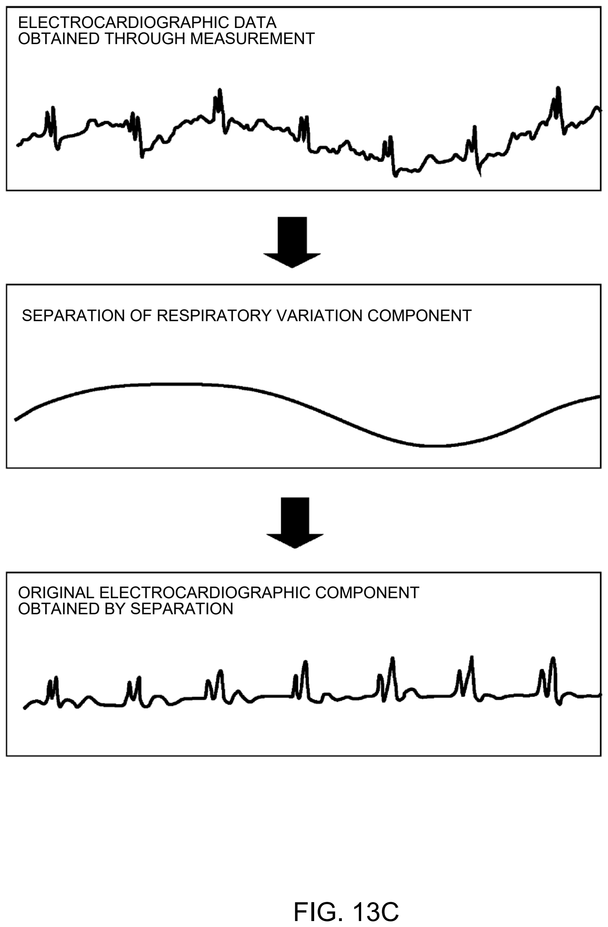

FIG. 13C is a diagram showing a state in which a waveform of a respiratory fluctuation component is separated from electrocardiographic data obtained by measurement to obtain an original waveform of an electrocardiogram. In this case as well, electrocardiographic data obtained by measurement is processed as "Frequency Tunable Data." That is, an amplitude, a phase, and a period are identified from a pulse indicating a heartbeat in the electrocardiogram, an amplitude, a phase, and a cycle of respiration are also identified, and these are separated from the electrocardiographic data. As a result, a waveform indicating an original electrocardiographic component with the respiratory fluctuation component removed is obtained.

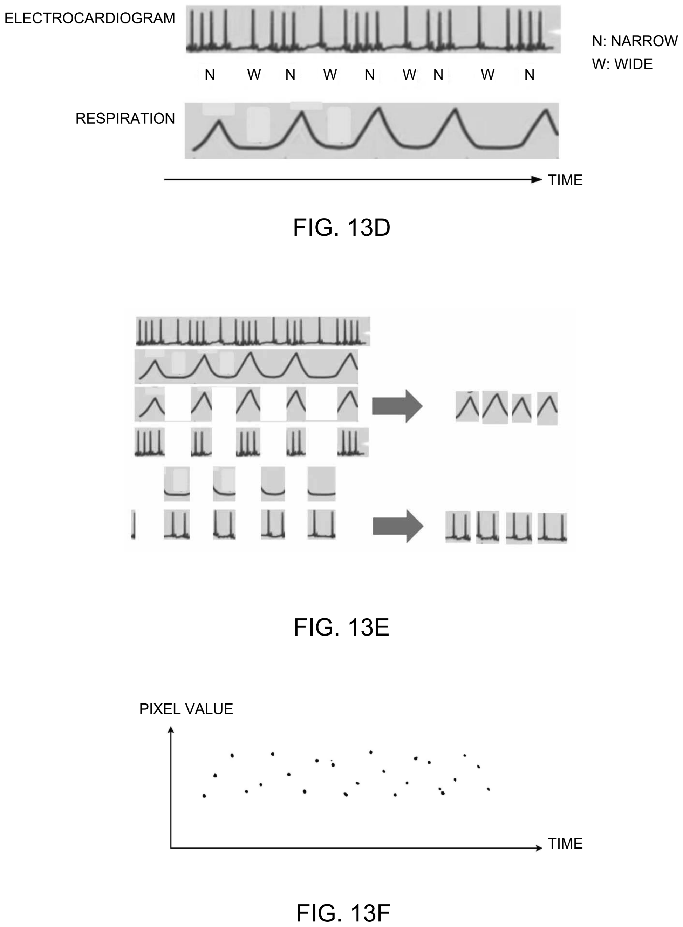

FIG. 13D is a diagram showing a waveform of an electrocardiogram and a waveform of respiration, and FIG. 13E is a diagram showing a concept of "Wave form tunable imaging." As shown in FIG. 13D, a waveform of the electrocardiogram includes a (narrow) portion with a narrow pulse width and a (wide) portion with a wide pulse width. This difference also appears in the respiratory waveform, and a peak portion in the respiratory waveform corresponds to the portion where the pulse width of the electrocardiogram is narrow. In addition, a portion of the respiratory waveform that is substantially horizontal corresponds to the portion where the pulse width of the electrocardiogram is wide. Accordingly, the (narrow) portion where the electrocardiographic pulse width is narrow is associated with the peak portion of the respiratory waveform, and the (wide) portion where the electrocardiographic pulse width is wide associated with the substantially horizontal portion of the respiratory waveform. In this case, a waveform may be divided by specific widths. As a result, a respiratory waveform may be obtained from the portion where the electrocardiographic pulse width is narrow and the portion where the electrocardiographic pulse width is wide. On the other hand, a waveform of the electrocardiogram may be obtained from the peak portion and the substantially horizontal portion of the respiratory waveform. In this manner, when an amplitude, a phase, and a cycle are identified, an assumed waveform (a pseudo waveform) may be created by "Waveform Tunable Imaging" based on basic data. Optionally, a logarithmic conversion process may be performed on basic data before "Waveform Tunable Imaging" is used to create a waveform.

In the present disclosure, "Frequency Tunable Data" is defined as information for tuning a frequency. This applies not only to a living body or an organ but also to general phenomena having periodicity. That is, processing of "Frequency Tunable Data" may be applied to "a periodic signal existing in nature or an artificially created periodic signal." This is because a periodic phenomenon corresponds to a waveform. A set of discrete points (data of pixel values) is processed by a least squares method and is converted and displayed as a wave shape. When a frequency is extracted from a waveform, the processing becomes "Frequency Tunable Imaging." This enables Fourier analysis. Further, local information may also be converted into a waveform. For example, by creating respective waveforms for respective regions of a lung, movements of a lung periphery and a lung apex may be displayed.

Further, for example, a pressure difference may be estimated from blood flow velocity. Based on a speed "V," a distance and a time may be grasped. By plotting a pulmonary hilum, a periphery, and the like and measuring density of each portion, a pressure of a pulmonary artery is found. Use of a catheter imposes a high level of invasiveness on a patient; however, when the pressure is grasped only with an X-ray image, the problem of invasiveness is resolved. Also, for example, in a normal person, a waveform from the pulmonary hilum toward the periphery is moderate, whereas in a person with heart failure, peripheral pulmonary vessels are narrow. This results from blood not flowing smoothly from beginning to end. Further, by measuring a caliber gap between a density at the pulmonary hilum and a density at the periphery, a structural disparity is grasped and a load state of a heart is found. This is due to the disparity between the pulmonary hilum and the periphery, and a state of the heart becomes graspable from how the density of a signal rises and falls. Optionally, the caliber gap between the density at the pulmonary hilum and the density at the periphery may be output as a numerical value, or may be used for training of AI. Furthermore, for grasping an increase or decrease in heart rate and a change in blood flow velocity, a difference in caliber is measured. Here, comparison may be made between a normal person and a patient, or measurement may be performed at different timings for the same patient. Further, comparison may be made between patient data and average data, or comparison may be made regarding how a comparative waveform differs from a basic waveform. Furthermore, although a method for obtaining a cardiac output called "thermodilution" is known, even when only image density is measured, a "vague and moderate curve" may be drawn. This enables distinction between a normal person and a patient with heart failure. In this case, only a signal value of the lung is extracted from pixel value data, and data of the lung is used.

Further, by using pixel value data of inside and outside a heart and of blood vessels and by computationally filling between data, any part may be visualized. For example, for "blood flow of an ascending aorta," once a bright line indicating blood flow is grasped to some extent, interpolation is enabled even when no data exists there-between. By measuring the density of the blood flow, similar data may also be obtained. This allows determination of whether the blood flow is in a "high heart rate system" or a "low heart rate system." For example, when an aortic aneurysm is present, subsequent blood flow velocity decreases. As a result, blood is not readily transported and swelling of legs and the like may occur. In treatment of aortic dissection, when a stent is inserted, whether blood flow is favorable or unfavorable becomes graspable. When MRI is used, a stent becomes an artifact due to use of magnetism, and display of an image around the stent becomes difficult. According to the present invention, because density before and after a target site is graspable, it may be displayed with sensitivity to a certain extent. That is, by using image density and performing image processing and pixel-value computation, a state of blood flow and a state of an organ become graspable. As a result, an invasive procedure for a patient becomes unnecessary. According to the present invention, a need to insert a catheter is eliminated, thereby reducing a burden on a patient.

Further, the present invention may be used for obtaining a ventricular volume. In calculation for a left ventricle of a heart, there is a formula concerning an epicardium; however, a right ventricle has a complicated shape and is difficult to grasp. Accordingly, by applying the present invention, "knowing a degree of change of an overall volume from a shape of a heart," "knowing a degree of movement from an outer wall of a ventricle," and "as a result, knowing a movement of a right ventricle" are provided. By calculating a ratio of signal values of an aorta of the heart and a pulmonary hilum, for example, an amount of blood flow supplied to a ventricle, a blood flow ratio, and an amount of blood in a single heartbeat become known. Then, based on these amounts, whether a left ventricle is impaired, a right ventricle is impaired, or both are impaired is inferable.

For another example, when a right lung is stenosed, blood flow in a left lung may become indiscernible in an image. Even in such a case, by using a ratio of pixel values, a difference of a resistance value of blood flow may be determined. Based on a rate of change of a density value, an overall difference, and an image pattern of blood flow, a state of a lung becomes graspable. According to the present invention, a distance from a pulmonary hilum to a periphery, a pressure of density, a valve of an aorta, a difference between left and right signal values of left and right pulmonary artery valves at the pulmonary hilum, a cardiac function, a proportion of a lung, and a resistance value may be obtained. That is, when an image of an aortic valve is captured, these numerical values may be obtained by image processing. For a blood vessel as well, in a portion where blood flows, numerical values are, in principle, obtainable. Accordingly, the present application is applicable not only to an X-ray image of a lung but also to a cross-sectional view in MRI and the like.

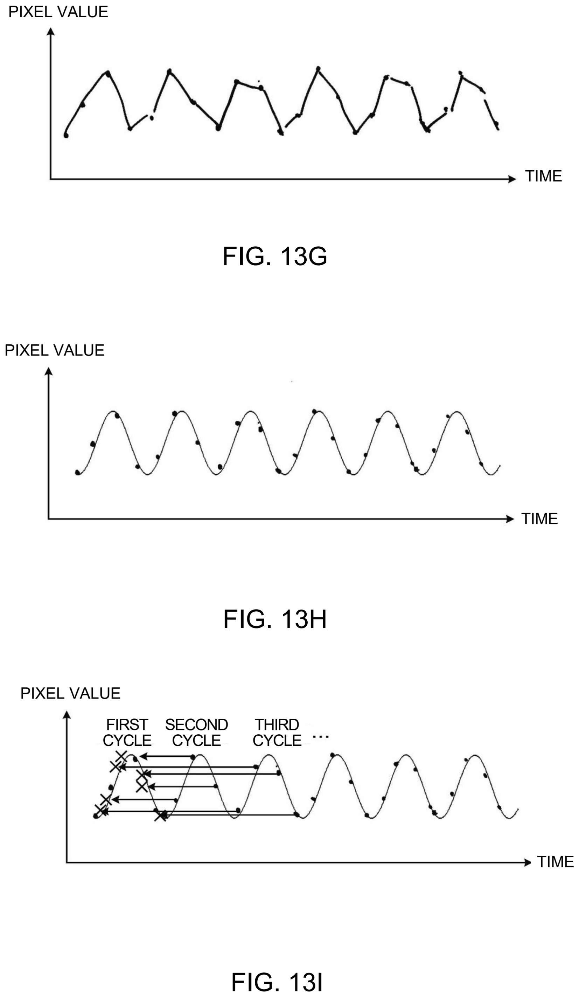

Data of pixel values appears as mere dots on a graph as shown in FIG. 13F; however, in the present invention, a wave is specified from a set of these dots. Here, when the dots are merely connected, a broken line graph results as shown in FIG. 13G, which may not be handled as a wave such as a sine wave. Accordingly, a frequency (Frequency) is identified, and by using a method such as a least squares method from a distribution of dots, a wave shape is obtained as shown in FIG. 13H. That is, as a method for filling between discrete points, interpolation is performed by curve fitting. In this case, use of a waveform may facilitate the completion. Then, for an outlier point, approximation to the waveform is performed using the least squares method. Namely, once one wave is obtained, a result when a phase shifts becomes predictable, and thus handling by a waveform becomes advantageous computationally. In other words, use of a waveform enables processing with a small amount of data. As such, once a waveform is obtained, an outlier point and a space between points become completable. Moreover, processing may also be performed by taking a difference alone. Furthermore, a Waveform may be created based on a measurement result, a Waveform as initial basic data may be created, or a Fourier transform may be performed and handled as a spectrum.

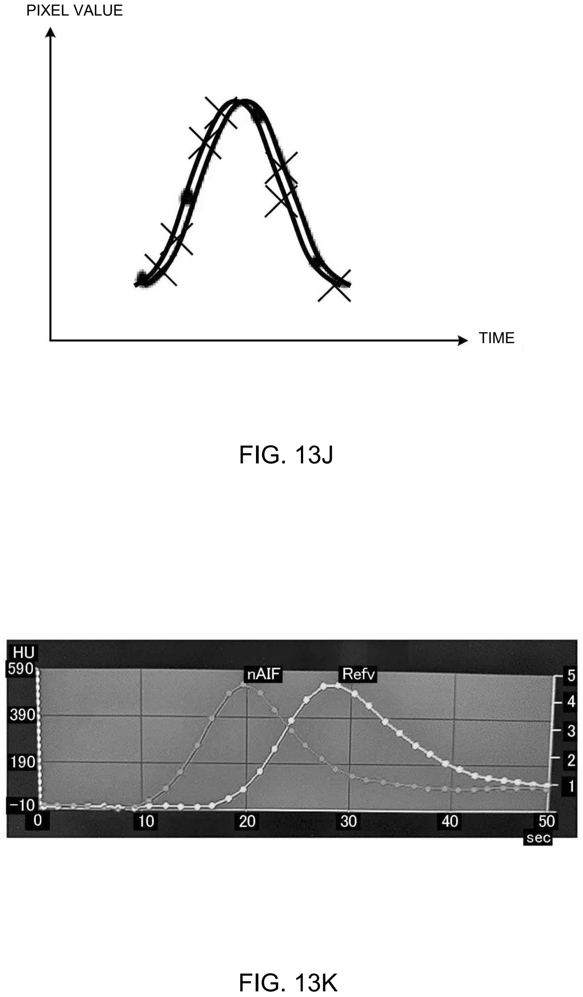

The present disclosure assumes that the waveform is obtained from an image of a living body. FIG. 13I is a diagram showing a state in which dots having an approximate phase are collected by phase within one cycle for a distribution of dots repeated over a plurality of cycles. Such dots existing over a plurality of cycles are collected by phase within one cycle, and a waveform (Waveform) is created using a method such as the least squares method. This facilitates creation of a sine wave from a state in which a change of pixel values appears as discrete dots. Regarding an error of the distribution of dots, as shown in FIG. 13J, processing may be performed such that a certain width is provided from an assumed waveform and dots within the width range are included in the waveform. In addition to extracting a waveform from an image of a living body, optionally, a waveform may be extracted from "data of living body"; further, not only for a living body but also for "an image indicating a repeating function" and for "data exhibiting a repeating movement," extraction of a waveform may be performed in the same manner as above.

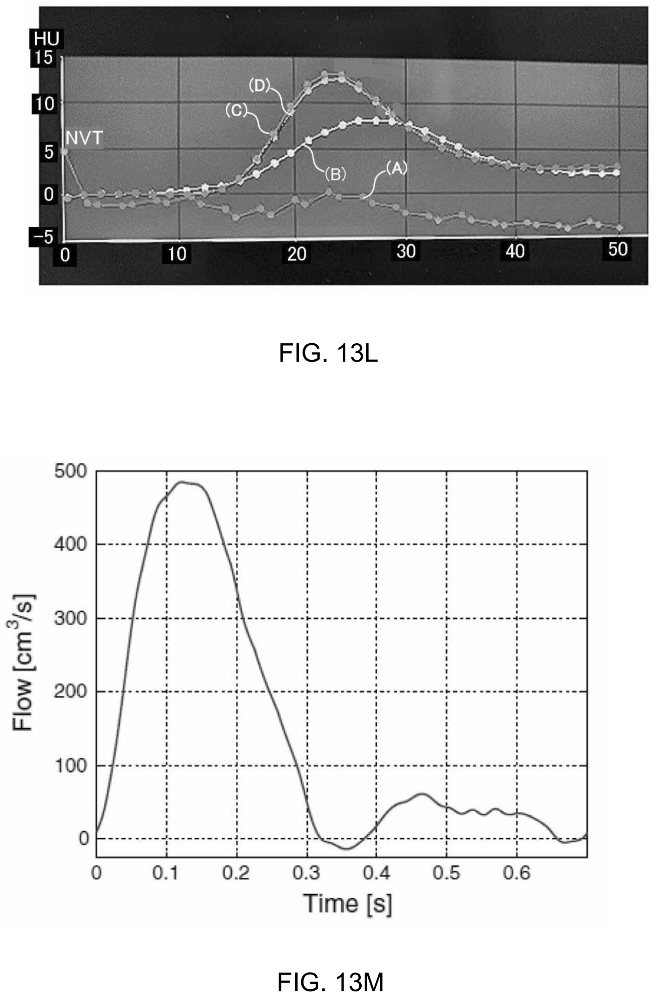

FIG. 13K is a diagram showing a distribution of pixel values. These pixel values are a graph indicating blood flow in a head of a certain patient. A left graph indicates normal blood flow, and a right graph indicates blood flow in a case of transient ischemia. The right graph shows that a phase is delayed and flow is slower relative to the left graph. FIG. 13L is a diagram showing a distribution of pixel values. These pixel values are a graph indicating blood flow in a head of a certain patient and indicate a situation worse than the case of FIG. 13K. In particular, graph (A) having the lowest amplitude value shows that "a situation where a phase is delayed and, furthermore, an amplitude is small." It is apparent that the blood flow causes an abnormality and that the blood does not flow normally. As such, dots indicating pixel values have phase information and signal value (amplitude information). As described above, a set of points is collected within one cycle, and a waveform is drawn using an amplitude, a phase, and a cycle. By observing a waveform, a phase shift relative to a normal value becomes apparent, and an abnormality in blood flow becomes graspable. Thus, a waveform may be used, or a set of points before a waveform is drawn may be used. When a set of points is used, optionally, information of preceding and succeeding points may be processed by taking a difference and calculating, or by performing logarithmic conversion. That is, together with an approach from a waveform, points (dots) serving as elements of the waveform themselves may be used and processed. Such a method may enable automatic diagnosis. Further, at a stage before conversion into a waveform, an abnormality may also be detectable as a numerical value (a point value). By calculating pixel values of dots, an abnormality may be detected and automatic diagnosis may be performed.

In the present invention, pixel values are acquired for each pixel in this manner and are graphed. This is an example of creating a waveform by collecting phase information and signal values (amplitudes). Note that, in FIG. 13L, graph (C) and graph (D) indicate normality. When a deviation occurs relative to such normal models, an abnormality may be diagnosed. That is, a phenomenon occurring in a living body is predicted using a model.

FIG. 13M is a graph showing a state of inflow into a main pulmonary artery (MPA). As shown in FIG. 13M, toward a center of a pulmonary artery, an amount of blood flow rapidly increases and rapidly decreases in a short time. This characteristic is similarly observed in a right pulmonary artery (RPA) and a left pulmonary artery (LPA). That is, in the main pulmonary artery (MPA), the right pulmonary artery (RPA), and the left pulmonary artery (LPA), a phase and a cycle of a wave match. However, in each graph, amplitudes differ, and consequently, waveforms differ. This may not be evaluated without actually describing waveforms. Then, when a deviation in phase or amplitude occurs relative to a waveform of a normal person, an abnormality is determined to be present. In other words, through comparisons between a normal person and a patient, between left and right brains, between different timings for the same person, or between persons of the same age group, an abnormality may be detected and a degree of abnormality may be assessed. All of these are information obtainable from pixel values of an image, and based on a change of a pixel value of any pixel, a phase, an amplitude, and a frequency are identified, and a waveform is created.

For example, in a waveform of a patient with myocardial infarction, a cycle may be shorter or an amplitude value may be lower compared relative to a normal waveform. In a patient with COPD, conversely, the cycle may be longer and the amplitude may be larger. As such, using any wave element including phase, amplitude, and cycle (frequency), creation of waveform may be processed. For example, myocardial infarction may be diagnosed from an electrocardiogram; however, determining whether a condition is improving or worsening afterward is difficult. According to the present invention, because portions where changes occur may be visualized by waveforms through comparison with previous images, one may grasp a history and transition of portions where changes have occurred in an electrocardiogram. As a method for selecting specific pixels from frame images, organs such as a pulmonary hilum and a myocardium are specified. For ribs, data is excluded as abnormal data based on the "FREQUENCY TUNABLE DATA" method.

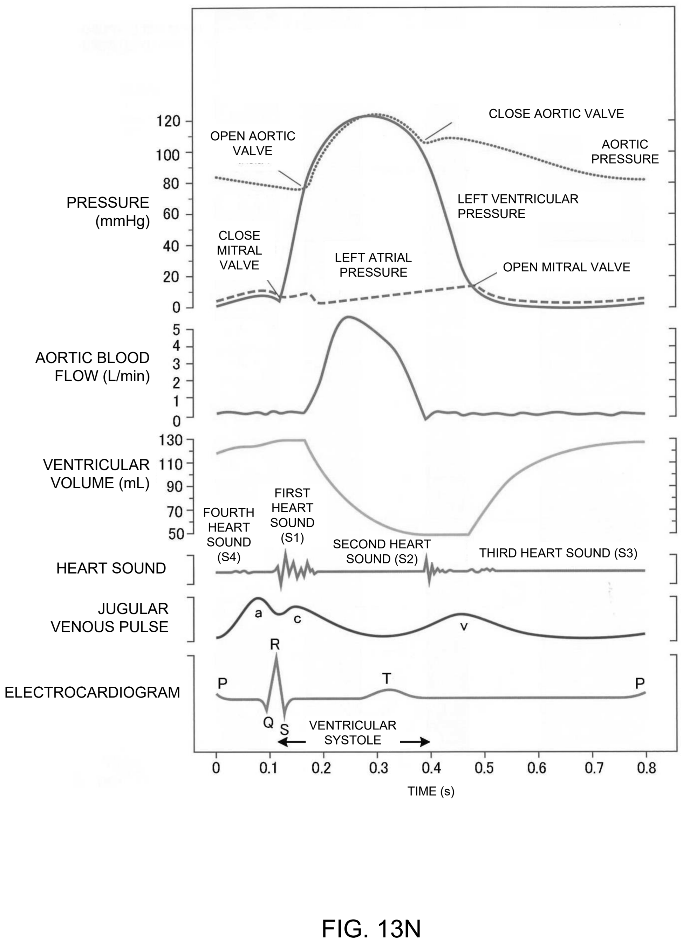

FIG. 13N is a diagram showing a waveform acquired within a single heartbeat of a heart. It shows that, even for the same heart, a waveform taken by an electrocardiogram and a waveform taken by a heart sound in each heartbeat differ from each other. However, by using one waveform (FREQUENCY TUNABLE DATA), a waveform of another organ may be identified. This is the meaning of "FREQUENCY TUNABLE TRANSFORM." A frequency may be extracted from a waveform, and also a waveform may be extracted from a frequency. In FIG. 13N, for example, when focusing on a ventricular waveform, a single heartbeat (cycle) may be identified. Then, by using this single heartbeat (cycle) and phase and amplitude information, a waveform of aortic blood flow rate may be obtained. That is, acquiring waveforms of ventricular pressure or aortic blood flow rate individually is no longer necessary. Further, when waveforms are the same (same organ), a frequency of another location may be extracted from the waveform. Moreover, the present invention is applicable not only to a living body but also to periodic motion. Dots on the graph may be used only as data or numerical values, or discrete information may be converted into a waveform and identified; that is, wave information (amplitude, phase, cycle) may be specified and used as a sine wave value. For example, while a pulmonary hilum is a part of a lung field, when a thrombus exists, observation of density at the pulmonary hilum may be difficult. In this case, waveform data of a ventricle may be inverted to create a wave similar to a change in pixel values at the pulmonary hilum and then logarithmically converted. Once an amplitude, a phase, and a cycle are specified, an assumed waveform (pseudo waveform) may be created based on basic data. "Waveform Tunable Imaging" is performed.

Further, in the present invention, a concept of "respiratory elements" is employed. The "respiratory elements" include all or a part of expiration or inspiration. For example, one respiration may be considered as a single expiration and a single inspiration, or may be limited to any one of 0%, 10%, 20%, 30%, 40%, 50%, 60%, 70%, 80%, 90%, or 100% of a single expiration or a single inspiration. Furthermore, only a certain ratio of each expiration, for example, only 10% of expiration, may be extracted and evaluated. By using any of these data or a combination thereof, more accurate images may be extracted. In this process, optionally, mutual calculations may be performed repeatedly.

This concept is applicable not only to respiratory elements but also to cardiovascular elements.

When creating base data, characteristic quantities obtained from one or more modalities (for example, variations composed of a certain range of "density" and "volumetry," chest wall movement, diaphragm movement, "spirometry," and two or more thoracoabdominal respiration sensors), or multiple waveform measurements in the same respiratory cycle may be used to complement each other's component extraction and improve accuracy. Accordingly, artifacts may be reduced, and accuracy may be improved based on certain predicted lines. Here, while "density" may be interpreted as "density," in imaging, it refers to the "absorption value" of pixels in a specific region. For example, in CT, air, bone, and water are represented as "-1000," "1000," and "0," respectively.

Further, an axis, width, range, and fluctuations of Hz of a wave are estimated based on mutual component extraction. That is, through multiple superpositions, the axis setting of Hz is averaged, and the axis, width, range, and optimal Hz range are calculated by dispersion. In this process, Hz of other activities (noise) is extracted, and the degree to which such waves are excluded may be relatively measured. In other words, only a part of waveform elements may be extracted from the entire waveform elements.

In the present disclosure, "density" and "intensity" are distinguished from each other. "Density" refers to absorption values as described above, and in original XP images or XP videos, regions with high air transmissivity are represented as white, with air displayed as "-1000," water as "0," and bone as "1000." On the other hand, "intensity" refers to relative changes from "density," for example, values converted from normalized concentration ranges or signal levels. That is, "intensity" represents relative values such as brightness or contrast in an image. While directly handling absorption values of XP images, they are expressed as "density" or "change in Δdensity." Then, for image representation purposes, the values are converted as described above and expressed as "intensity." For example, when displaying in color with 256 scales from 0 to 255, the color is represented as "intensity." This terminology distinction applies to XP and CT.

Meanwhile, in a case of MRI, even if air, water, and bone are set to "-1000," "0," and "1000," respectively, pixel values vary greatly depending on the type of MRI device, physical condition of a person at the time of measurement, body shape, measurement time, and signal acquisition method such as T1-weighted imaging, where these values are not constant across facilities and devices. Therefore, in the case of MRI, "density" may not be defined as that in the case of XP or CT. For this reason, MRI is handled with relative values from the initial rendering stage, and those relative values are expressed as "intensity" from the beginning. Moreover, the signal processed therein is also "intensity."

As described above, base data may be obtained. For a new target to be measured, data may be extracted from the waveforms and within a certain width and range of wave Hz in the base data. For example, it may be extracted by solely respiration or by width or range of vascular extraction, or waveform elements. The waveform and Hz width are determined relatively and comprehensively based on statistics, based on waveform elements in other functions, "artifacts" such as noise, waveforms of other "modalities" that are considered to be synchronized, and reproducibility over multiple runs. This requires adjustment and experience (machine learning may also be applied). This is because if the width and range are widened, elements of other functions tend to be included, whereas if they are too narrow, elements of the target function itself are eliminated; therefore, adjustment of the range is required. For example, if multiple sets of data are available, the range, Hz, and measurement matching width may be defined easily.

### [Synchronization Matching Rate]

In the present disclosure, tendencies of image changes are described in terms of synchronization matching rate. For example, a lung field is detected and divided into a plurality of block regions, and an average density (pixel value x) of each block region in each frame image is calculated. Then, a ratio (x') of the average pixel value of each block region in each frame image to the change width (0%-100%) between the minimum and maximum values of the average density (pixel value x) is calculated. Meanwhile, a value of the ratio (x'/y') between x' and a ratio (y') of diaphragm change (y) relative to the change width (0%-100%) between the minimum and maximum positions of the diaphragm is used, and only block regions where the ratio value (x'/y') falls within a predetermined range are extracted.

Here, when y' = x' or y = ax (where a is a coefficient representing a numerical value of diaphragm amplitude or "density"), it matches perfectly. However, perfect match is not the only meaningful value, but values within a certain width should be extracted. Accordingly, in one embodiment of the present invention, logarithms (log) are used to define a certain width as follows. That is, when calculated based on the percentage of y = x, perfect synchronization corresponds to "log y'/x' = 0." Further, for a narrow range (mathematically narrow) of synchronization matching rate, the range may be defined near 0, for example, "log y'/x' = -0.05 to +0.05," and for a wide range (mathematically wide) of synchronization matching rate, the range may be defined near 0, for example, "log y'/x' = -0.5 to +0.5." In other words, the logarithmic value of synchronization rate is defined as a certain range including 0. It may be considered that the narrower this range is, and the higher the number of matches within that range is, the higher the synchronization matching rate is.

When this ratio is calculated for each pixel and the number of occurrences is counted, for a healthy person, a normal distribution with a peak at perfect match is obtained. In contrast, for a person with a disease, the distribution of this ratio collapses. Note that the above-described method of defining a width using logarithms is merely an example, and the present invention does not limit the method thereto. That is, the present invention performs "image extraction" based on equivalence between (a rough range of density change) ≈ (chest wall movement) ≈ (diaphragm movement) ≈ (pulmonary function test) ≈ (thoracoabdominal respiration sensor movement) ≈ (lung field area and volume), and methods other than logarithmic methods are also applicable. With such methods, synchronized images may be displayed.

In a case of blood vessels, because a slight time delay (phase change) exists in a series of "density" changes x (one waveform at the pulmonary hilum) that occurs in response to a series of heart contractions y, the relationship is expressed as y = a'(x - t) (i.e., Y ≈ X). When synchronization matches perfectly, t = 0, and therefore y = x or y = a'x. Similarly to the diaphragm case, when extracting a narrow range (mathematically narrow) of synchronization matching rate, the range is defined near 0, for example, "log y'/x' = -0.05 to +0.05," and when extracting a wide range (mathematically wide), the range is defined near 0, for example, "log y'/x' = -0.5 to +0.5." It may be considered that the narrower this range is, and the higher the number of matches within that range is, the higher the synchronization matching rate is.

For other blood vessels, "the portion corresponding to the heart" described above is excluded, and the "density" plotted from the pulmonary hilum on the central side is used. Peripheral blood vessels may be handled in the same manner.

Furthermore, the present invention is also applicable to the circulatory system. For example, changes in "density" of the heart are directly related to changes in "density" of blood flow from the pulmonary hilum to the peripheral lung fields, and a series of "density" changes in the heart and in the pulmonary hilum are propagated after undergoing a type of transformation. This is considered to occur due to a slight phase difference between changes in "density" of heart and changes in "density" of pulmonary hilum. Moreover, because changes in "density" in areas such as the pulmonary hilum are directly related to changes in "density" of blood flow in the lung field, synchronization may be expressed based on a direct proportional relationship (Y ≈ X matching ratio). Similarly, in major vascular systems such as cervical vessels, thoracic, abdominal, pelvic, and limb vessels, changes in "density" plotted at nearby central cardiovascular structures are considered to be directly related or related with slight phase differences. Then, as the "density" varies and propagates depending on the background, the state of the "density" change may be analyzed as a synchronization matching rate.

Here, for both the change amount of a single image and the change rate of a single image, the total inspiratory volume may be approximated as equal to the total expiratory volume. Accordingly, for calculating relative values based on the difference from surrounding air permeability and expressing them as relative values (Standard Differential Signal Density/Intensity) using a change amount of 1 for lung field "density," the change amounts and change rates may be visualized for each of the following: (1) the image where the difference between each image is set to 1 for each image (normal assumption); (2) the total "density (amount of change or rate of change)" for each image is added together for the entire inspiration or expiration, or the ratio when the absolute value of inhalation and exhalation is set to 1; and (3) the ratio where the total "density" for each respiration (selecting several times when the rate is 10%) at multiple times of imaging is set to 1.

Further, in the case of 3D imaging such as MRI, the "intensity" (for MR) or "density" (for CT) of the entire inhalation may be summed (set to 1), and the difference in "intensity" or "density" may be converted into the "peak flow volume delta" of the inspiration (even at rest or during labored breathing). By calculating the ratio of these "intensity" or "density" values, it is possible to convert the actual respiratory volume and respiratory rate for each lung field, at least when calculating "3D x time" in MRI or CT. Similarly, by inputting stroke volume, it is possible to present an estimated distribution of peripheral pulmonary blood volume and capacity by converting the distribution in the "capillary phase" of pulmonary flow.

That is, (inspiratory change per image) · (total number of inspiratory images) ≈ (expiratory change per image) · (total number of expiratory images) ≈ (inspiratory volume at that time during natural or forced breathing) ≈ (expiratory volume at that time during natural or forced breathing) ≈ (amount of inspiratory or expiratory change in the "volume" during natural or forced breathing at that time) holds true. When extracting only the change amount for a single frame at 10% or 20%, an estimated value may be calculated by multiplying the total number of frames by the change amount at that time.

The extracted amount of change is visualized and rendered on the image. This provides the respiratory function analysis and vascular analysis described below. Thus, rates of change of the thorax and diaphragm are visualized. In this process, optionally, artifacts in the results may be excluded again, and functional extraction may be performed based on newly extracted waveforms, initial baseline waveforms, waveforms from other modalities, surrounding data, or multiple waveforms. Methods for artifact removal will be described later.

In some cases, characteristic features may be identified by excluding components of change extracted from sources other than those described above. For example, when analyzing bowel movement, respiratory and vascular influences are excluded from the abdominal region to extract intestinal motion.

Optionally, the least squares method may be used to generate a "wave form" as the baseline data, and the "wave form" may be subjected to the logarithmic transformation or digital filtering described above.

### [Combination with AI]

Normal and abnormal images may be trained using an Artificial Intelligence (AI) and applied for diagnosis assistance. For example, the lung periphery is thin while the interior is thicker. By taking differences corresponding to the spirograph's breathing curve, high and low regions may be distinguished. A normal lung is characterized by "thin periphery and thick interior," and "small slope at the start of motion, which gradually increases." When visualized, colors change gradually with the changes. Here, the gradient of lung expansion is represented by the time derivative of lung volume. For example, red indicates regions where density rapidly increases, signifying rapid contraction in the lungs, and blue indicates regions where density rapidly decreases, indicating rapid expansion. In mild abnormalities, some areas may not move properly, and no red or blue coloration may appear. In some cases, red and blue may even be reversed. In some cases, portions of the lung exhaling when they should inhale, or inhaling when they should exhale may be observed. While normal lungs show consistent change, abnormal lungs may show reversed or irregular change patterns. The AI may be taught with these change patterns, which may then output corresponding feature quantities. By associating these features with specific diseases and cases, diagnosis assistance may be available.

In creating a model by an AI training and making determinations using the model, information is collected, and the waveform is segmented by phase to capture the rising and falling portions of the waveform. For example, the phases of normal expiration and inspiration may be trained. That is, a normal model is first created, and then abnormal lungs that do not match the normal pattern are trained. Then, in the application step, the model is used to determine abnormalities and outputs, for example, the percentage of abnormality. For instance, the model may detect phenomena such as sudden narrowing or disappearance of vascular trunks or branches, sudden post-phase increases in density in narrow vessels, phase shifts, defects, or sparse image distributions. When these phenomena are recognized, a diagnosis of pulmonary thromboembolism (PTE) may be made. PTE is cited here as an example, but similar phenomena may occur in various other diseases. For example, pathological conditions may be grasped through changes such as decreases or increases in signal strength, variations in reactivity (signal change sensitivity), and signal defects. In other words, phenomena occurring in the living body may be predicted using the model.

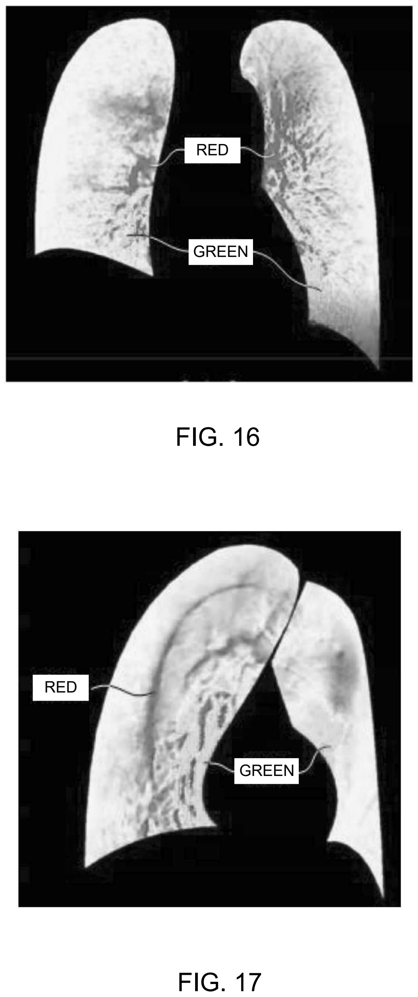

As shown in FIGS. 16 and 17, PTE patients exhibit non-uniform and phase-shifted pulmonary blood flow. That is, a phase shift occurs. According to the present invention, the baseline assumption is that high-signal regions normally appear radially and become globally visible in the next phase. When phase shifts occur, they may be determined as pathological abnormalities. An AI may be used to make these determinations. In such combination with AI, the key feature is the tracking of periodically moving phenomena.

### [Removal of Artifact]

Artifacts caused by the ribs must be identified and removed. In the lungs, the vascular branches extending from the hilum to the periphery are visible, and they are generally uniform and symmetrical. When visualized, the vascular branches appear, and the spaces between them are also displayed in red. This represents density changes extracted as frequency signals. Artifacts tend to occur around the diaphragm and heart, as well as in the upper inner lung region, because the ascending aorta is visible there. An AI is used to model lung motion for each cycle, learn abnormalities, and perform artifact removal.

The inventors of the present invention devised a method for removing the artifacts caused by ribs. It is necessary to distinguish between artifacts caused by ribs and respiratory changes, and artifact removal is achieved by using one or more of the methods described below. These methods may be applied to fluoroscopic images, difference images of fluoroscopic images, or filtered images. As a secondary effect, the position of the ribs may be identified, enabling not only rib removal but also visualization of rib motion.

The block intensity, representing pixel values in a small region, is calculated for each frame. In blocks where ribs enter the small region in only certain frames, the absolute value of block intensity change becomes relatively larger compared to respiratory changes. This relatively large change is regarded as a rib-induced artifact, and by applying corrections, rib artifacts may be identified and removed. Examples of correction logic include: "clamping values by defining maximum and minimum values (restricting value x within a predetermined range [a, b])"; "determining artifacts based on whether a predetermined threshold is exceeded"; "shrinking values so that the maximum change remains within the threshold when exceeded"; and "treating deviations beyond a certain proportion from a reference waveform as artifacts."

As described above, the presence of frames in which ribs enter and frames in which the ribs do not enter a given block is the source of artifacts. Therefore, artifacts may be reduced by modifying the block shape so that ribs are either included in all frames or excluded from all frames. Examples of methods for modifying block shape include logics of "enlarging the block size," "making the block a rectangle about twice the rib thickness," and "excluding outlier pixel areas within the block."

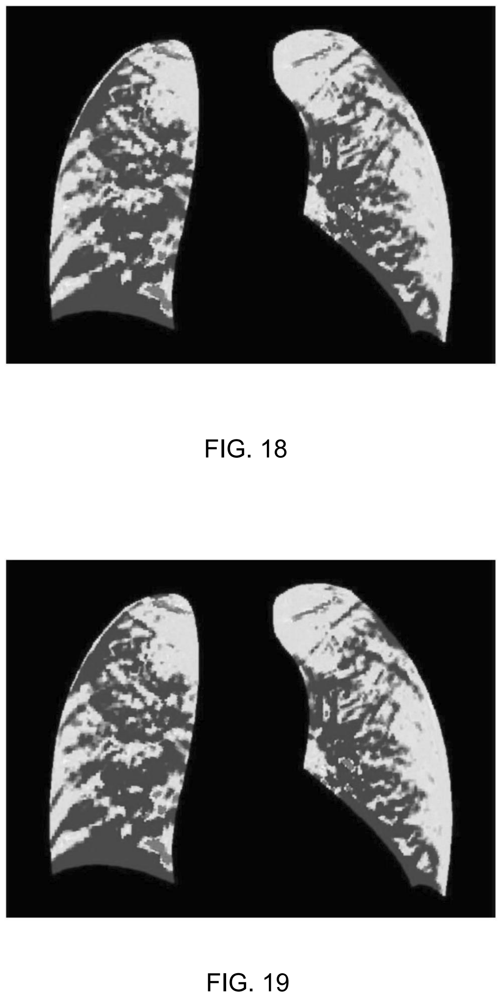

A drawback of the above method is that increasing block size reduces resolution. By taking the difference between the low-resolution difference image shown in FIG. 18 (obtained by increasing block size and aggregating luminance changes) and the high-resolution difference image shown in FIG. 19 (obtained by using smaller blocks), blocks with greater changes compared to surrounding blocks in the high-resolution image may be extracted. This method enables artifact reduction while preserving resolution. Additionally, the difference between low-resolution and high-resolution difference images may be used as a parameter characterizing lung structure movement, suggesting its potential clinical utility.

Another artifact removal method involves extracting high-density areas from outside the lung contour and identifying them as bone. This is a method where density outside the lung contour is measured and positions thereof are calculated to identify the ribs as artifacts. In other words, although depiction of ribs may be problematic, the original image is an X-ray image, and high-density areas may be presumed to correspond to ribs. Therefore, rib regions may be eliminated by applying masking and overlaying to cancel pixel values. After removal of the ribs, interpolation or difference processing is preferably performed. Optionally, when identifying lung contours, models of lung contours generated through Al-based machine learning may be used.

### [Learning and Determining by AI for Pulmonary Blood Flow]

In X-ray images, transmissivity of the lungs becomes high during inspiration because air enters and the structural density of the lungs decreases, and the image appears white. Conversely, during expiration, air decreases and lung structural density increases, resulting in low transmissivity, and the image appears dark (black). For blood vessels as well, during diastole, the amount of blood in the vessel lumen increases and the vessel diameter enlarges, resulting in reduced X-ray transmissivity. During systole, the blood volume within the vessel lumen decreases and the vessel diameter becomes smaller, leading to increased X-ray transmissivity. Thus, the density in images of the lungs and blood vessels fluctuates periodically. In the present invention, such fluctuation cycles, i.e., frequencies, are tuned to visualize specific regions (such as organs) within the image.

For example, for visualizing pulmonary vascular motion, the following procedure may be performed:
(1) Identify the blood flow frequency at the time of imaging.
(2) Create a frequency spectrum component table for the difference values of density before and after for each region in the X-ray projection (XP) video.
(3) Extract the frequency obtained in step (1) from the spectrum components.
(4) Visualize the extracted frequency (respiratory frequency).
(5) Output and display the frequency-tuned image.

Videos obtained through this procedure exhibit the following characteristics. That is, in healthy lungs, the resulting video shows a radial expansion pattern from the center. On the other hand, in patients (e.g., PTE: pulmonary thromboembolism), vessels that should be visible disappear, resulting in a video with uneven patterns.

In the present invention, for example, the video may be recorded at 12.5 frames per second. That is, 12 images (frames) may be captured per second. With this setting, two major perspectives are considered for normal lungs:
- "Pulmonary vascular structure"
   In normal lungs, peripheral vessels branch out from the main vessels like tree branches. They appear to flow uniformly in a radial manner. The central vessels appear thick and dense.
- "Flow in capillaries and peripheral pulmonary vessels (Normal pulmonary flow)"
They appear as a continuous distribution of fine dots, resembling clouds. Such visualization of pulmonary vascular structures and capillary/peripheral pulmonary vessels is due to faster blood flow in the pulmonary trunk and central side, and slower flow in peripheral vessels.

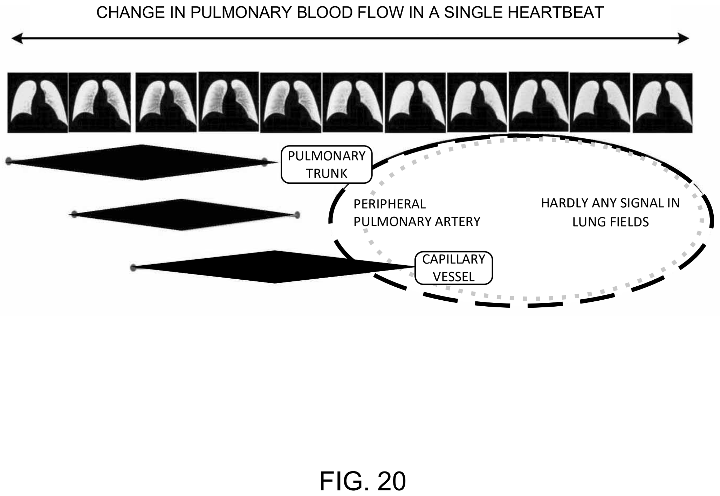

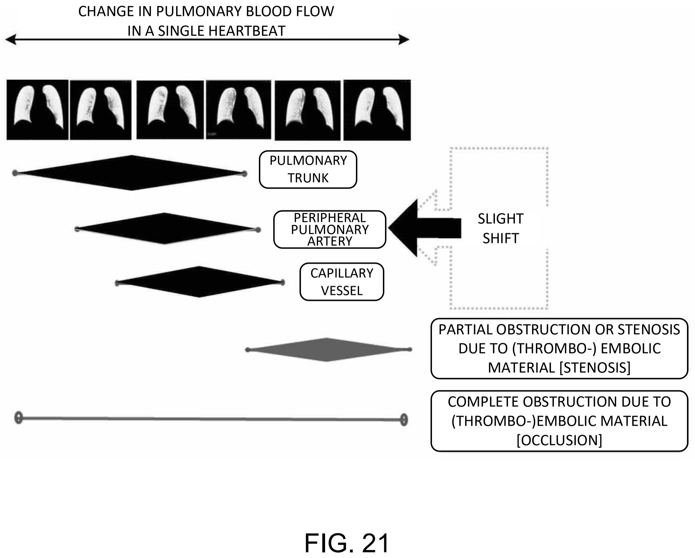

FIG. 20 is a diagram showing a change in pulmonary blood flow within a single heartbeat in normal lungs. A single heartbeat is represented by 12 frames. In normal lungs, blood flow is observed throughout the lungs during approximately the first 45% of the respiratory progression. During the latter half of the single heartbeat, as the heart rests, blood flow is less prominent compared to the first half. In contrast, in patients with pulmonary thromboembolism, it appears differently. FIG. 21 is a diagram showing a change in pulmonary blood flow within a single heartbeat in abnormal lungs, where a single heartbeat is represented by 6 frames. As shown in FIG. 21, the pulmonary vascular structure does not appear to spread radially from the center and is uneven. Missing areas in the image indicate abnormalities. While the vascular structure of the left lung is normal, the right lung appears sparse with significantly reduced blood flow. The vessels themselves are not visible. In a normal lung, the vascular structure should appear clearly radial, which is not the case in FIG. 21. As such, the pulmonary vascular structure may be diagnosed. Moreover, even in MIP images, abnormal lungs exhibit gaps and lack fine details. They contain many voids and have low density. As such, the present invention enables checking of vascular structures and allows the potential presence of pulmonary thromboembolism to be identified based on image display patterns.

### [Flow in Capillaries and Peripheral Pulmonary Vessels (Normal Pulmonary Flow)]

In abnormal lungs, the flow in capillaries and peripheral pulmonary vessels does not appear in the image. This allows the abnormality to be determined. For example, in the peripheral region of the lung, the lung thickness decreases; however, in an abnormal lung, the areas where density increases (appearing red in the image) rarely appear. In normal lungs, the areas with increased density are distributed with a gradient, whereas in abnormal lungs, such distribution is absent. As such, differences between normal and abnormal conditions are displayed.

When acute pulmonary embolism (APE) is suspected, the image exhibits the following features. That is, blood flow appears in the peripheral regions of the lungs after the respiratory movement. In other words, delayed blood flow appears in the image. This occurs because blood begins to flow into occluded vessels, resulting in delayed visualization of blood flow. In cases of "stenosis" (partial obstruction or narrowing), as shown in FIG. 21, the vessel appears in the latter half of a single heartbeat. In other words, it becomes visible in the later phase of the cardiac cycle. In cases of "occlusion" (complete blockage), as shown in FIG. 21, no blood flow appears in the image at all. It is not visible even in MIP images. As such, images of normal and abnormal lungs are used as training data for an AI.

Meanwhile, if the flow in capillaries and peripheral pulmonary vessels is not visualized in the phase immediately following the pulmonary arterial phase, a thromboembolic visualization defect may be suspected. Here, (a) if the capillary and peripheral pulmonary vessel flow is visualized with a delay, the vessel is in a narrowed state, and the visualization occurs in a late phase due to stenosis or similar conditions. (b) If the flow is not visualized in any phase, it may be inferred as "occlusion," suggesting complete blockage of blood flow.

That is, defect regions in the vascular structure may be identified, and the flow in capillaries and peripheral pulmonary vessels of terminal vessels may be examined in conjunction with the vascular structure. By checking areas without flow in the early phase (a phase immediately after vascular visualization) and areas with flow in delayed phases, the presence of "partial obstruction" (stenosis) may be determined. By checking areas with no flow in all phases from early to delayed phases, the presence of "complete obstruction" (occlusion) may be determined.

For example, in APE, blood flow in the right lung may suddenly appear. That is, flow in capillaries and peripheral pulmonary vessels may appear in regions that should be occluded. This phenomenon occurs when the main vascular flow is blocked and dammed, causing a sudden surge of blood into the peripheral vessels.

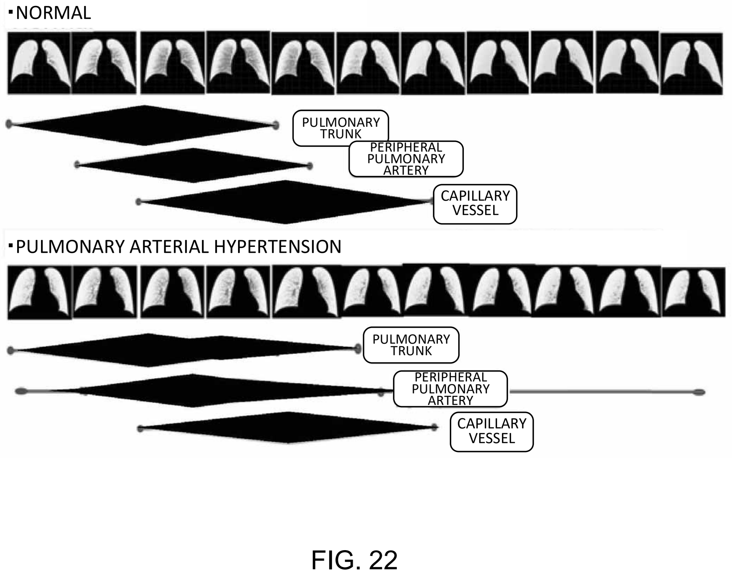

FIG. 22 is a diagram showing normal lungs and lungs with PAH (pulmonary arterial hypertension (including pulmonary arteriosclerosis)). In PAH, there are no large defects when viewing the lungs globally, but irregularities appear. Normal vascular structures extend radially from the center of the lung toward the periphery. Few branches are observed. However, when PAH-related abnormalities are present, the vascular branches exhibit "irregular shapes." That is, when PAH-related abnormalities are present, the number of radial lines in the vascular structure decreases, and the vessels become rapidly thinner toward the periphery. In other words, the vascular structure becomes sparse and irregular, with extremely thin peripheral vessels. Although the overall distribution may appear uniform, detailed inspection reveals a random distribution differing from normal. Gaps also appear in the central vascular structure of the lungs. This indicates a lack of motion in that region. Furthermore, the onset of blood flow is delayed, and the vascular appearance occurs later. For example, vascular structures may begin to appear around the center of the lung with a delay of 1 to 2 frames. This is abnormal. Moreover, although the peripheral vessels are thin, blood flow appears at a delayed timing. This indicates delayed blood flow and abnormalities in the lungs. Further, flickering or delayed appearance of vascular structures may be observed in the image. Furthermore, the absence or minimal visibility of capillary and peripheral pulmonary vessel flow indicates abnormalities. These phenomena are visualized as differences from the normal state.

Here, the mechanism by which pulmonary vascular signals are visualized is described. In normal pulmonary vessels, pressure differences are first applied to the vessels by cardiac pumping. Next, vessel elasticity causes expansion and contraction. In other words, the vessel expands and contracts due to its elastic force. As a result, the vessel diameter varies. In other words, in normal conditions, variations in the lumen size of vessels are observed. The signal is then recognized as periodic changes in XP density and reflected in the image. In cases of arteriosclerosis such as PAH, even when cardiac pumping occurs, pressure differences are less easily transmitted to pulmonary vessels. Moreover, vessel elasticity-based expansion and contraction also become less pronounced. This is because arteriosclerosis (fibrosis) causes thickening of the lumen, which reduces elastic force. As a result, the force by which the cardiac pressure difference propagates to the peripheral vessels decreases. This is less reflected in vessel diameter changes. That is, even with cardiac pumping, vessel diameter fluctuations weaken. Consequently, various abnormal factors appear in the image as periodic XP density changes. For example, a general reduction in signal values (pixel values) is observed. Also, difficulty in applying pressure differences and reduced vessel elasticity cause delayed or weak ejection through the pulmonary artery. Furthermore, reduced vessel elasticity diminishes capillary and peripheral pulmonary vessel flow from the central to peripheral lungs, causing inconsistent vascular phase shadows. Weak vessel diameter changes make it difficult to visualize peripheral and small vessels. The central vessels exhibit club-shaped or branching forms, and the "diffuseness" centered in the periphery decreases.

Accordingly, when vascular elasticity is lost due to sclerotic (elastic) changes of vessels, it may be considered that depiction of blood flow becomes difficult. Moreover, when internal density changes hardly occur, signals (pixels) may no longer be observed. In addition to PAH described above, similar image patterns are predicted to appear due to chronic heart failure, hyperparathyroidism, chronic renal failure, collagen diseases such as amyloidosis, and other pulmonary arteriosclerotic changes.

### [Ventilation-Perfusion Mismatch]

Conventionally, ventilation-perfusion mismatch in the lung has been diagnosed by pulmonary perfusion scintigraphy. These relationships are understood physiologically. In the present invention, ventilation and blood flow are simultaneously displayed on a screen, and determination is made based on a combination of respiration and blood flow. For example, when lung motion is generally normal but pulmonary blood flow does not correspond, an abnormality may be determined. For another example, when ventilation is normal but blood flow is abnormal, pulmonary hypertension or pulmonary thromboembolism is suspected, and when both are poor with reduced flow, it may be determined as emphysema. On the other hand, there may be cases where vascular motion is normal but lung motion is abnormal. For example, heart failure accompanied by abnormal respiration falls into this case. Even in healthy persons with normal heartbeat, dyspnea, for example due to choking on food, falls into this case. By machine learning using an AI, a model of "ventilation-perfusion mismatch" such as these is generated.

For example, in cases of hypoxic lung disease (HLD), the flow in capillaries and peripheral pulmonary vessels is deficient. That is, defects exist in respiratory regions of the lung, and blood flow in corresponding regions is also reduced. The same phenomenon is observed in COPD. According to the present invention, diagnosis may be performed by training an AI with correspondence and contrast between ventilation and blood flow.

### [Correlation with Pulmonary Scintigraphy]

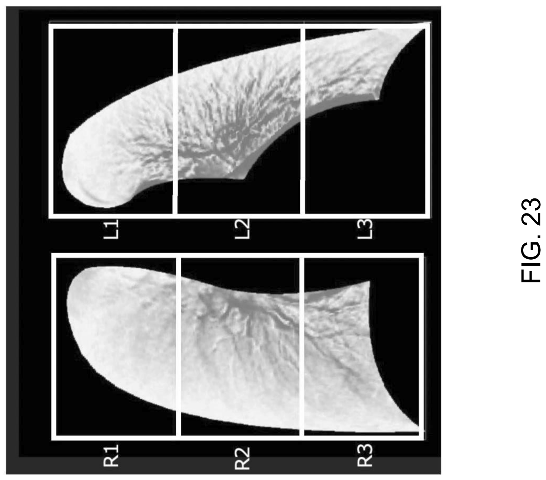

FIG. 23 is a diagram showing example images of MBDP (Maximum Blood-flow-related Differential Projection) and ABDP (Accumulated Blood-flow-related Differential Projection). MBDP "projects, for each block, the largest inter-frame value onto a single image," whereas ABDP "projects, for each block, a value obtained by summing positive-direction changes onto a single image." As an application of MBDP and ABDP, the lung image may be divided into six areas, and averages and distributions of intensity values in each area may be calculated to serve as clinical indices.

For example, correlation may be established with pulmonary scintigraphy (an existing method for evaluating pulmonary perfusion). Pulmonary scintigraphy measures "degree of blood accumulation" by injecting a radiotracer and counting radioactive substance, and is characterized by higher signals at sites of blood stasis. In pulmonary scintigraphy as well, the lung image is divided into six areas, and the radioactive substance is counted for each area. Because pulmonary scintigraphy is evaluated relatively, for example, when the radioactive substance is counted as "100, 200, 300, 400, 500, 600," the ratios to the total count of 2100 are calculated as "4.7%, 9.5%, 14.3%, 19%, 23.9%, 28.6%," respectively. In MBDP and ABDP as well, totals of intensities in each area may be regarded as counts in pulmonary scintigraphy and evaluated as ratios to the total intensity. This enables acquisition of images similar to scintigraphy. For example, when counting respiration and blood flow, delays or advances and whether substantive flow is present may be counted as signals. Because the total amount of flow may be assessed, this contributes to assist diagnosis.

### [Reproducible Events]

According to the present invention, an AI is trained by machine learning on events that appear repeatedly, that is, reproducible events. This enables approaches in which normal states are learned for abnormality determination or abnormal states are learned for outputting candidate cases. As an output method for the AI determination, feature quantities for each case are output. These feature quantities may be defined based on image patterns (such as average pixel values per region and rates of pixel value changes) and include multiple diseases.

### [Pulmonary Blood Flow and AI]

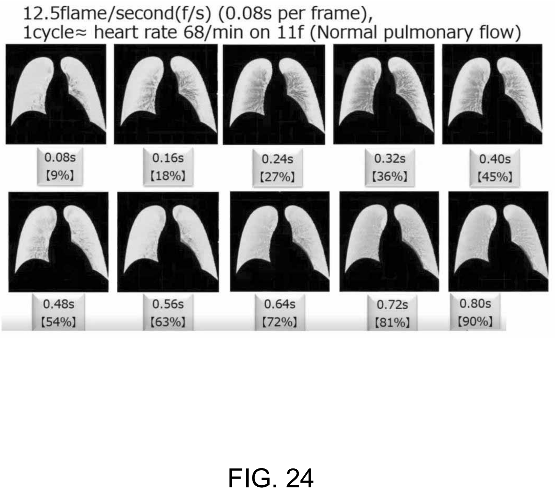

FIG. 24 is a diagram representing pulmonary blood flow for each frame within a single heartbeat of a heart. In FIG. 24, the pulmonary blood flow during a single heartbeat is represented by ten frames. During a single heartbeat of pulmonary blood flow, flow begins in the central pulmonary vessels and then spreads to the peripheral terminal vessels. Because terminal vessels are thin (small lumen), flow slowly, and are numerous, they appear in the image as a delayed, spreading like cloud following the central flow. That is, terminal vessels become visible after the flow in the main arteries (main pulmonary artery and distal pulmonary artery) appears.

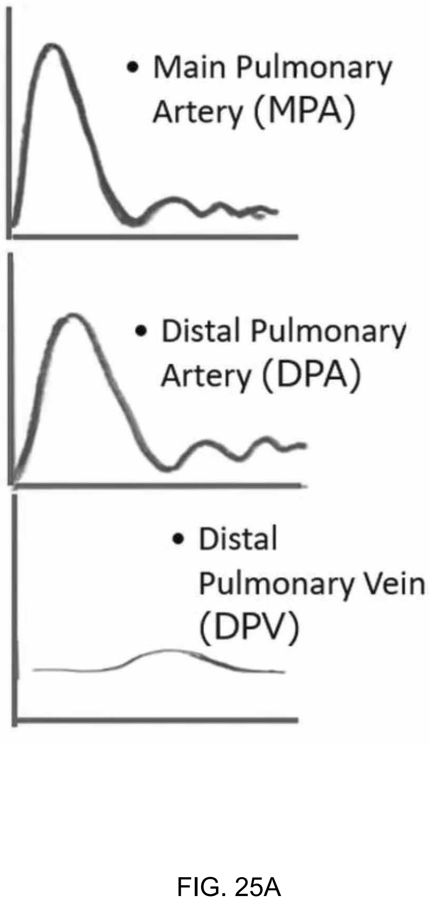

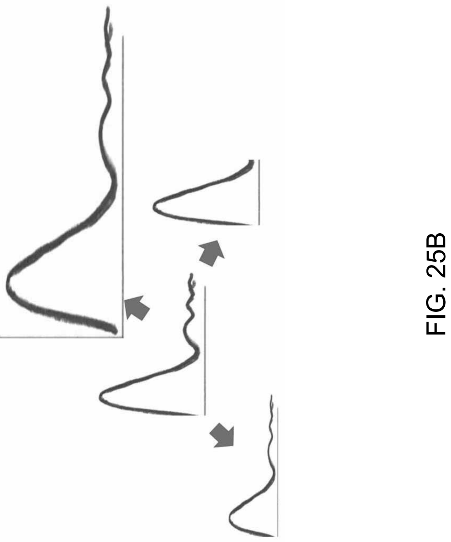

FIG. 25A is a diagram showing example measurement results of an MPA (Main Pulmonary Artery), a DPA (Distal Pulmonary Artery), and a DPV (Distal Pulmonary Vein). DPA is observed with a slight delay after MPA, and the value drops sharply from MPA. The terminal vessels described above appear before DPV. In FIG. 24, as shown in the measurement results of FIG. 25A, the vessels from central to peripheral regions appear in the second to third frames, followed by the appearance of fine vessels in the fourth, fifth, and sixth frames. Optionally, as mentioned above, waveform tunable imaging may be applied to these waveforms. As shown in FIG. 25B, processing such as "amplitude modulation," "phase expansion/contraction," "phase trimming," and "phase stitching" may be performed on the basic waveform to adjust the waveform shape.

For training an AI on pulmonary blood flow, the standard lung field may be measured radially from the hilum, or a "bull's eye" representation may be used to express concentric circles as in the case of the heart. Although vessels spread concentrically from the hilum, there is a phase in which vessel pixel values peak (e.g., the third or fourth frame in FIG. 24). Both vascular structures and peripheral vessels are included in this phase. Accordingly, to grasp pulmonary blood flow signals (pixel values), the pixel value peak is first detected. Then, since there is a phase just before this peak in which the vascular structure is visible, this phase is extracted. This detection method includes: (1) "extracting vascular structures;" and (2) "extracting at or near the hilum, based on the fact that pulmonary blood flow moves from the hilum to the periphery."

For example, in the lungs of a patient with acute pulmonary embolism (APE), vascular structures become sparse, so the lung field is divided into about 17 segments along concentric circles in the bull's eye representation. By arranging the lung shape concentrically, the phases of blood flow through the main vessels and the terminal vessel flow may be observed. Therefore, displaying variations in distribution facilitates an AI processing. This is because variations in distribution make it easier to distinguish between normal and abnormal states. In other words, the lung field is spatially divided by area, and pixel values of the areas with and without blood flow are quantified. This quantification may be performed automatically. Such quantification enables application of AI. Using this method, calculations may be made according to segmentation, enabling detection of defects and the like.

For terminal vessels, their pixel values are obtained by subtracting the pixel values of the vascular structure areas from the total. That is, the remaining portion after excluding vascular structures corresponds to the terminal vessels, and it is possible to show the nature of blood flow in the terminal vessels.

Furthermore, detecting which parts of the three-dimensional structure are defective (i.e., whether blood flow is present or absent) is possible. The same visualization may be performed when using 3D or 4D data. In particular, the vascular structure is divided into areas, and the phase just before the region of high pixel value (signal) of the vascular structure is detected. By this method, both the vascular structures and terminal vessels may be detected, and their blood flow characteristics may be visualized. Collecting these signals produces bar graphs that rise and fall, representing the behavior of the vessels within each area. Moreover, the behavior of the terminal vessels within each area may be displayed. Furthermore, the overall flow pattern may be displayed. When these are calculated over a single heartbeat, the waveform shown in FIG. 25 is obtained.

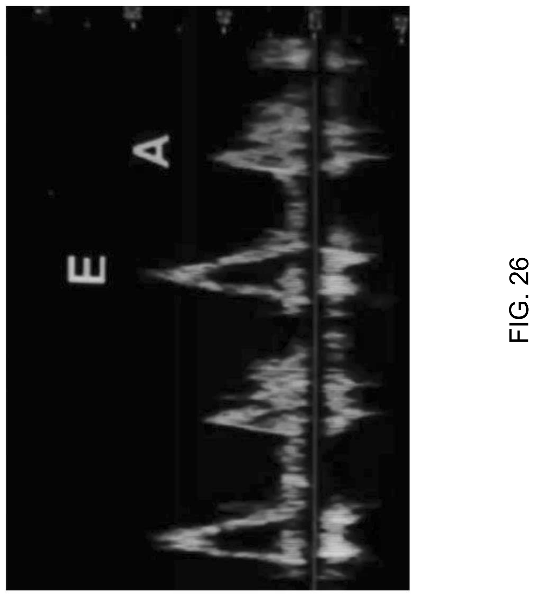

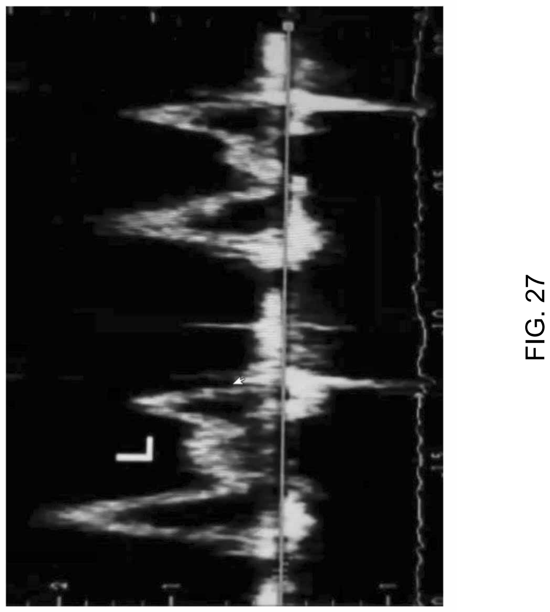

FIGS. 26 and 27 are diagrams showing signal intensities in ultrasound images of the atrium. In FIG. 26, a peak signal E and a signal A representing the effect of atrial motion are observed. FIG. 27 shows that a residual signal L appears between the signals E and A. If the signal L appears between the peak signal E and the atrial motion signal A, heart failure may be suspected. Thus, by graphing and quantifying differences relative to normal organs, pathological changes may be predicted.

A method of comparing past and current ECG waveforms by superimposing them is also known. For example, by superimposing waveforms shown in red, blue, and green, diagnosing automatically is possible by comparing pre- and post-treatment, healthy individuals and patients, or different time points for the same patient. For example, by detecting T wave shifts or instability after the peak, automatic diagnosis may be performed, enabling physicians to prepare for writing their reports.

### [AI-Based Learning and Assessment of Pulmonary Respiration]

For pulmonary respiration, the synchronization of changes within the lung field is tracked. In other words, whether the movement of the lungs spreading from the central to the peripheral regions is normal is determined. Specifically, linked movements of the diaphragm, ribs, and lung tissues are examined. Additionally, the movement of the pectoralis major may also be considered. The degree of spacing in the images may also be evaluated. Furthermore, even normal lungs may appear differently under spontaneous breathing versus labored breathing, but both of these are determined to be normal. The determination is made by "linkage" based on "area over which lung movement propagates," "continuity and flow of blood," "continuity of movement from the lung center to the periphery," and "diaphragm's own movement."

More specifically, a single waveform (e.g., a wave of respiratory element) extracted from the image is used as a reference, and linkage with respect to the reference is evaluated. Lung motion is reduced to a waveform, and its amplitude, phase shift, and vector direction are checked, and the AI makes assessments in combination with anatomical knowledge. In this case, the evaluation target depends on which waveform is extracted from the image. That is, since diseases (cases) have characteristic frequencies and phase shifts, diseases may be identified based on the detected frequencies and phase shifts. For example, in areas where the lungs are fixed due to tuberculosis, the frequency becomes zero. For example, in cases with impaired neural transmission, such as myocardial disorders, the phase shifts and waveform delays occur, resulting in different waveforms. Since disease-specific frequencies exist, disease-specific frequency tuning images may be output. This enables the extraction of new features and the generation of new images.

In the present invention, imaging is performed in accordance with the respiratory frequency. That is, when images corresponding to the respiratory frequency are extracted, images showing the movement of the lungs due to respiration may be obtained. Here, differential values of lung movement are visualized. Such images extract only changes corresponding to the respiratory frequency in X-ray images. By tracking changes in density, regions that appear whiter (lower transmissivity) on XP correspond to higher density (green, yellow, red), while regions that appear darker (higher transmissivity) correspond to lower density (green, light blue, blue). These changes are visualized in the images. It should be noted that the images in the present invention are the reverse of the inspiratory/expiratory graphs obtained in precise pulmonary function tests.

Changes in signal during lung movement show that at the beginning of respiration, the signal intensity increases from the trachea and bronchi (central) to the bronchioles and alveoli (peripheral), and toward the end of respiration, the signal intensity decreases from the alveoli (peripheral) toward the bronchioles and trachea/bronchi (central). This applies to both inspiration and expiration. The same holds for XP: during inspiration, increased transmissivity corresponds to decreased density, resulting in a negative vector (shown in blue). Conversely, during expiration, decreased transmissivity corresponds to increased density, resulting in a positive vector (shown in red).

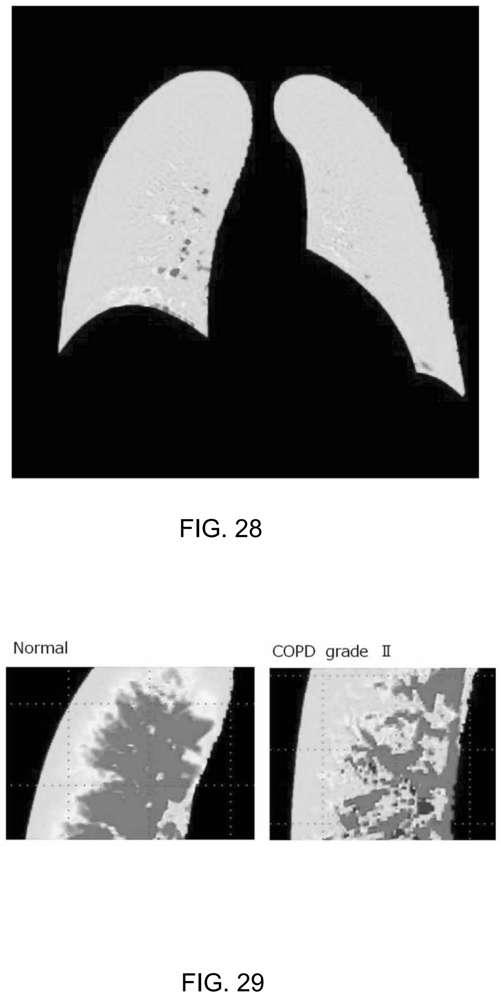

FIG. 28 is a diagram showing an image of normal lungs. As inspiratory motion (increased transmissivity) intensifies, the color changes from light blue to blue, indicating a strong negative density vector. Conversely, as expiratory motion (decreased transmissivity) intensifies, the color changes from yellow to red, indicating a strong positive density vector. The thinner the outer portion of the lung, the weaker the overall signal; conversely, the thicker the inner regions and areas near the diaphragm, the stronger the overall signal. The strength of the signal increases or decreases according to the inspiratory and expiratory motion patterns.

### [Correspondence Between Images and Diseases]

FIG. 29 is a diagram displaying a normal lung and a lung with COPD. In a normal lung, "expansion may be observed from the bronchi to the segmental bronchi, bronchioles, and alveoli," whereas in a COPD-affected lung, expansion is limited to "the bronchi and segmental bronchi" and does not reach "the bronchioles or alveolar ends." As a result, the central part of the COPD lung shows irregular and mottled imaging. Left-right asymmetry also contributes to abnormality determination. Linkage is also affected: the movements of the diaphragm, ribs, and lung tissues become uncoordinated. In the present invention, the linkage between the reference waveform and each structure is evaluated in association with anatomical structures.

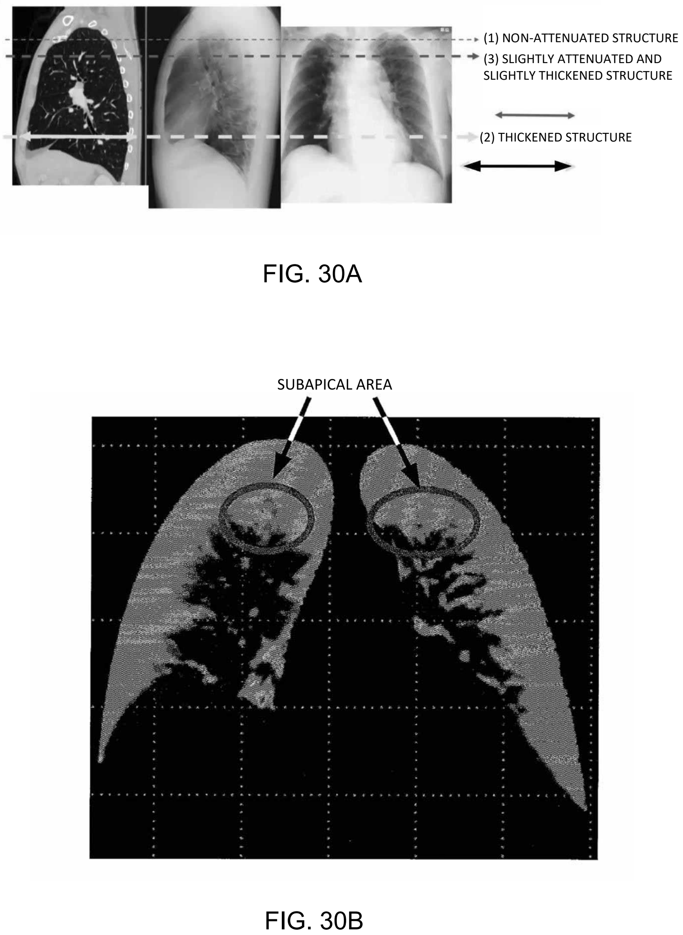

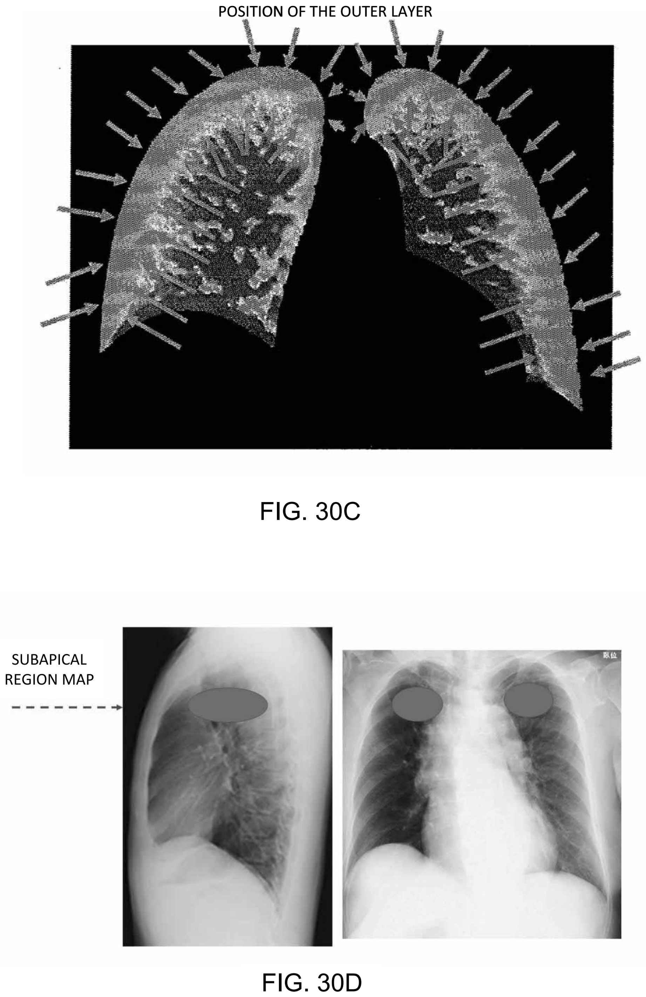

FIG. 30A is a diagram showing a three-dimensional structure at an apex-diaphragm side. FIG. 30B is a diagram showing positions of subapical areas. FIG. 30C is a diagram showing "positions of outer layers." "Subapical area" is defined as the region slightly below the outer layer of the lung apex. This is a small region where individual differences appear in the anteroposterior direction. More specifically, it corresponds to a part of the upper lung field when the lung is divided into upper, middle, and lower regions. For the outer edges (upper, middle, and lower lung field outer layers) and the lung apex, the anteroposterior thickness is small, resulting in signal attenuation in most individuals. In regions with signals, the lung field is typically divided into upper, middle, and lower regions. In an area directly below the apex ("subapical area"), slender individuals have shorter anteroposterior diameters, whereas individuals with larger anteroposterior diameters, as shown in FIG. 30C, exhibit less thinning. Thus, the area below the lung apex is specifically defined as the "subapical area." Defining this region allows for special handling "during segmentation" of regions with weak vascular or respiratory shadows, facilitating diagnosis. For the remaining lung fields, "segmentation" is performed from outer to inner regions, from upper to lower lung fields, and radially from the hilum for vascular segmentation, thereby defining regions for Al learning and signal analysis. This facilitates comparative evaluation. Although curves or Bezier curves are used to define the lung fields, optionally, anatomically based lines may be used.

FIG. 30D is a diagram showing the positions of the subapical areas. The subapical area is located slightly below the lung apex and, except for thin portions at the outer edges of the lung field (in the anteroposterior and lateral directions), has less thickness than the overall lung field. It is a region where interindividual differences tend to arise relatively easily. In other words, it is located below the apex and corresponds to the anterior-posterior outer layer (where the lateral thickness of the lung is thin). This area typically makes it difficult to detect changes in pixel values, requiring contrast enhancement, brightness adjustment, or interpolation from surrounding pixels. When the lung field is divided into three regions such as upper, middle, and lower regions, it often belongs to the upper lung field. When divided into four regions, it often lies approximately in a middle between the top lung field (block region) and the second lung field (block region).

As shown in FIG. 30A, the "apex" of the lung has a thin three-dimensional structure. Therefore, the changes themselves appear small in the images of the present invention. Moreover, overlapping during pathological conditions causes little degradation in image depiction. In the "above-diaphragm" region, the lung structure is three-dimensionally thick. Thus, changes appear strongly in the images of the present invention. Meanwhile, overlapping during pathological conditions results in more pronounced depiction defects. In the "slightly caudal to the apex (subapical)" region, the changes are relatively strong. Overlap during pathological conditions is also relatively strong. For example, differences in lung structure may easily cause left-right differences in thickness. In the "below-diaphragm" region, strong soft-tissue shadows make it difficult to detect numerical differences in individual XP images, complicating evaluation.

Therefore, even in normal lungs, the following phenomena may occur:
- Decrease in the rate of change in the peripheral lung field;
- Attenuation of lung structure around the interlobar fissure;
- Non-uniform changes caused by movement of structures near the hilum;
- Slight signal decrease on the medial side of the right lower lung field;
- Changes in lung thickness at the apex due to anteroposterior chest thickness and pectoralis major;
- Rib bone artifacts; and
- Shadows from only indistinct vascular branches.

In the present disclosure, after detecting the lung field from multiple frame images, the outer contour and diaphragm are used as references for determining the three-dimensional structure of the lungs. That is, the outer contour and diaphragm serve as reference standards for specifying the three-dimensional structure of the lungs, and the lung shape is determined by taking this structure into account. More specifically, because a basic three-dimensional lung field model exists with approximately fixed anteroposterior and lateral ratios, detecting the lung field from multiple frame images allows determination of the three-dimensional lung structure, and hence, the lung shape. Furthermore, the depth of the lung field may be estimated from the degree of signal (pixel value variation) in a normal lung field, enabling determination of the three-dimensional lung structure based on this depth. Further, as shown in FIG. 30A, taking images from multiple directions, e.g., frontal and lateral views, enables identification of the lung field shape. The lungs, whose three-dimensional structure has thus been determined, may then be divided into multiple fan-shaped regions centered at the hilum, and pixel value variations for each region may be calculated.

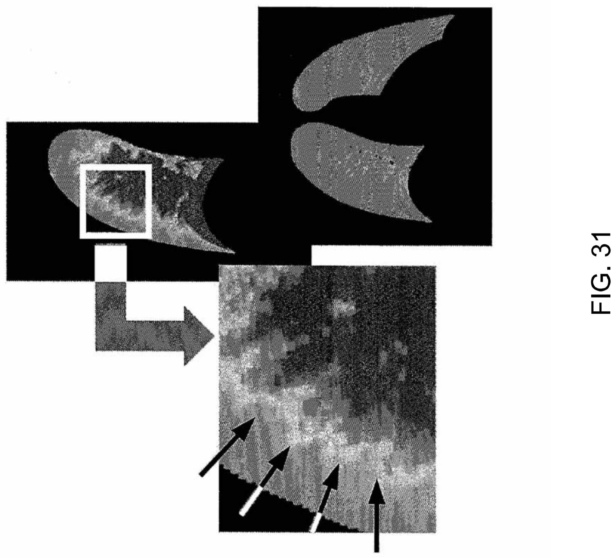

FIG. 31 is a diagram showing a state of a peripheral lung field. In the peripheral lung field, the lobular structure is three-dimensional and complex, which is reflected in the images, resulting in distinctive peripheral variations. An extent of peripheral lung expansion may relatively decrease depending on lung shape, rib depiction, or depiction of the pectoralis major. Visibility of the lung periphery may differ in the depiction ability from peripheral bronchioles to alveoli. This may also vary depending on respiratory depth and conditions. In other words, signal changes appear uniformly according to the respiratory cycle. According to the respiratory cycle, changes occur from the apex toward the diaphragm and from the outer to the inner lung regions, reflecting differences in thickness. However, these are generally symmetrical between left and right. The present invention evaluates whether lung motion is linked with the wave pattern of the respiratory cycle. It evaluates whether the thorax, diaphragm, and ribs move in linkage with wave as the respiratory cycle.

Around the interlobar fissures, decreases in signal (pixel) values are observed. This is because at the edges of the interlobar fissures in the left lower peripheral lung field, alveolar density changes are small, resulting in little signal change. Meanwhile, a slight signal decrease is also seen on the medial side of the right lower lung field, sometimes due to structures below the diaphragm causing negative signals; these are corrected to be regarded as within the normal range. According to the present invention, in order to determine normal images, three-dimensional thoracic structure and physiological respiratory variation rates are combined to be reviewed and interpreted comprehensively. Additionally, by considering various structures and image transformations in XP, it becomes possible to determine various aspects such as partial lung functions.

When machine learning and determination are performed by an AI, a reference wave such as a respiratory cycle is defined, and whether linkage to the reference wave is present becomes a key determination point. That is, linkage with respect to the wave is evaluated from the viewpoints of whether variation is large, small, or absent relative to the wave. When a portion expected to vary greatly varies little, or a portion expected to vary little varies greatly, an abnormality may be determined. A phase shift relative to the reference wave may also be determined as an abnormality. In other words, a lung having certain linkage to the wave formed by the respiratory cycle is regarded as a normal lung. Training an AI with this yields a model of a normal lung. In determination, the Al determines whether the linkage is maintained or lost. There may be cases where amplitude is abnormal or a phase is abnormal.

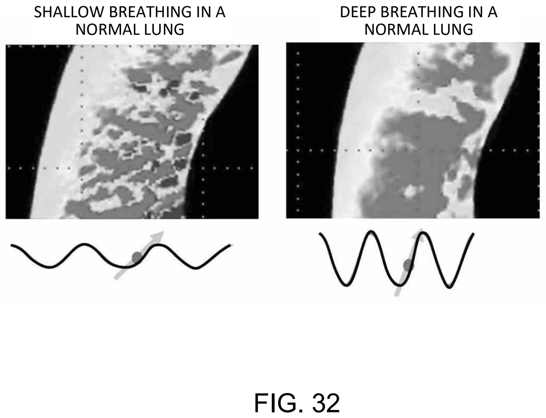

FIG. 32 is a diagram showing shallow respiration and deep respiration of a normal lung. In shallow respiration of a normal lung, the slope of the change wave is small and the variation is limited, so depiction of the periphery (from peripheral bronchioles to alveoli) is not achieved. In this case, only the bronchi to the bronchioles are depicted. In contrast, during deep respiration of a normal lung, the slope of the change wave is large and the variation is sufficient, so depiction of the periphery (from peripheral bronchioles to alveoli) is achieved. In deep respiration of an abnormal lung, motion lags relative to diaphragm motion. Thus, in the present invention, evaluation is based on "linkage" with respect to respiration, enabling diagnosis by linkage.

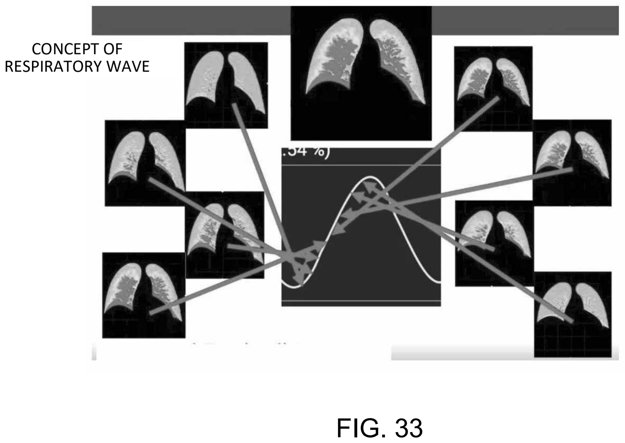

FIG. 33 is a diagram showing a movement of a respiratory wave. When the waveform slope is large, visualization on the image becomes clearer. This is because all components are linked to the wave shape and its slope. In a normal lung, the diaphragm, thorax, trachea, and signal manifestation behave similarly. Graphing the degree of conformity to the wave enables diagnosis.

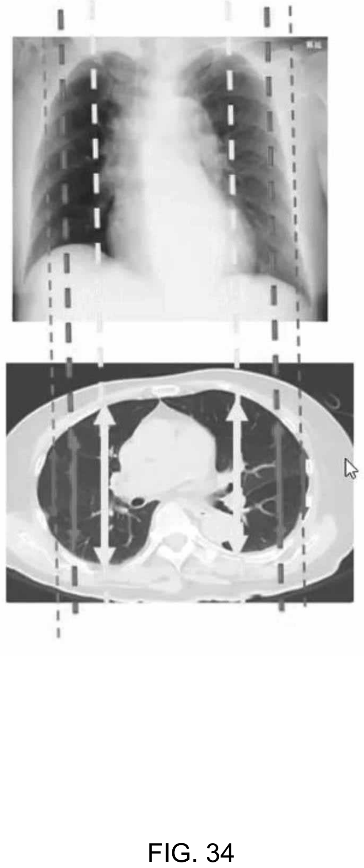

Moreover, as a three-dimensional structure, the anterior-posterior lung thickness may be determined. For example, in chest XP images, CT is expressed as tomographic slices, whereas a projection image represents a superposition of three-dimensional structures. FIG. 34 is a diagram showing a three-dimensional structure and a cross-section based on a chest XP image. The outer lung regions are three-dimensionally thin, resulting in smaller image changes. When a lesion is present, depiction defects due to overlap are relatively small. The inner lung regions are thicker, yielding larger image changes. When a lesion is present, depiction defects due to overlap become more pronounced. In the slightly inner (outer-middle) regions, image changes are relatively large, and, when lesions exist, depiction defects due to overlap are also relatively large.

FIG. 30 described above is a diagram showing a lateral view of the lungs. Similarly, in the lateral view, anteroposterior thickness is relevant. Through the lateral view, whether pixel values increase within the three-dimensional structure or not may be grasped. The lateral view may display whether each structure appears to expand or not according to the three-dimensional lung structure. In shallow respiration, lung expansion is small; therefore, signals are weak. However, based on the wave, the expansion may be visualized by assessing the slope, which may aid diagnosis. Regarding the structure of the trachea itself, it should be easier to find when it begins to expand (because the gradient of the change is large); therefore, if the expansion cannot be seen, it should be considered to be abnormal, enabling prediction of abnormal possibility. Similarly, when expected visualization is absent on the central tracheal side, it may be determined as abnormal. Amplitude varies along the three-dimensional structure and the wave shape. Therefore, when the amplitude does not follow, it may be determined as abnormality. Based on the similarity of amplitude, and if the amplitude is not similar, t may be determined as abnormality.

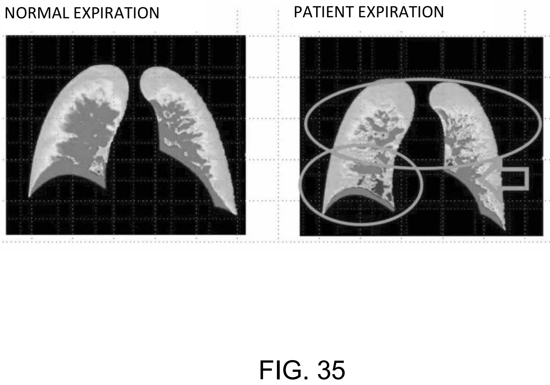

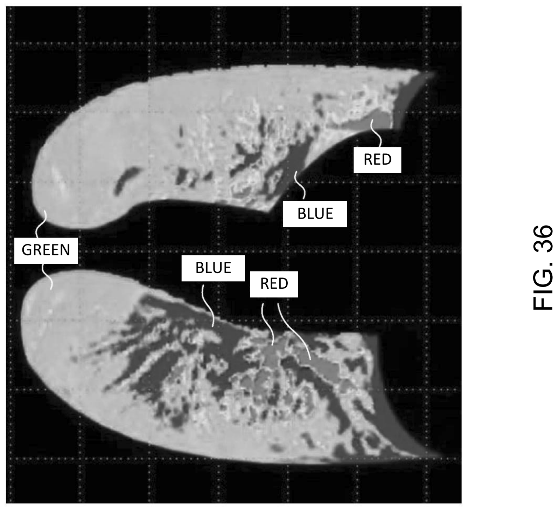

FIG. 35 is a diagram representing normal lungs and lungs of a patient. FIG. 36 is a diagram showing lungs of a patient. In these lung images, "red" regions indicate increased pixel values and represent exhalation. Both lung area and volume decrease, yielding positive pixel values. "Blue" regions indicate decreased pixel values and represent inhalation. Both lung area and volume increase, yielding negative pixel values. These allow abnormalities to be displayed during motion. In lungs of a patient, blue may appear when red should appear, or red may appear when blue should appear. In other words, motion opposite to that originally expected is exhibited, enabling determination of an abnormality. Moreover, a case in which diaphragm motion occurs without color change may also be determined as abnormal. For example, after an AI learns images of normal lung motion to construct a model, the presence of lesions may be determined in the image of the patient's lungs. Optionally, abnormality may also be determined by quantifying features of normal lung images and comparing them with quantified features in the images of the patient's lungs.

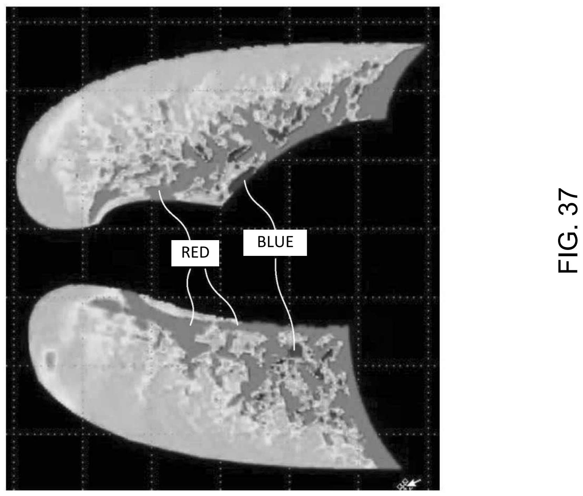

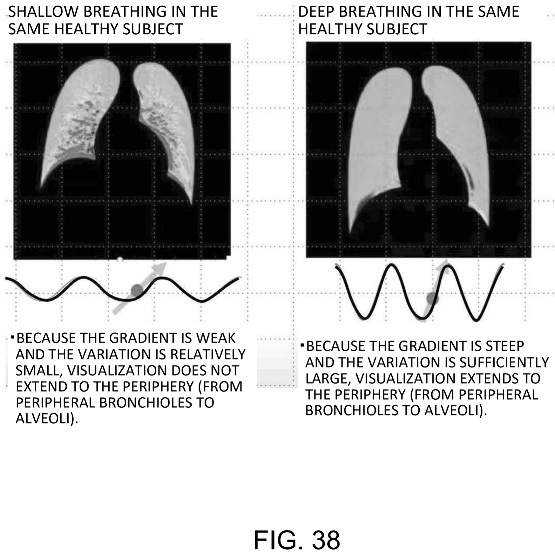

FIG. 37 is a diagram showing lungs of a patient. In this case, central vascular structures of the lung are visible, whereas peripheral vascular structures are not. Peripheral alveoli are not moving. A normal lung exhibits waveform correlation, whereas an abnormal lung lacks waveform correlation. This may be learned and determined by an AI. Further, it may be quantified within a certain area, and how much it differs may be understood. FIG. 38 is a diagram showing a contrast between deep respiration and shallow respiration. Images are faint during shallow respiration and dense during deep respiration. In accordance with the wave shape, segmentation is computed and graphed.

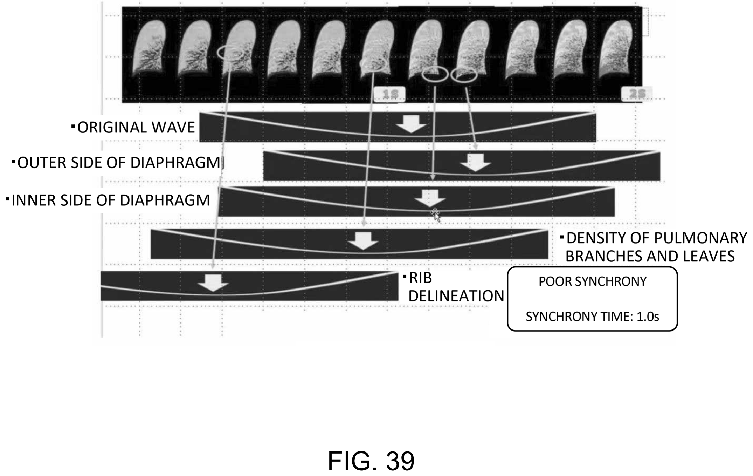

FIG. 39 is a diagram showing waves of a diaphragm and the like being shifted relative to an original wave. These shifts are based on phase differences. For example, in lungs of a patient with interstitial pneumonia, the phase is shifted relative to the original wave, as shown in FIG. 39. This phase shift is quantified. This is particularly applicable to heart. When neurotransmission is delayed, myocardial motion occurs with a delay. This delay in myocardial motion corresponds to a phase delay. These phase shifts are quantified and visualized in pixel form. When a delay in neurotransmission (impairment) is observed, there is a high possibility of developing myocardial infarction within one or two years. In other words, whether the wave amplitude or the wave phase is shifted is quantified or pixelized.

In cases of COPD, the phase of pixel value changes in lungs is also shifted. Motion is shifted relative to the lung apex, and responses are poor. Patients with interstitial pneumonia also exhibit phase shifts in changes of lung pixel values. By contrast, normal people have no phase shift. Such phase shift patterns may be learned and determined by an AI. Since these are particularly difficult to recognize visually, quantification, AI training, and determination are useful.

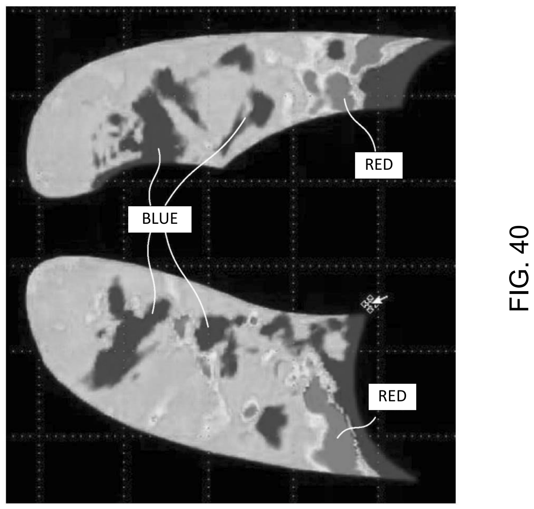

FIG. 40 is a diagram showing an image of a pneumonia patient. It appears such that some lung structure is lost. The vascular structures, which normally radiate from the hilum, appear quite randomly. This may be determined as abnormal. Further, in patients with partial pleural effusion, vascular structures are also displayed randomly as in FIG. 40. In such cases, white spots appear also on X-ray images, allowing physicians to make a diagnosis in combination with these findings. In other words, comprehensive diagnosis is possible using images of respiration alone, images of blood flow alone, combined images of these, and with X-ray images. For asthma cases as well, an asthma determination model may be created through deep learning by an AI. In asthma cases, the heart rate increases during an attack, and pixels indicating respiration and blood flow deviate from normal values, resulting in an overall mottled image. By deep learning of such case images with an AI, a determination model may be constructed.

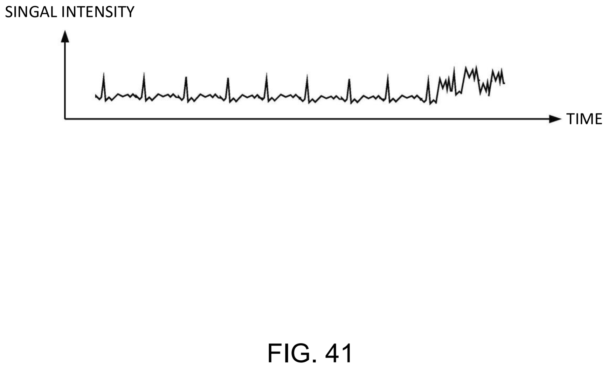

FIG. 41 is a diagram showing an example of an electrocardiogram. As shown in FIG. 41, in addition to the pulse portions appearing at regular intervals, signals with shifted cycles may be displayed. This mainly occurs because muscle movement, lung motion, and rib creaking enter ECG data as noise. That is, ECG data is affected by other organs or body parts, resulting in deviations. Conventionally, these deviations have been manually corrected, but according to the present invention, this correction is automated. In other words, these data are treated as "frequency tunable data" and processed as frequency-synchronized images. More specifically, these data are Fourier-transformed, only the frequencies of periodic pulse signals appearing in the ECG are extracted by filtering, and then an inverse Fourier transform is performed to obtain noise-free ECG data. Noise from respiration may likewise be removed.

### [Lungs of Patient with Heart Failure]

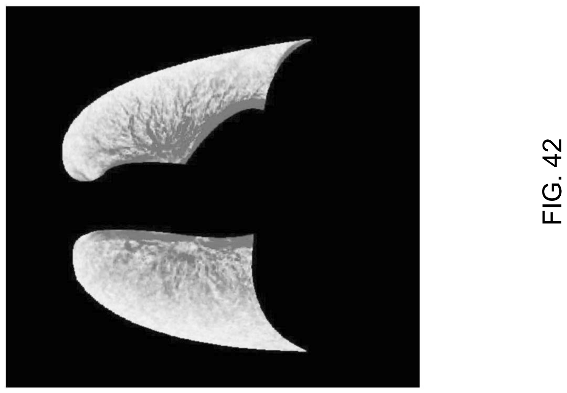

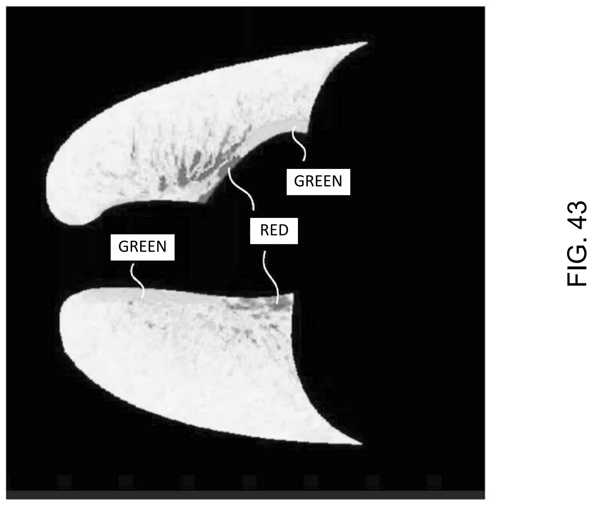

FIGS. 42 and 43 are diagrams showing lungs of a patient with heart failure. FIG. 42 shows an example of an early portion of the first half of single heartbeat, and FIG. 43 shows an example of a later portion of the first half of single heartbeat. A normal vascular structure extends radially from the center of the lung toward the periphery, with few branches. However, in case of heart failure patients, branching of pulmonary vessels exhibits an "irregular shape." That is, in lungs with abnormalities due to heart failure, the number of radial lines in the vascular structure decreases and vessels taper sharply in the periphery. In other words, the vascular structure becomes sparse and irregular, and peripheral vessels are extremely thin. Although the distribution appears uniform overall, on closer inspection the vascular structure is "sparse" and "diffusely sparse." This differs from normal. Capillary and peripheral pulmonary vascular flow may be observed, but an overall delay in blood flow is observed. Overall motion is shifted, and the blood flow wave exhibits phase delay. Moreover, as shown in FIG. 43, blood flow is observed even during a period when flow should have ended. This indicates delayed blood flow from the pulmonary hilum. Blood flow is observed at a time when it would not be observed in a normal lung. These phenomena are visualized as differences from normal.

Furthermore, the extent of vascular shadow propagation between frames is measured as pulmonary vascular pressure. For example, based on depiction of vascular shadows between frames for vessels from the pulmonary hilum to the lower or middle lung fields, the "distance" is taken as the linearized distance along the pulmonary vessels (CM to m), and dividing this distance by the frame interval time (e.g., 0.065 seconds) yields the velocity of blood flow through the vessels. By thus determining the velocity of blood flow through the vessels, pulmonary arterial pressure may be estimated. Specifically, by combining "4·V²" with a standard fixed value of 5 or 10 mmHg and other "modarity" such as inferior vena cava diameter from ultrasound examination, estimated pulmonary arterial systolic pressure may be measured, contributing to pulmonary hypertension diagnosis and estimation of pulmonary capillary wedge pressure. Furthermore, by standardizing based on a standard AP image and a "custamized" total lung "volume" in subapical regions, lung ventilation volumes may be measured based on signal ratios. Thus, various functions such as cardiac function, pulmonary blood flow, and overall respiratory function may be calculated from inter-image data.

### [Tomographic Imaging in 3D]

In three-dimensional image display techniques, a projection image based on three-dimensional information is displayed on an image display device such as an LCD, which is a two-dimensional plane. This three-dimensional image display technique is classified into projection methods onto a two-dimensional plane serving as the display surface (parallel projection and perspective projection) and image display methods (surface rendering, volume rendering, maximum intensity projection, minimum intensity projection, and summation projection, etc.). Furthermore, a multiplanar reconstruction (MPR) method, in which any cross-section is displayed without using a projection method, evoking a three-dimensional impression to an observer, is understood as included in three-dimensional display schemes.

Conventionally, in IVR (Interventional Radiology), it has been common that a subject is transported to a CT suite for tomographic imaging, or an IVR-CT apparatus is used. Here, a C-arm CT imaging technique using an angiography system equipped with a C-arm obtains tomographic images in situ during IVR, thereby avoiding risks of transportation and achieving superior cost performance in cost and operation relative to the IVR-CT apparatus. A technique for reconstructing tomograms from rotational images by a C-arm includes a 3D angiography technique; whereas the 3D angiography technique mainly depicts contrast-enhanced vessels, C-armed CT imaging provides very high low-contrast resolution and may depict faint tumor enhancement.

In image processing for C-armed CT imaging, a tomographic image is obtained through three stages of processing for acquired rotational images: conversion process to water permeation length, pre-reconstruction process, and reconstruction process. In the conversion process to water permeation length in the first stage, pixel values of the rotational images are converted into water permeation lengths. The water permeation length is a quantity obtained by converting a pixel value into the number of millimeters of water that it permeates when the X-ray conditions are the same. Specifically, after the gain of the detector is corrected, the scattered ray component is corrected by the scattered ray correction process, and converted into the water permeation length in a beam hardening correction process. Dark regions in rotated images indicate high X-ray absorption, which results in a longer water permeation length, making the converted image appear as if negative-positive contrast is inverted.

In the subsequent pre-reconstruction process, four types of corrections are performed: C-arm trajectory correction, ring artifact correction, truncation correction, and π correction. The C-arm trajectory correction corrects a deviation between an actual C-arm trajectory and an ideal theoretical trajectory. The ring artifact correction reduces ring-shaped artifacts in tomograms that hinder image observation. The truncation correction improves tomographic flatness by interpolating sinograms for object portions that extend beyond the field of view during rotation. The π correction compensates for differences in acquired data amounts between central and peripheral regions during half-scan reconstruction due to cone-beam effects. After these corrections, tomograms are computed by reconstruction processing. The reconstruction algorithm is referred to as the filtered back projection (FBP) method.

In C-armed CT imaging, images are captured from various angles over approximately 30 minutes. For example, because the respiratory cycle is not constant, the images are corrected using reconstruction. Here, regions near the X-ray tube are clearly visualized, whereas distant regions are not. Raising tube voltage may improve visibility, but it requires taking the difference while preserving the difference in transparency and the noise to be removed. For example, by observing a near region, then observing the same region from the rear, and subtracting, both front and back may be visualized. Increasing voltage may visualize everything; however, it may cause confusion; therefore, the near side is subtracted to facilitate visualization of the far side, or conversely the far side is subtracted to facilitate visualization of the near side.

Conventionally, no method has been realized that enables visualization while correcting images in a breathing state. In the present invention, inhalation and exhalation motions are aligned to a constant pattern. Phase and frequency are matched, and reconstructed. That is, under multiple conditions, multiple images acquired from multiple directions are processed mutually with varied conditions to create tomograms. This is then used in radiographic imaging. Namely, tomograms in frequency-analyzed radiographic images are generated. Finally, fine adjustment by reconstruction is performed. For a particular organ, tomographic images are created at multiple locations and under multiple conditions. For example, with moving lungs and blood flow, waveforms are matched based on phase. Phase for respiration is also matched. When visualized, images are aligned and stereoscopic presentation are provided. Temporal alignment (synchronization) is also required for blood flow. In this case, for example, images are acquired for 10 heartbeats each and then phases are matched.

According to the present invention, since images are captured while the organ is in motion, the frequency characteristics of the organ are taken into consideration. Therefore, temporal and phasic registration, phase alignment, and further angular adjustments are performed. In C-armed CT imaging, the imaging position relative to the organ is not exactly the same for each capturing; therefore, positional and angular registration is required. For example, when attempting to capture images of the lung at an angle of 45 degrees from the right anterolateral direction, the actual angle may be 43 degrees or 49 degrees. Such deviations need to be finely adjusted based on the positional relationship with the ribs and thorax.

That is, positional registration is performed so that CT images, C-armed CT cross-sectional images, and XP images acquired from various angles match pre-acquired three-dimensional images of structures such as vertebral bodies, the heart and mediastinum, ribs, and bronchi, including their positions and angles. The order of priority for matching is "vertebral bodies, heart/mediastinum, ribs." In particular, higher weighting is given to matching the angle and position of vertebral bodies, while lower weighting is applied to ribs, which are affected by pulmonary motion. Using AI or similar techniques, the most consistent angle and position relationships for each acquired XP image are identified as image patterns, and cross-sectional, three-dimensional, or four-dimensional structures are generated based on that information. Particularly, since CT images are "inspiratory images" captured in a state of inhalation, they are considered to match most closely when aligned with XP images captured during maximal inspiration. When XP images are captured from multiple angles but subject-specific CT or C-arm CT tomograms are not available in advance, standard image models may be calculated from information such as age and height, and the resulting data are used with fine adjustments to generate cross-sectional and three-dimensional structures corresponding to respiratory or cardiac organ motion.

Furthermore, albumin may be injected into a patient to intentionally create a state of fine pulmonary thrombosis. In a normal lung, albumin distributes throughout the entire lung. In a case of pulmonary thromboembolism, the peripheral vessels are occluded and albumin does not reach them. As a result, inhomogeneities appear in the images. When observed in an integrated three-dimensional structure, albumin is seen in the anterior part (front side) of the lung but not in the posterior part (back side). Then, tomographic images are generated from the three-dimensional structure. In the image, if there is a large phase delay, the flow does not propagate smoothly, indicating abnormality.

In the case of an AP (anterior-posterior) X-ray, (1) it is observed in the lungs and anterior (A) side, possibly due to the closeness to the X-ray tube and the distance to the detector. (2) Because images are captured multiple times, low-dose conditions may make it difficult for radiation to reach deeper to the posterior (P side). For these reasons, in default fluoroscopic imaging, the side closer to the tube (A side in AP imaging, P side in PA imaging) is more strongly represented, whereas the detector-side (farther from the tube, i.e., P side in AP or A side in PA) exhibits relatively weaker signal and less identifiable changes than the tube-side. Therefore, (1) by performing similar imaging at higher exposure (yielding more uniform image quality and emphasizing the P side) together with conventional low-dose imaging, anterior, overall, and posterior imaging may be differentiated. (2) By AP imaging and PA imaging, an image of each side may be captured. (3) In regions such as the dorsal lower lobe, signal delays may occur, increasing phase signal differences. (4) During respiration, posterior images may be more easily visualized compared to breath-holding conditions. (5) Changes are more likely to appear during deep breathing compared with shallow or normal breathing.

By utilizing the above tendencies and superimposing the two (superimposing AP and PA images, objectively depicting the overall signal ratio), and also by subtracting the above, images may be captured to emphasize the anterior (A side) or the posterior (P side) depending on conditions such as "whether the anterior should be prioritized or the entire image should be positive," "the posterior should be prioritized slightly," or "those that are more visible due to higher voltage (higher voltage > lower voltage)." Further, images are captured from multiple directions including RAO, LAO, and RL directions. Furthermore, by capturing images while rotating or spiraling, multiple and varied images may be captured from the tube side. Since respiration is not constant, standardized expansion, contraction, and temporal reconstruction based on a master waveform may be used to overlay a 3D tomographic image of a standard lung or patient captured previously or simultaneously, matching signals in position and relative phase, making it possible to image approximate respiratory and blood flow components in each cross section. Moreover, multi-angle transmissive imaging enables multi-directional 4D images of three dimensions over time and signals at each cross section.

### [Method of Applying Pulses to Human Body]

ASL (arterial spin labeling) is an imaging method to obtain a perfusion image and a relative cerebral blood flow image without using a contrast agent by labeling arterial blood flowing into an imaging slice with an RF pulse. An RF pulse serves as an impact signal, and when applied periodically, it functions as a marking, allowing identification of how far it has circulated in a neck. For example, when an RF pulse is applied to a neck, a pulse may diffuse throughout a head. Normally, pulse diffuses, but there may be a case where it does not diffuse. Here, frequency tunability inside a head may be recognized and estimated, enabling assessment of whether blood flow is facilitated or impeded. Since a frequency applied to a human body is definite, deviation or delay from the frequency may be used to determine whether a condition is normal or abnormal.

### [Representation by Sine Wave]

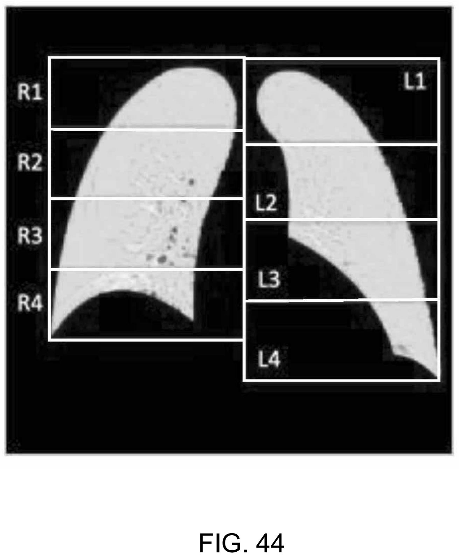

As shown in FIG. 44, in an image of the lungs, an analysis range was divided into four regions, and the following method was used to verify whether a difference appears in signal changes in each region. In other words, "signal change: f(x)" was approximated by "g(x) = A * sin(Cx + B)," and correlations between each parameter and an FEV% predicted value were examined.

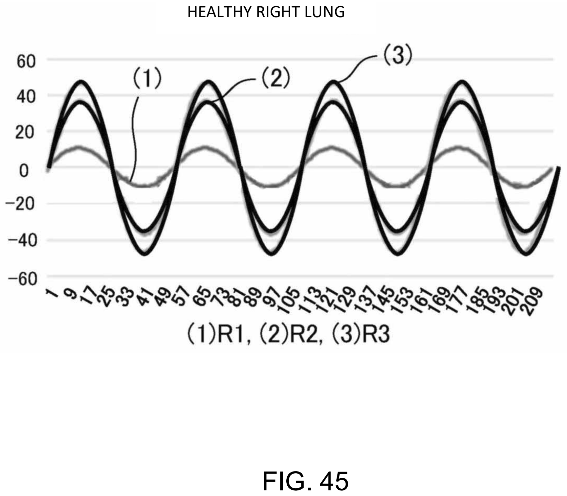

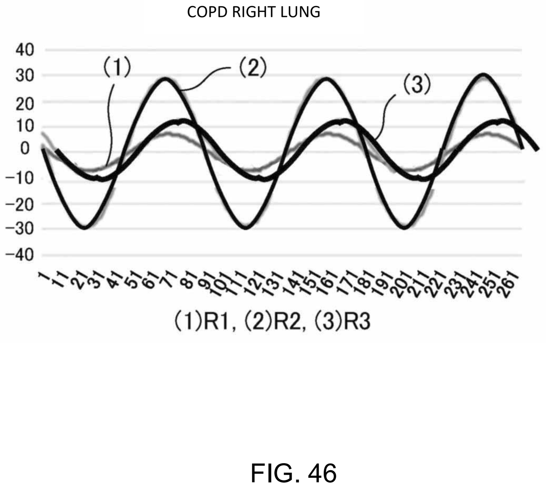

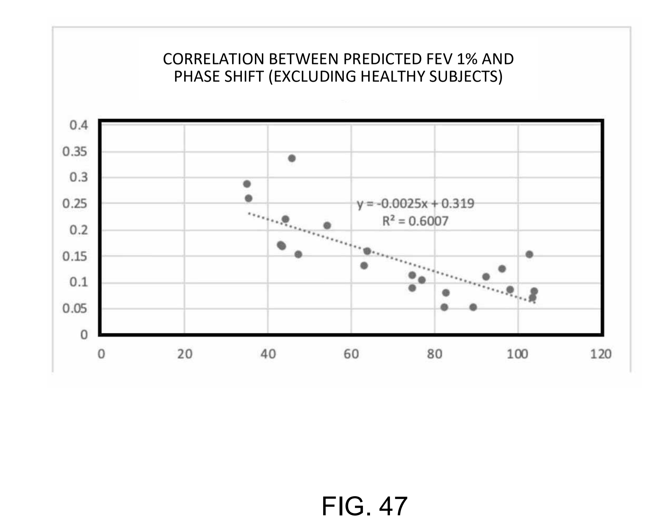

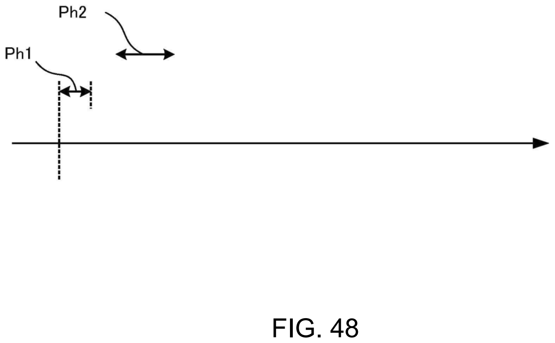

As a result, as shown in FIGS. 45-48, in a healthy lung, the amplitude showed a tendency to increase in an order "R4-R3-R2-R1," and phases tended to coincide. In contrast, in COPD cases, visual inspection confirmed variability in both amplitude magnitude and phase. From examination of sine-wave approximation results, a phase shift was observed to have a negative correlation with the FEV% predicted value (R = -0.775). When calculating the correlation, one point was excluded as an outlier, which is thought to be due to the way the lung field (analysis range) was created. Meanwhile, the diaphragm and rib artifacts mainly affected the amplitude, and no tendency for them to contribute to phase shift was observed. At least, it is considered that there is no need to actively exclude R4/L4 when evaluating the phase shifts. On the other hand, when performing any evaluation using amplitude, it is necessary to note that artifacts may influence the result.

Moreover, as described above, dots on the graph may be used solely as data values, or discrete information may be converted into a waveform by specifying wave information (amplitude, phase, and cycle) and used as values of a sine wave. An organ-specific waveform may be obtained, and multiple different waveforms may be obtained even for the same organ; these waveforms may be replaced with a sine wave to compute phase and amplitude for waveform-based evaluation. Information between dots may also be obtained by drawing a sine wave. Note that, as shown in FIG. 13L, a waveform such as a pulmonary arterial waveform is not limited to a sine wave. According to the present invention, the waveform may also be treated as an organ-specific waveform within a single cycle.

### [Estimation of Pulmonary Blood Pressure]

Conventionally, measurement of pulmonary blood pressure requires catheter insertion and is highly invasive. According to the present invention, waveform data may be obtained from a part of an organ, and an average over a portion exhibits a sine wave. This sine wave is considered to be correlated with pulmonary blood pressure. That is, image analysis according to the present invention enables computation of a pulmonary blood pressure waveform based on a waveform frequency, phase, and signal value. Here, a periodic motion synchronized with a heartbeat is extracted and visualized. In doing so, changes synchronized with blood flow in the image are tracked. For example, a sine wave does not propagate to a pulmonary hilum or a periphery. Moreover, a delay from a lung center to an outer side occurs. Accordingly, a blood flow velocity may be calculated from the delay. Pulmonary blood pressure may be estimated from vessel caliber and flow velocity. Furthermore, vessel wall thickness, phase shift, differences in distributed patterns, respiratory state, and other factors described herein may be used to grasp pathophysiology associated with heart failure and to assess disease extent for whole or partial conditions. For example, by training an AI to learn values such as a cardiac index and a pulmonary arterial wedge pressure, guidance for treatment strategy of heart failure may be supported. By calculating the extent and proportion of respiratory motion and superimposing respiratory coefficients from an apex to a diaphragm and from an outer to an inner region, an estimated respiratory function may be computed. Further, by measuring a mismatch coefficient in matching between a respiratory function constant and a signal of blood flow, a state of ventilation-perfusion mismatch or imbalance, or a state where both ventilation and perfusion are impaired (corresponding to a lung injury-related pulmonary perfusion disorder) may be predicted. In other words, it may be classified into "good ventilation/good perfusion," "good ventilation/abnormal perfusion," "abnormal ventilation/good perfusion," and "abnormal ventilation/abnormal perfusion," enabling determination of pathological status and countermeasures for treatment strategy according to severity.

A pulmonary blood flow waveform calculated in accordance with the present invention is a numerical graph representing a degree of change in an arbitrary region, and it approximates a pulmonary arterial waveform recorded during an actual right heart catheterization. A portion of a pulmonary arterial pressure waveform after a pulmonary valve closure notch corresponds to a right ventricular diastolic phase, and an angle until the waveform returns to a baseline reflects pulmonary vascular resistance. By utilizing this characteristic, differences between a high PVR group and a low PVR group, namely between CpcPH patients and IpcPH patients, may be detected with respect to the relationship between a pulmonary blood flow waveform calculated in the present invention and a diastolic angle of an actual pulmonary arterial pressure waveform.

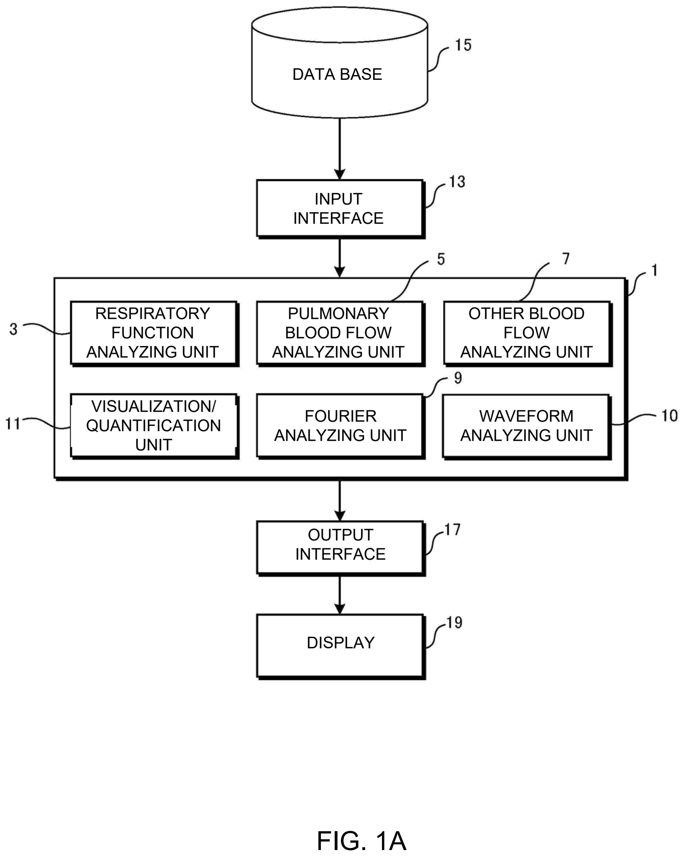

Embodiments of the present invention are described below with reference to the drawings. FIG. 1A is a diagram showing an outline configuration of a diagnosis assistance system according to the present embodiment. The diagnosis assistance system exhibits specific functions by causing a computer to execute a diagnosis assistance program. A basic module 1 includes a respiratory function analyzing unit 3, a pulmonary blood flow analyzing unit 5, another blood flow analyzing unit 7, a Fourier analyzing unit 9, a waveform analyzing unit 10, and a visualization/quantification unit 11. The basic module 1 acquires image data from a database 15 through an input interface 13. The database 15 stores, for example, images in DICOM (Digital Imaging and COmmunication in Medicine) format. An image signal output from the basic module 1 is displayed on a display 19 through an output interface 17. Next, functions of the basic module according to the present embodiment are described.

### [Analysis of Respiratory Element Cycle]

In the present embodiment, a cycle of respiratory elements is analyzed based on the following indices. The term "respiratory element" refers to a concept including all or part of expiration or inspiration as described above. That is, at least one frequency of a respiratory element is analyzed using at least one of "density"/"intensity" in a certain region inside a lung field, diaphragm motion, and thoracic motion. The "at least one frequency of a respiratory element" refers to a concept including one or more frequency spectra of a respiratory element and may include a case having a certain bandwidth. In the present embodiment, a lung field is considered as a set of blocks, multiple frequencies are extracted from each block, and these are processed as a frequency group. As described above, the base data includes both concepts of "wave form itself" and "wave interval (frequency: Hz)," which may therefore be processed as wave form. Optionally, data obtained from other measurement methods such as a spirogram or external input information, or a range composed of a certain volume "density"/"intensity" measured at a site with high transmissivity in X-rays (or other modalities such as CT or MRI), may be used.

Improving data accuracy by comparing analysis results for each breath and analyzing trends from multiple data sets is also possible.

Further, a respiratory element may be corrected by changing the phase of at least one frequency of the respiratory element or by smoothing its waveform. In this case, motions such as (motion of thorax and diaphragm), (motion of thorax), (density), (precision pulmonary function), and (thoracic sensors) are used to align phases with the wave. Moreover, an average "density" of a lung field is tracked, and the final change is approximated using, for example, a least-squares method on the wave form to identify the wave. Here, in a case of "density" of a chest, since the most variable value is the "density" of a lung, lung "density" change may be evaluated by evaluating overall "density" of a displayed screen. When plotting a wave, a phase shift may occur between actual motion and measured values. In such a case, the phase may be corrected using the positions of maximum and minimum values or the overall wave form.

### [Waveform Analysis]

Frequency components of a waveform may be calculated based on a waveform of a respiratory element. This allows the above-described "waveform tunability image" to be acquired. Specifically, a waveform of a site within an analysis range is identified, frequency components of the identified waveform are extracted, and an image corresponding to the frequency components of the waveform is output.

### [Cardiovascular Beat analysis and Vascular Beat Analysis]

In the present embodiment, cardiovascular beat and vascular beat are analyzed based on the following indices. Specifically, a heart, a pulmonary hilum position, and major vessels are identified from electrocardiogram or pulse meter measurements obtained by other modalities or from a lung contour, and vascular beats are analyzed using changes in "density"/"intensity" of each site. Optionally, changes in "density"/"intensity" of a target site may be analyzed by manually plotting points on an image. At least one frequency (waveform) of a cardiovascular beat element obtained from a heartbeat or a vascular beat is identified. Preferably, data accuracy may be improved by comparing analysis results for each beat and analyzing trends from multiple data sets. Accuracy may also be improved by extracting "density"/"intensity" of each site multiple times or over a certain range. Further, inputting a cardiovascular beat frequency or frequency band may be suggested.

### [Identifying Lung Fields]

Images are extracted from a database (DICOM), and a lung contour is automatically detected using the results of the above analysis of respiratory element cycle. For this automatic detection of a lung contour, known techniques may be used. For example, techniques disclosed in Japanese Patent Laid-Open Publication No. S63-240832 or Japanese Patent Laid-Open Publication No. H2-250180 may be used. Next, the lung field is divided into a plurality of block regions, and changes in each block region are calculated. Optionally, the size of a block region may be determined according to a capturing speed. When the capturing speed is low, identifying a corresponding site between successive frame images may become difficult; therefore, the block region is enlarged. On the other hand, when the capturing speed is high, the number of frame images per unit time is large, allowing even a small block region to be tracked. Optionally, the size of a block region may also be calculated according to which timing of a respiratory element cycle is selected. In some cases, it is necessary to correct deviations of lung field regions. In such a case, thoracic motion, diaphragmatic motion, and positional relationships of blood vessels in the entire lung field are identified, and relative positions of lung contours are grasped and evaluated based on their motions. If a block region is too small, image flicker may occur. In order to prevent this, a block region needs to have a certain size.

A lung field automatically detected as described above may be represented as coordinates of points and control points by using at least one Bezier curve. The lung field may also be represented by using a plurality of so-called "simple closed curves" enclosed by at least one Bezier curve. Similarly, an analysis target may be represented using one or more simple closed curves.

A lung field of each frame may be detected using at least one Bezier curve detected on a lung field in a specific frame. For example, one method is to detect the two, a maximum and a minimum, lung fields and use these two lung fields to calculate the lung fields of other frames. Here, a variable called "change rate" is defined for the other frames. The "change rate" represents the size of the lung field, i.e., a respiratory state, and may be calculated from a diaphragm position, an average "intensity" of an entire image, and the like. The change rate may also be calculated using measurement data from external devices such as a spirograph or using a modeled change rate. As such, the "change rate" may be freely defined, and the calculation may be performed, for example, by assuming that the lung field changes at fixed ratios (10%, 20%, 30%, etc.). The change rate defined in this manner may contain errors, so subsequent processing may be performed using the results of automatic or manual error removal, or the results of approximation using the least squares method, etc. Assuming that the lung field deforms linearly between the maximum to the minimum, the lung field in each frame is calculated by applying linear transformation techniques using change rates of respective frames.

The above-described processing may also be applied to any range of consecutive frames. For example, in respiration, the lung field repeatedly changes between its maximum and minimum states; however, in actual measurements, the shape at the maximum is not always constant. By applying the above processing to each range from the maximum to the minimum and from the minimum to the maximum, the lung field may be calculated with higher accuracy than by defining only the maximum and the minimum lung fields for computation. Although the specific example described here uses the maximum and the minimum, the present invention is not limited to this but may be applied to "any range." Therefore, it is also possible to perform the measurement at positions midway through breathing such as 0% and 30%, or 30% and 100%.

Although accuracy may decrease, calculating the lung field of each frame from a single lung field is also possible. For example, the change vector of the lung field may be determined by estimation based on the shape of the thorax. Specifically, a method in which a change vector is defined for each control point of a Bezier curve is employed; however, the invention is not limited to this method. Then, the detected single lung field, the change vector, and the change rate of each frame are used to calculate the lung field in each frame. Automatic or manual correction of the calculated result may further improve accuracy. The above method is also effective in 3D images. That is, even in 3D images, detection of lung fields in other frames using at least one Bezier surface detected on a lung field in a specific frame may be processed. Thereby, lung field images between frames may be obtained.

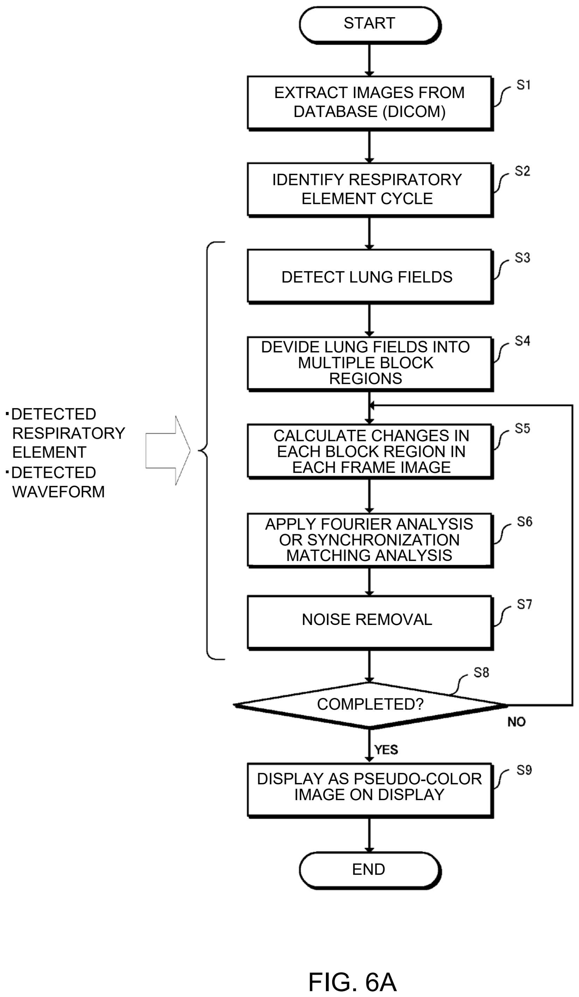

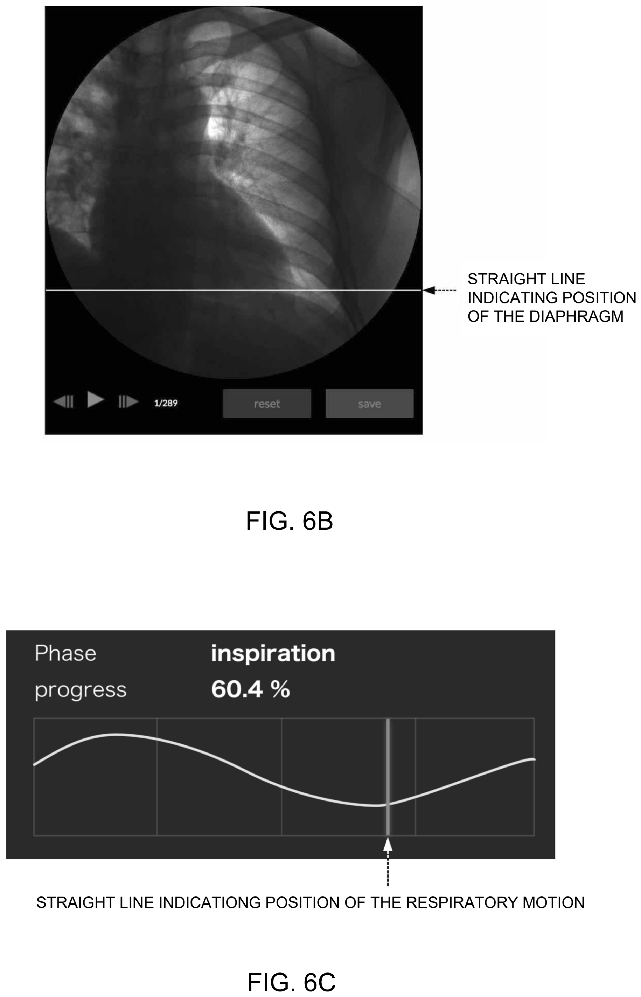

FIG. 6C is a graph showing a cycle of a respiratory element. In the image of FIG. 6C, a white vertical line is shown, which indicates the current position within the respiratory element cycle. The line moves in accordance with the motion of the lung video shown in FIG. 6B to indicate the current position within the respiratory element cycle. By indicating the current position within the respiratory element cycle, the current position in the lung motion cycle may be clearly understood. In the present invention, not only the respiratory element cycle but also items linked to lung motion, such as blood flow "density," the thorax, and the diaphragm, may each be expressed in a graph.

When a subject "holds a breath," the respiratory element frequency may not be identifiable. In this case, Fourier analysis described later is performed using at least one frequency of a cardiovascular beat element extracted from the subject's heartbeat or vascular beat. In this case, the method of dividing the block regions described later may be varied according to the movement of dynamic parts associated with the heart, diaphragm, or respiration.

### [Detection and Evaluation of Boundaries]

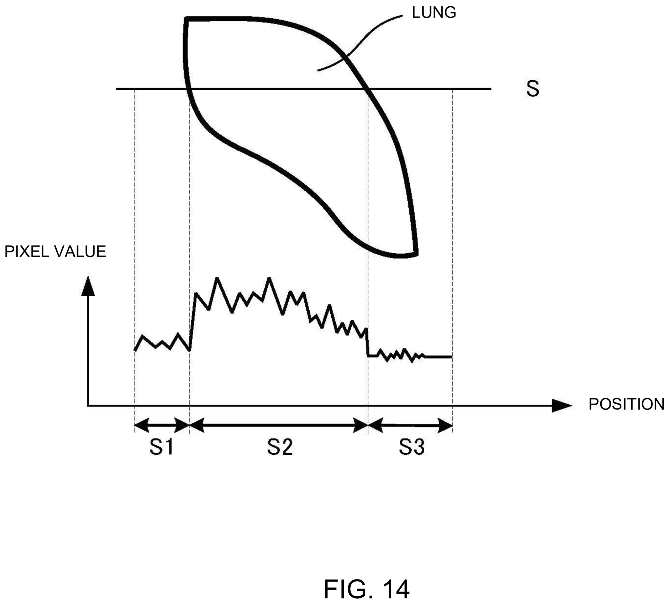

The present invention enables detection and evaluation of lung boundaries. For example, once the lung field is calculated by the above method, the position and shape of the boundary may be detected again with high accuracy. Within the calculated lung field, a point is plotted at any position, lines are extended in all directions from the point, and changes in pixel values along each line are evaluated. For example, FIG. 14 shows that, when pixel values are calculated along a line segment S cutting across the lung, pixel values vary significantly at the boundary, but their absolute values differ. For example, adjusting thresholds for detecting the left and right boundaries may improve boundary detection accuracy. The characteristics of pixel value variations in each region may also be utilized. As shown in FIG. 14, even if the difference between the edges of region S2 and region S3 is small, the edges may be identified from the variance of pixel value variations. Although variance is focused on here, the invention is not limited to this.

Furthermore, by applying the same concept, detecting boundaries of analysis regions of organs, blood vessels, tumors, and the like other than the lung is possible. For example, when a contrast medium is present in a blood vessel, the inside of the vessel may be clearly visualized, but calculating the outer side and thickness of the vessel clearly is not easy. The present embodiment enables accurate detection of the boundaries; therefore, the shape and features of a blood vessel within an analysis range may be calculated. This enables the thickness and circumference of blood vessels to be grasped, which were conventionally difficult to grasp, and may be used diagnosis.

### [Creating Block Regions]

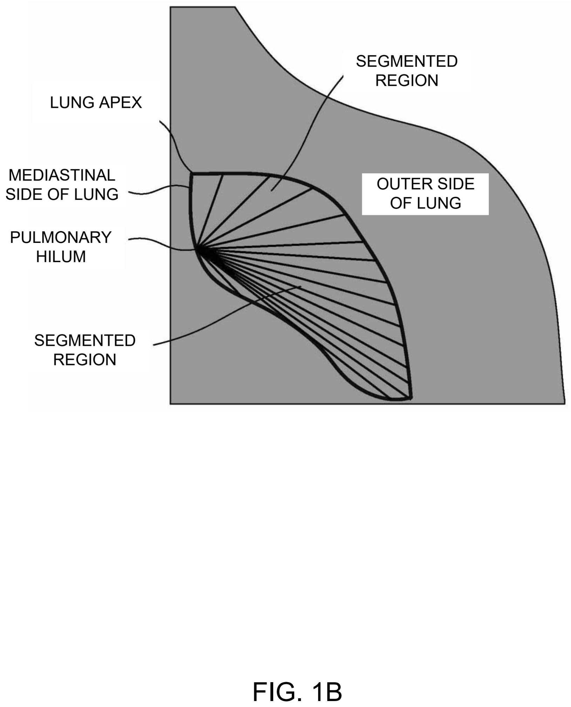

A method of dividing a lung field into multiple block regions is described below. FIG. 1B is a diagram showing an example of the method for dividing a lung field radially from the pulmonary hilum. The diaphragm side moves more than the apex side; therefore, points may optionally be plotted more coarsely closer to the diaphragm side. Optionally, in FIG. 1B, vertical dotted lines may additionally be drawn to divide the lung field into multiple rectangular (square) block regions. Thereby, lung motion may be expressed more accurately. The lung field may also be divided by methods of, for example, "plotting points vertically and dividing transversely," "plotting points horizontally and dividing longitudinally," "drawing tangents at the apex and diaphragm, setting their intersection as a center, and dividing by line segments drawn at fixed angles from a straight line through that point (e.g., vertical line)," "cutting by multiple planes orthogonal to a line connecting the apex (or hilum) and the diaphragm end." These methods are also applicable to three-dimensional images. In 3D images, each organ is regarded as a space enclosed by multiple curved or flat surfaces. An organ may be further subdivided. For example, in a 3D model of a right lung, it may be divided into upper, middle, and lower lobes.

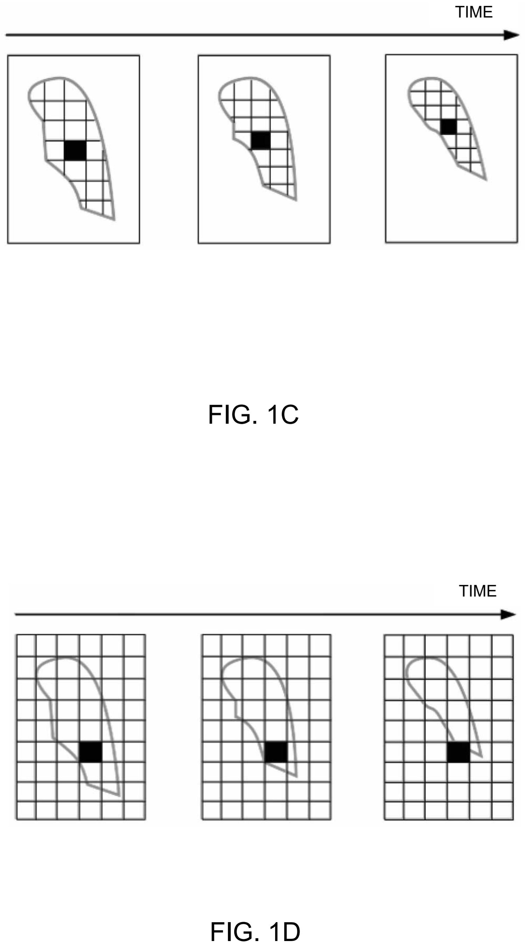

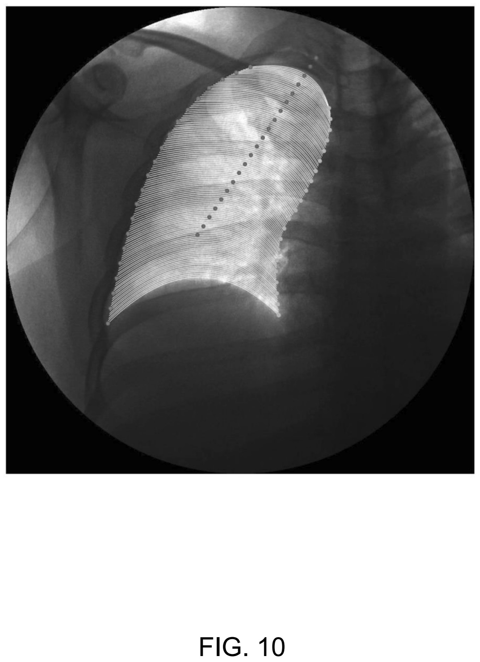

The lung field region should be relatively evaluated based on identifying the movement of the thorax, the movement of the diaphragm, and the positional relationship of blood vessels in the entire lung field, and by grasping the relative position of the lung contour and evaluating it based on its movement. Accordingly, in the present invention, after the lung contour is automatically detected, the region specified by the lung contour is divided into a plurality of block regions, and the values of changes (pixel values) of images contained in each block region are averaged. For example, as shown in FIG. 10, plotting points on opposing edges of the lung on a Bezier curve, connecting them, and using a curve passing through the midpoint between them are possible. As a result, as shown in FIG. 1C, even if the lung morphology changes over time, the temporal changes of the target region may be tracked. On the other hand, FIG. 1D the temporal changes in the case where the organ to be analyzed (the lung in this case) is divided into block regions without considering its morphology. As described above, the lung field region should be evaluated based on the movement of the thorax, the movement of the diaphragm, the positional relationship of blood vessels in the entire lung field, and the relative position of the lung contour. However, as shown in FIG. 1D, if the lung field is divided into block regions without specifying the lung field region, the target region deviates from the lung field due to temporal changes in the lung, resulting in meaningless images. In particular, since the movement of the diaphragm strongly contracts the lung field, it is preferable not only to correct the diaphragm or overall numerical values, but also to include thoracic components and other multiple elements to correct the lung field region. A method of inputting the respiratory element frequency or frequency band may also be used. The same calculation for region segmentation may also be applied to three-dimensional images.

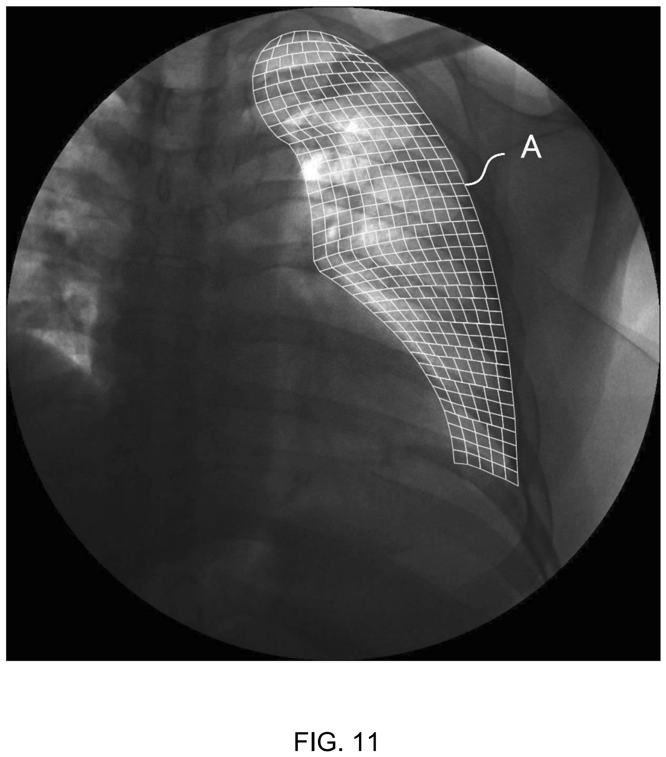

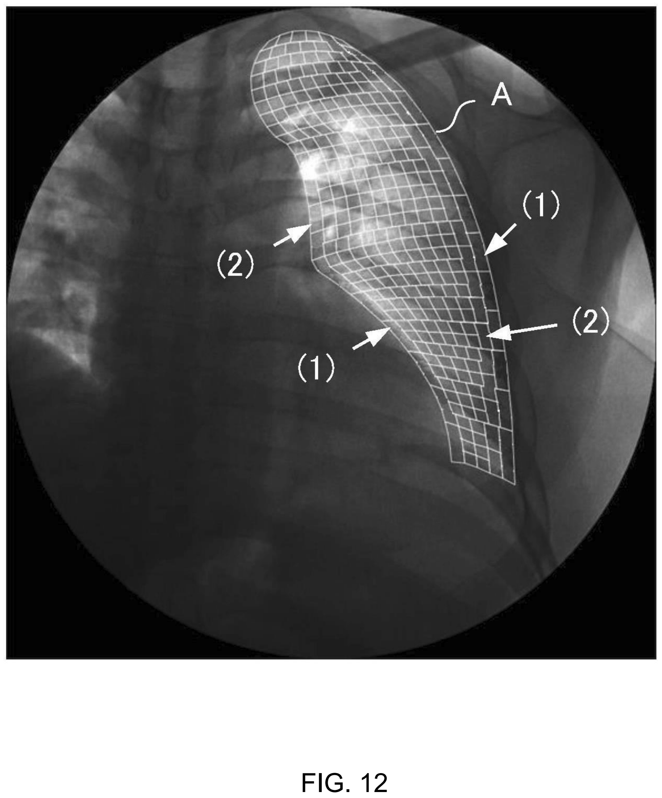

Furthermore, as shown in FIG. 11, within lung field A, using Bezier curves to select internal control points inside the detected lung field, the lung field may be divided by curves or straight lines passing through those internal control points. That is, control points are set not only along the outer boundary of the lung field but also inside the lung field region, and lung field region (A) is divided using these control points. In this case, as shown in FIG. 12, the spacing between control points located on and near the periphery of the detected lung field may be set relatively large (1), while the spacing between internal control points inside the detected lung field may be set relatively small (2), according to the expansion ratio of each part. In lung field A, the spacing of control points may be set relatively larger in the head-to-tail direction of the body, or relatively larger in a specific vector direction. This vector may be defined in any way, for example, in the direction from the apex of the lung to the opposite side of the lung field, or in the direction from the hilum of the lung to the opposite side of the lung field as shown in FIG. 1B. Defining the vector direction according to the structure of the lung is also possible. By "dividing unequally" to divide the lung field, displaying images considering the characteristics of each region is possible. For example, the outer periphery of the lung field exhibits large movement and large displacement, so the blocks are made larger, whereas the interior of the lung field exhibits small movement and small displacement, so the blocks are made smaller and finer. For another example, on the diaphragm side of the lung field, where movement and displacement are large, the blocks are made larger, while on the cranial side of the lung field, where movement and displacement are smaller, the blocks are made smaller and finer. This enables the display accuracy to be improved. This technique is not limited to lung fields and may also be applied to dynamic regions that move in association with respiration. Such a technique may also be applied when dividing the lung three-dimensionally by lobes. It may also be used, for example, to enclose and display regions located below the diaphragm, such as the heart or other organs, using Bezier curves. In this case as well, it is possible to define vectors in directions corresponding to the structure of the heart or other organs and to perform unequal partitioning of the region.

Next, artifacts are eliminated and the image data are interpolated. That is, since the presence of bones or the like within the analysis range appears as noise, removing such noise using a noise-cut filter is preferable. In X-ray images, typically, air is assigned a value of -1000 and bone is assigned a value of 1000. Therefore, areas with high transmissivity have low pixel values and are displayed as black, whereas areas with low transmissivity have high pixel values and are displayed as white. For example, when pixel values are represented in a 256-level grayscale, black is represented by 0 and white is represented by 255. In lung field regions where no vessels or bones are present, X-rays transmit easily, resulting in low pixel values in the X-ray image, which thus appears black. On the other hand, in regions where vessels or bones are present, X-rays transmit poorly, resulting in higher pixel values, and the X-ray image appears white. The same applies to CT and MRI images. Here, based on the results of the above analysis of respiratory element cycle and the waveform per breath, interpolating data using values at the same phase and eliminating artifacts are possible. Furthermore, if "different coordinates," "extreme fluctuations in pixel values," or "abnormally high frequencies or densities" are detected, these may be cut off, and the remaining images obtained may be used to calculate the Hz of the diaphragm and adjust the lung fields by identifying a continuous and smooth wave shape using, for example, the least squares method. Moreover, when images are superimposed, available methods include (1) directly overlaying an acquired comparison image with its original coordinates, and (2) acquiring one image as a base, then relatively scaling the other image and overlaying it based on relative positional information. Using these methods, the shape of the lung field may be corrected or changes in the block region images may be adjusted. That is when artifacts in the results are excluded again, and functional features are extracted from newly extracted data waveforms, the initial base waveform, other modality waveforms, surrounding data, or multiple waveforms. The number of iterations may be either once or multiple times.

Here, "reconstruction" along the time axis is explained. For example, when the inspiration time is 2 seconds at 15 frames per second, 30+1 images are obtained. In this case, "reconstruction" by 10% may be performed simply by superimposing three images at a time. For example, when 0.1 seconds corresponds to 10% but only images at 0.07 seconds and 0.12 seconds have been acquired, "reconstruction" at 0.1 seconds is required. In this case, "reconstruction" is performed with a given intermediate value (average of two) between the images around 10%. Optionally, the coefficient may be adjusted according to the proportion on the time axis. For example, if no image was taken at 0.1 seconds but images exist at 0.07 seconds and 0.12 seconds, "reconstruction" may be performed by recalculating as "(value at 0.07 s) · 2/5 + (value at 0.12 s) · 3/5." Furthermore, the positional relationship of changes at the specific time may be recognized from the average respiratory or diaphragmatic coefficient variations, and numerical ratios may be calculated using those values as coefficients. It is desirable to calculate with a thickness, such as "reconstruction" by 10% to 20% or "reconstruction" by 10% to 40%, including "Maximum Differential Intensity Projection" by 0 to 100%. As such, "reconstruction" at the ratio of one respiration may be performed even for portions where no image was captured. The present invention allows similar "reconstruction" not only for respiration but also for blood flow, thoracic motion, diaphragmatic motion, and other correlated movements. Moreover, "reconstruction" may be performed for each block or each pixel. It is desirable to calculate with a thickness, such as "reconstruction" by 10%-20% or "reconstruction" by 10%-40%, including "Maximum Differential Intensity Projection" by 0-100%.

Optionally, the lung field detected using the above method may be normalized. That is, the detected lung field may be spatially normalized or temporally normalized using reconstruction. Although the size and shape of the lung field differ among individuals, normalization allows display within a standard region.

### [Diaphragm and Thorax]

By identifying the lung field as described above, movement of the diaphragm and the thorax may also be analyzed. Specifically, a curve of the diaphragm or a thoracic curve on the recognized Xp (2D) image is calculated as a set of fine coordinates. By quantifying the mean values, local downward change rates and amounts of the curves, and by applying "curve fitting" to the diaphragm to obtain deformation ratios, functional evaluation may be performed based on the image. Similarly, curves representing thoracic edges outside the diaphragmatic surface may be calculated as sets of fine coordinates, and functional evaluation may be performed by quantifying the mean values and the change rates of the curves. The two rates of change and changes described above are evaluated relative to each other and in coordination with each other, and movement function is evaluated by quantifying and visualizing areas with different rates of change (such as areas that do not move in the same way).

Here, a method for evaluating diaphragm and thorax is explained. First, movement of a diaphragm is displayed along a left-right horizontal line perpendicular to a body axis (so-called midline). Next, a diaphragm line is flattened to a baseline. That is, the diaphragm line is aligned to a horizontal straight line. Then, the movement of the diaphragm is evaluated. Furthermore, the diaphragm line is extended and flattened, and the orthogonal movement of the curve is evaluated. Next, on the lateral thoracic side, movement is evaluated using a baseline (axis) drawn from the lung apex to the costophrenic angle. A thoracic line is flattened to the baseline, i.e., aligned to the straight line between the lung apex and the costophrenic angle, to evaluate movement. The thoracic line is then extended and flattened, and the orthogonal movement of the curve is evaluated. The curvature and radius of curvature of the above thoracic and diaphragmatic lines are evaluated. The above variations are calculated as "amount of change," and their derivatives are evaluated as "rate of change."

FIGS. 6B and 6C are diagrams showing an example of an image displayed on a display. In FIG. 6B, the movement of the left lung is displayed in a video. A white horizontal line is shown in the image in FIG. 6B, which is a straight line (indicator) indicating the position of the diaphragm, which moves vertically following the movement of the diaphragm as the video plays. As such, the diaphragm is detected and the indicator showing its detected position (i.e., the white horizontal line) is displayed, which enable image-based diagnosis by a physician. In addition to detecting a part of the diaphragm, recognizing the lung field-to-diaphragm line enables identification of all points and allows diagnosis of one region of the diaphragm (e.g., lateral, medial, left, or right) as well as the entire diaphragm. Similarly, not only the diaphragm but also other dynamic structures that move in association with respiration, such as the thorax, may be evaluated based on tangential straight lines or thoracic lines identified by lung field recognition. Thus, based on an assumption that edges move, and by taking differences between consecutive images, edges may be detected. For example, tumors are usually rigid while their surrounding tissues are soft, so the tumor itself moves little whereas its surroundings move actively; thus, tumor edges may be detected through differential imaging.

In three-dimensional images such as MRI or CT, the diaphragm surface may be treated as a coordinate surface or a three-dimensional curved surface. By computing this surface as a set of fine coordinates (including the diaphragm edge contour, plane, and coordinate set), calculating average values, local downward change rates and amounts, and applying "curve fitting" to the diaphragm as a curved surface to quantify deformation ratios, functional evaluation may be performed based on images. Similarly, for other curved surfaces representing thoracic edges outside the diaphragmatic surface, functional evaluation may be performed by computing them as sets of fine coordinates and quantifying their average values and change rates. By evaluating the two rates and amounts of change relatively and interactively, and quantifying and visualizing the areas that do not move in coordination (i.e., where the change rates differ), movement functions may be evaluated.

### [Fourier Analysis]

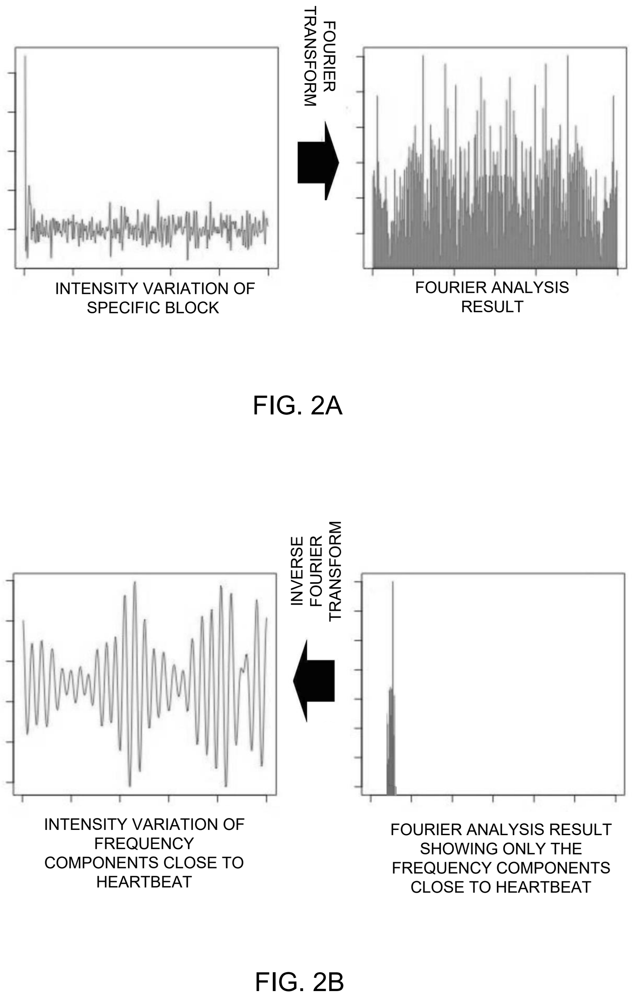

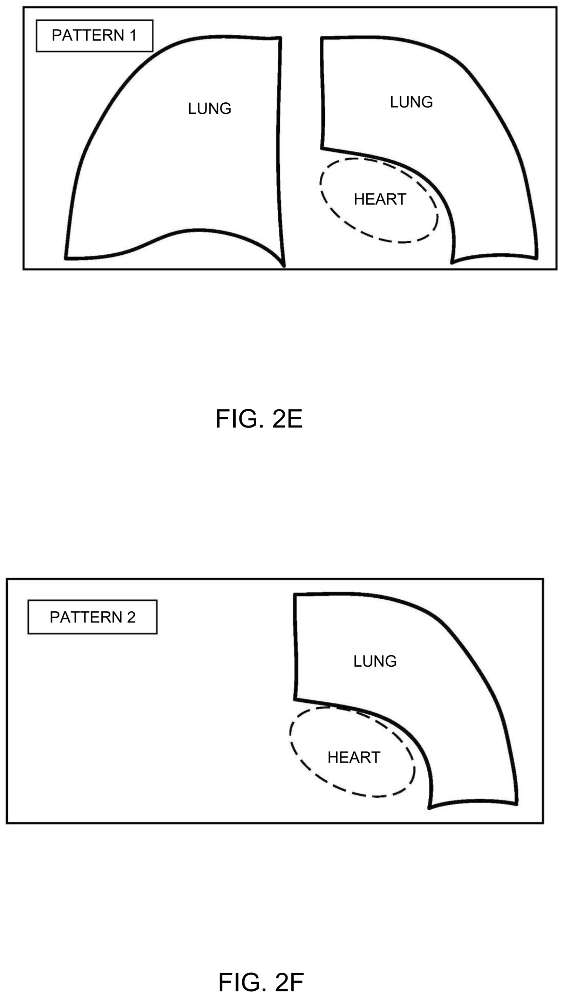

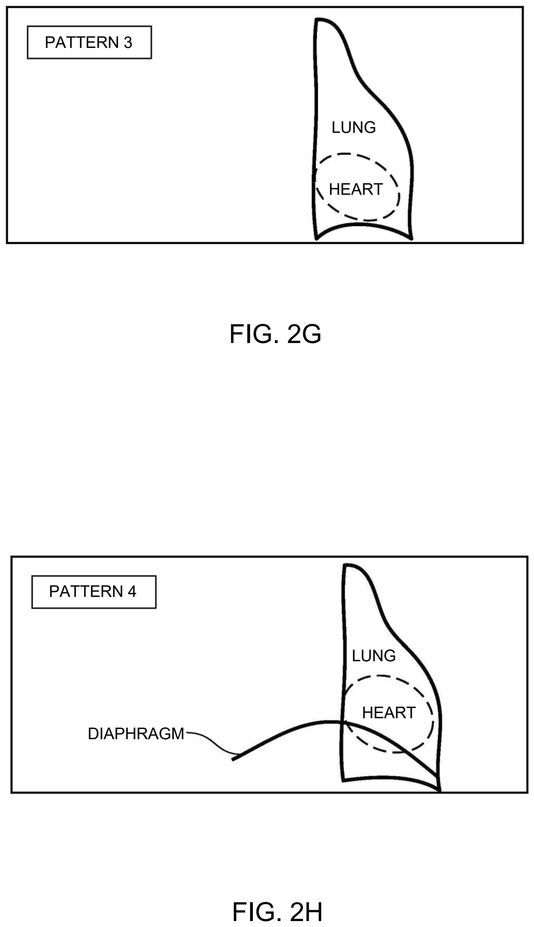

Based on the respiratory and vascular beat cycles analyzed as described above, Fourier analysis is performed on the values and variations of "density"/"intensity" in each block region. FIG. 2A is a diagram showing an "intensity" change of a specific block and a result of Fourier analysis thereof. FIG. 2B is a diagram showing a Fourier transform result with a frequency component close to a heartbeat extracted, and an "intensity" change of the frequency component close to the heartbeat obtained by inverse Fourier transformation thereof. For example, when the "intensity" change of a specific block is Fourier transformed (analyzed), a result as shown in FIG. 2A may be obtained. Then, by extracting frequency components close to the heartbeat from the frequency components shown in FIG. 2A, a result as shown on the right side of FIG. 2B may obtained. By performing an inverse Fourier transform, an "intensity" change synchronized with heartbeat variations may be obtained, as shown on the left side of FIG. 2B.

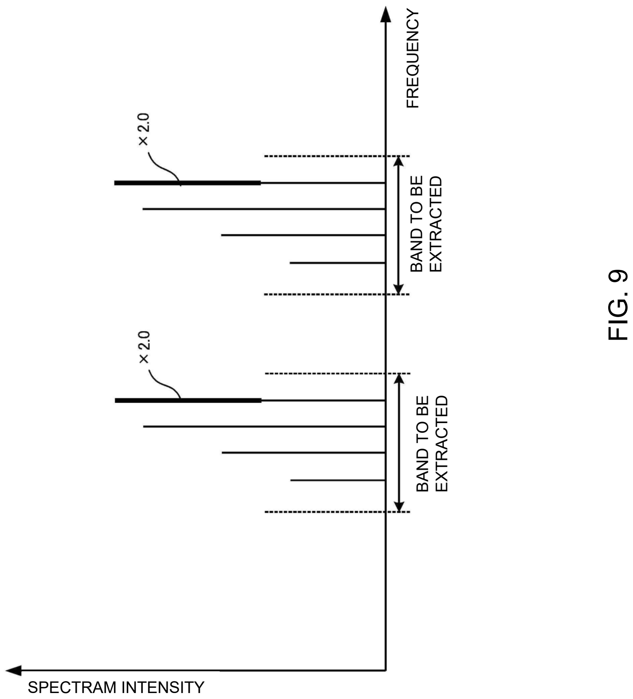

As shown in FIG. 9, weight may be applied to a specific spectrum by multiplying it by a coefficient. For example, this method may be used to achieve waveform synchronization. Specifically, in selecting frequencies for the inverse Fourier transform, multiple frequencies are chosen, their relative ratios are applied, and then the inverse Fourier transform is performed. For example, if the highest frequency spectrum within the extracted band is to be emphasized, its spectral intensity may be doubled. In this case, frequency continuity is not required. Spectra that exist discontinuously may be selected.

Furthermore, the position of "density" of the heart may be inferred from the morphology of the left lung (or the right core in cases such as situs inversus), particularly from the recessed region of the left lung identified through lung field extraction, along with the positions of the vertebral body and diaphragm. In this case, from a region of interest (ROI) for the heart, "density" is extracted. For this extraction, the approximate region is inferred using the relative spectral values of respiration and blood flow. Moreover, "filtering" may be performed in advance using the frequency band associated with cardiovascular pulsation (e.g., 40-150 bpm, ≈0.67-2.5 Hz) to remove frequencies caused by respiration and other artifacts. Because the heart position also changes depending on respiratory conditions, the relative position of the heart is adjusted based on thoracic morphology as the thorax moves, allowing for more accurate extraction of cardiovascular pulsations, the pulmonary hilum, and major vessels. Furthermore, as with diaphragmatic movement, the frequency may be calculated based on the regularly moving contour of the heart.

Here, for performing the inverse Fourier transform on a spectrum consisting of frequency components, both or either one of the frequency elements (respiratory frequency, cardiovascular pulse frequency) identified from the "density" of breathing and blood flow and the bandwidth of the spectrum (a BPF: band pass filter may also be used) may be considered to perform the inverse Fourier transform. Optionally, at least one frequency may be selected for the inverse Fourier transform based on the spectral composition ratio corresponding to organ-specific periodic variations obtained from the Fourier-transformed spectrum. Furthermore, identifying waveforms of specific organs or target regions for analysis is possible based on the composition ratio of multiple frequencies obtained after the Fourier transform (to create waveform synchronization images).

For performing Fourier analysis, an autoregressive moving average (AR) model may be used to enable faster computation. In the AR model, methods using the Yule-Walker equiation or Kalman filters may be employed, and the resulting Yule-Walker estimates, PARCOR method, and least-squares method may be applied to supplement the calculations. This allows for faster, nearly real-time image acquisition, as well as supplemental computation and artifact correction. Through such Fourier analysis, the characteristics of images in each block region may be extracted and displayed.

Moreover, a method using a "digital filter" may be employed in this Fourier analysis. That is, a "digital filter" is applied to the original wave after performing a Fourier transform to obtain the parameters of each spectrum, and then applying mathematical processing to the original wave. In this case, the inverse Fourier transform is not performed; instead, the digital filter is used.

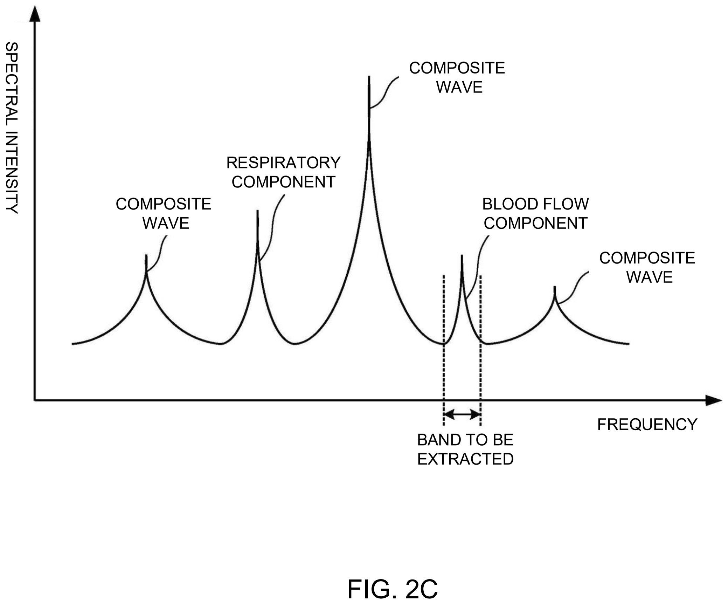

Here, the variations in each block region of each frame image are subjected to Fourier transformation, and from the resulting spectrum, extracting the spectral components is possible within a specific band that correspond to the respiratory element cycle. FIG. 2C is a diagram showing an example of extracting a certain band among spectra obtained after Fourier transformation. The frequency f of the composite wave spectrum satisfies the relationship "1/f = 1/f1 + 1/f2" with the component frequencies f1 (respiration) and f2 (blood flow), and the following methods may be employed for spectrum extraction.
(1) Extract portions of the spectrum with a high ratio of blood flow components; (2) extract the spectrum by segmenting at the midpoint between the peak corresponding to respiration/blood flow and nearby peaks of multiple composite waves; and (3) extract the spectrum by segmenting at the trough between the peak corresponding to respiration/blood flow and nearby troughs of multiple composite wave spectra.

As described above, the present invention may not use a fixed BPF but instead extracts the spectral components within a specific band that correspond to the respiratory element cycle. Furthermore, in the present invention, it is also possible to extract, from the spectrum obtained after Fourier transform, a spectrum within a certain band that includes frequencies other than respiratory elements obtained from frame images (e.g., density/intensity of each part, heartbeat elements obtained from heartbeat or vascular pulse), or a spectrum corresponding to a frequency input from outside by an operator (e.g., a spectral model).

Here, when the composite spectrum consists of only two components (respiration and blood flow), they are distributed 50% each; for three components, the distribution is 1/3 each. Accordingly, the composite spectrum may be estimated to some extent from the relative proportions and amplitudes of the respiratory and blood flow spectral components. Extracting spectra at points where these ratios are high is possible. In other words, the ratio between blood flow and respiratory elements and the composite wave is calculated, and spectra with high blood flow or respiratory element values are extracted. For identifying a diaphragm or in similar analysis, there may be a case, in which spectra corresponding to regions where the frequency (Hz) remains relatively constant (i.e., with minimal frequency variation) are selectively extracted. For determining the spectral band, in order to identify the diaphragm, there may be a case in which the spectral band is determined within the range where the change in Hz occurs and its surrounding area. The waveform components may also be considered.

For the spectrum used in the inverse Fourier transform, one of options of "extraction using (one or more) high frequency parts from a simply modeled frequency and frequency band (simulation approach)" and "extraction of high frequency parts or low frequency parts according to the spectral value based on the actual frequency or frequency band (on-site approach)," may be selected. Moreover, if the heart frequency is A, and the lung frequency is B, subtracting A from the entire frequency band yields B. With regard to the spectrum obtained by Fourier transform, not only one point on the frequency axis but also multiple points may be extracted.

According to the above, not only spectra that exactly match respiratory or vascular pulsation cycles but also spectra that should better be considered may be extracted, contributing to image diagnosis. It is known that "respiration" and "heartbeat" fall within specific frequency bands. Therefore, for respiration, a filter of "0-0.5 Hz (0-30 breaths per minute)" may be used; for cardiovascular activity, a filter of "0.6-2.5 Hz (36-150 beats per minute)" may be used so that the respective frequencies may be specified in advance. This enables frequency synchronization images to be displayed. This is because, for example, respiratory (lung) "density" changes may be unintentionally captured when extracting cardiac density changes, and conversely, cardiac "density" changes may be unintentionally captured when extracting respiratory (lung) "density" changes.

### [Visualization and Quantification]

The analysis results described above are visualized and quantified. For visualization and quantification, the present disclosure defines a "modeled lung." When displaying the lung as a dynamic image, positional relationships shift, which may make relative comparisons difficult. Therefore, positional discrepancies are spatially normalized and averaged. For example, the shape of the lung may be fitted to a geometric shape such as a fan and displayed in a standardized form. The concept of reconstruction is then applied to achieve temporal normalization. For example, the "lung state at 20% of multiple breaths" may be extracted and defined as the "lung state at 20% of a single breath." As such, a lung that is spatially and temporally standardized is referred to as a "modeled lung." This allows easier relative comparisons between different patients or between past and present states of the same patient.

For example, as standard uptake, an average value of the measured density/intensity across the entire lung field may be set to 1 and displayed relatively/logarithmically. Moreover, in order to focus only on the direction of blood flow, changes in specific directions may be extracted. This enables extraction of only meaningful data for analysis. Using the identified lung fields, pseudo-coloring is applied to track changes in the analysis region. That is, the analysis results of each individual (subject) are mapped onto a relative region along a specific phase-dependent shape (minimum, maximum, average, or median).

The data are also transformed into specific shapes or phases that allow comparison between multiple analysis results. Furthermore, when constructing the modeled lung, the results of the respiratory element cycle analysis are used to calculate the relative spatial relationships within the lung field. The modeled lung is generated using averaged lines that integrate thoracic contours, density distributions, diaphragmatic positions, and other features of multiple patients. When modeling lung blood flow, distances from the pulmonary hilum to the peripheral lung regions may be measured radially. For respiration, corrections need to be made according to the movements of the thorax and diaphragm. Optionally, calculations may be performed in consideration of the distance from the lung apex.

Optionally, after inverse Fourier transformation, only those blocks with relatively large amplitude values may be extracted and displayed. In other words, when Fourier analysis is performed for each block, some blocks will show large wave amplitudes after inverse Fourier transformation, while others will show small amplitudes. Therefore, extracting and visualizing only the blocks with relatively large amplitudes may be effective. Moreover, after inverse Fourier transformation, the real and imaginary parts of each value may be used separately. For example, an image may be reconstructed from only the real parts, or an image may be reconstructed from only the imaginary parts, or an image may be reconstructed from the absolute values of the real and imaginary parts.

Fourier analysis may also be performed on the modeled lung. That is, the modeled lung may also be used for aligning images from multiple breaths, performing Fourier analysis, or determining relative positions. By using the modeled lung, multiple frames having been acquired may be fitted to the modeled lung. In the case of blood vessels, the modeled lung may be adapted according to the cardiac cycle (e.g., heartbeat obtained at the pulmonary hilum), thereby maintaining consistent relative positioning during Fourier analysis. Using the modeled lung to capture the baseline respiratory state also enables stable calculation results. Furthermore, by modeling the lung, spatial differences may be fixed, enabling easier visualization of the lung motion.

In visualization of images, a labeling method for relative evaluation is as follows. The image is labeled relatively using grayscale or color mapping. A few percent of the values around the differential density/intensity may be cut, and the remaining upper and lower portions may be displayed relatively. Or, values that are a few percent above or below the differential may be outliers; therefore, these values may be removed as artifacts, and the remaining parts may be displayed relatively. Rather than 0-255 scale, values may also be displayed as values between 0 and 100%.

The pixels may be displayed in a somewhat unclear manner to display the entire image in a blurred form. In particular, for pulmonary vessels, low signal values may be interspersed among high signal values, but as long as the high signal areas may be roughly identified, the overall display may be somewhat blurred. For example, in a case of blood flow, signals above a threshold may be extracted, whereas for respiration, signals below the threshold may be left unextracted. Specifically, for taking the central value from the numbers in the following table as one pixel, the proportion of the central value may be determined, and averaging within the pixel allows for smoother representation between adjacent pixels. This method may also be applied for calculating the average intensity for each block.

### [Table 1]

This method may be applied not only to lung fields but also to detect the density of any analysis region and remove areas where the density changes significantly. Moreover, points that greatly exceed a preset threshold are cut off. Further, rib morphology, for example, sudden high or low signal lines, may be recognized and removed. Similarly, from the phase, signals that appear suddenly, such as those characteristic of patients who exhibit artifacts at around 15% - 20% of the reconstruction phase, may be removed. When first collecting the base data, the phase may differ in calculations such as (diaphragm) ≈ (thorax) ≈ (thoracic movement) ≈ (spirometer) ≈ (lung field), field (density) ≈ (volume), etc., and the phase may be applied to an actual recognizable shape (XP contour).

Once a modeled lung is created, parameters such as synchrony, match rate, and mismatch rate may be quantified and displayed (as frequency-synchronization or wavelength-synchronization images). This allows visualization of deviations from the normal state. According to the present embodiment, performing Fourier analysis enables the detection of potential new diseases and comparisons with one's normal state, between hands and feet, or between opposite limbs. Furthermore, abnormalities in actions such as leg movements or swallowing may be identified by quantifying synchrony. Moreover, determine whether a condition of a patient has changed over time may be determined, and, if the condition has changed, states before and after the change may be compared. Moreover, by shaping the lung fields into a circular or quasi-circular form with a fixed radial distance from the periphery, inner, middle, and outer layers may be evaluated easily, and it may be expressed according to conditions whether the blood vessels are "peripherally dominant" or "mid-layer dominant."

When visualized, Fourier-transformed and pre-transformed images may be toggled or may be displayed side by side on the same screen. Further, Fourier transforms may be applied to the obtained images to acquire frequency information. This allows the data necessary to identify waveforms to be obtained.

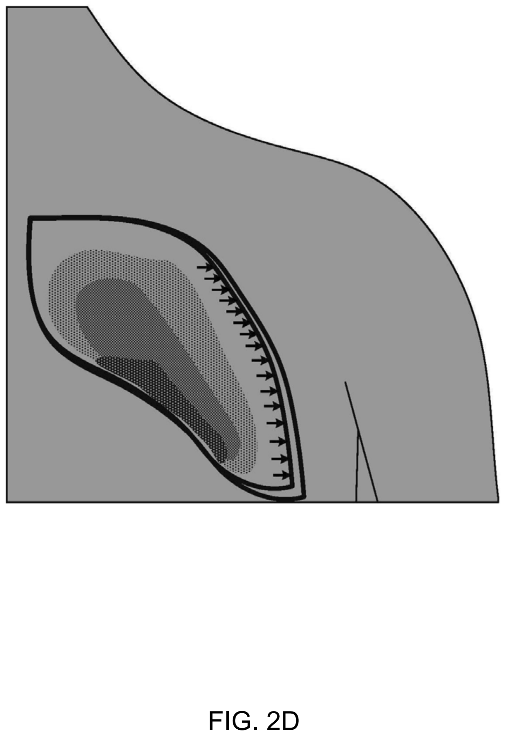

As shown in FIG. 2D, by defining the modeled lung as 100, the percentage difference in the lung of the subject may be determined, and the rate of change may be displayed. Differences or coincidences may be identified not only for the entire lung but also for specific regions. In particular, as described above, the movement of the diaphragm may be isolated while fixing the shape of the other lung fields, enabling visualization of diaphragm motion along with synchrony and variation rates. Furthermore, the entire lung field may be fixed, and the synchrony rate or the variation rate may be displayed. Furthermore, "variation classification" enables the identification of standard blood flow. That is, by identifying the respiratory element cycle and calculating the relative vascular positional relationships, the hemodynamics of the subject may be characterized as standard blood flow.

Optionally, lungs may be detected using a pattern-matching approach. FIGS. 2E-2H are diagrams showing examples of pattern images of a lung field region. As shown in FIGS. 2E-2H, lung shapes may be pre-classified into patterns, and the most similar one may be extracted. This method allows determination of whether the image represents a single lung or both lungs. It may also determine whether it is the right or left lung. The number of patterns is not limited, but assumably, four to five patterns may be prepared. This method may enable recognition of the right lung, left lung, or both lungs based solely on shape. Furthermore, by recognizing the thick, band-shaped "low-transparency region" formed of the vertebral column and mediastinum and analyzing its positional relationship with the "high-transparency regions" of the lungs, left, right, or both lungs may be recognized. As shown in FIG. 2H, this method may also be applied to regions below the diaphragm. This enables the subdiaphragmatic regions and the heart to be recognized.

Furthermore, air has the highest transparency, which is higher than that of the lung fields; therefore, preferably, air may be considered in the calculation. That is, determinations may be made depending on a position of the air on the image, as follows: If the air region at upper-right in the screen is larger than the air region at upper-left in the screen, the lung is determined to be the left lung, because, in areas around the shoulders, the air regions appear to be larger in the image. If the air region at upper-left in the screen is larger than the air region at upper-right in the screen, the lung is determined to be the right lung, because, similarly to the above, in areas around the shoulders, the air regions appear to be larger in the image. Next, if the air region at upper-right in the screen is nearly equal to the air region at upper-left in the screen, it is determined to be the both lungs, because, the air regions are substantially in an equal size.

Occasionally, intestinal air may enter below the diaphragm, in which case the region may not be recognized correctly. Therefore, optionally, from the central part of the lung field, the mediastinal side, cardiac side, and diaphragmatic side may be first roughly identified, and the line surrounding these lower-transparency regions may be used to recognize the lung boundary. This method may utilize techniques such as those disclosed in a website: https://jp.mathworks.com/help/images/examples/block-processing-large-images_ja_JP.html

Accordingly, differences between one patient and the other patient may be compared and quantified. Further, comparisons and quantitative evaluation between normal lungs or vessels and typical cases of abnormal lung function or blood flow are possible. Furthermore, the modeled lung and standard blood flow may be used for relative evaluation of lung function or pulmonary blood flow at different times for a single patient. Such modeled lungs and standard blood flow are created by aggregating typical cases of various patient types and healthy individuals, and may serve as reference standards for morphological fitting and evaluation of a given patient.

### [Drawing Lung Field]

In general, since lung fields contain ribs with low transparency, identifying the lung contour mechanically using only density as an index is difficult. Therefore, the present disclosure employs a method that uses a combination of Bezier curves and straight lines to tentatively draw the lung contour and adjust it to achieve the highest degree of conformity.

For example, if the contour of the left lung is represented by four Bezier curves and one straight line, the lung contour may be drawn by determining five points on the contour and four control points. By shifting the positions of the points to draw multiple lung contours and evaluating the match using conditions, such as "the total density value within the contour is maximized" or "the difference between the total density of several pixels inside and outside the contour is maximized," the lung contour may be detected with high accuracy. In practice, points along the easily detectable upper lung contour or along the diaphragm detected by a method which will be described later may be used to reduce the number of simulation iterations. Moreover, contour points near the outer boundary may be extracted by classical binarization, and the Bezier control points may be adjusted using least squares or similar methods.

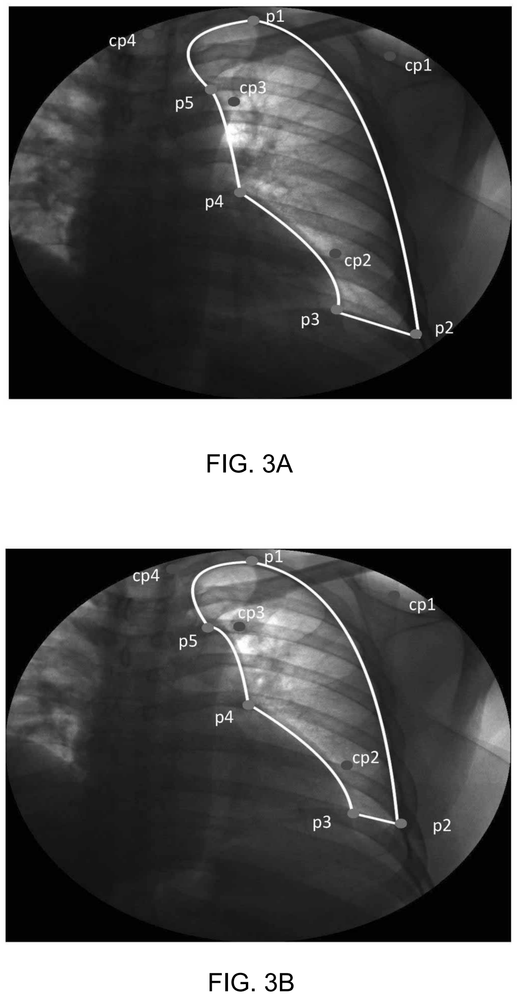

FIGS. 3A and 3B are diagrams showing examples, in each of which a contour of a lung field is drawn using both the Bezier curve and the straight line. FIG. 3A shows a case where the lung area is maximum (maximum contour), and FIG. 3B shows a case where the lung area is minimum (minimum contour). In each figure, "cp-cp5" denote control points, while "p1-p5" represent points located on the Bezier curves or the straight line. Determining both the maximum and minimum contours enables calculation of intermediate contours. For example, states corresponding to 10%, 20%, and so on of exhalation may be displayed. As such, according to the present embodiment, at least the lung fields, blood vessels, or heart may be drawn using at least one Bezier curve. The method described above is not limited to the lungs; it may also be applied to the detection of other organs. For example, at least one Bezier curve may be applied to a predefined analysis region in a specific frame (e.g., a tumor, hypothalamus, basal ganglia, or internal capsule boundary), and corresponding regions in other frames may be detected through this process.

Moreover, this method is applicable not only to planar images but also to three-dimensional (3D) images. By defining equations for surfaces and setting their control points, analysis regions bounded by multiple surfaces may be specified.

### [Detection of Diaphragm or Respiratory-Linked Dynamic Motion]

Motion of the diaphragm or other dynamic regions associated with respiration in continuously captured images may be detected. When images are selected at any interval from continuously captured images and the difference between the images is calculated, the difference becomes large, especially in areas with high contrast. By appropriately visualizing these differences, regions where movement occurred may be detected. For visualization, noise may be removed using thresholding, and curve fitting methods such as least squares may be employed to emphasize the continuity of areas with large difference values.

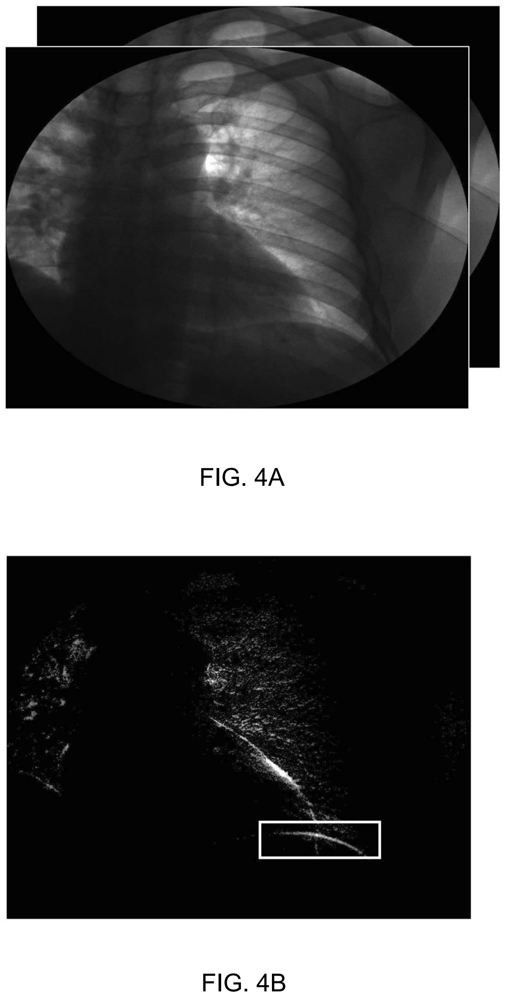

In lung fields, the contrast along the lines where the diaphragm and heart contact the lungs is particularly distinct. As shown in FIG. 4A, by taking the difference between two lung images and visualizing it with a set threshold, the lines corresponding to the diaphragm and heart boundaries may be visualized, as shown in FIG. 4B.

### [Estimation of Diaphragm Motion]

According to this method, a position of the diaphragm may be detected between target images when the diaphragm is moving; however, detecting sections where the diaphragm motion is slow is difficult. That is, detection becomes difficult at the moments when exhalation and inhalation switch, during breath-holding, or at the very beginning or end of capturing the images. In this method, the diaphragm motion is estimated using any interpolation techniques.

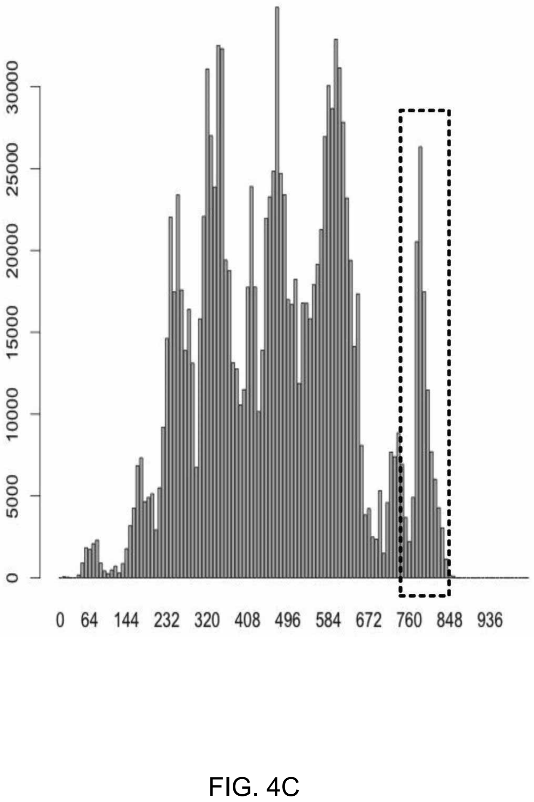

After visualizing the diaphragm line using the method described above, as shown in FIG. 4B, an image with a height of 1024 pixels is divided into 128 rectangular regions, each 8 having a height of 8 pixels, and the signal values within each rectangle are summed and plotted as a bar graph, as shown in FIG. 4C. Among the multiple peaks, the lowest peak indicated by the dotted rectangle is expected to correspond to the position of the diaphragm. In a typical upright XP image, the diaphragm is displayed as a curve, and these coordinates are used to approximate the position of the diaphragm.

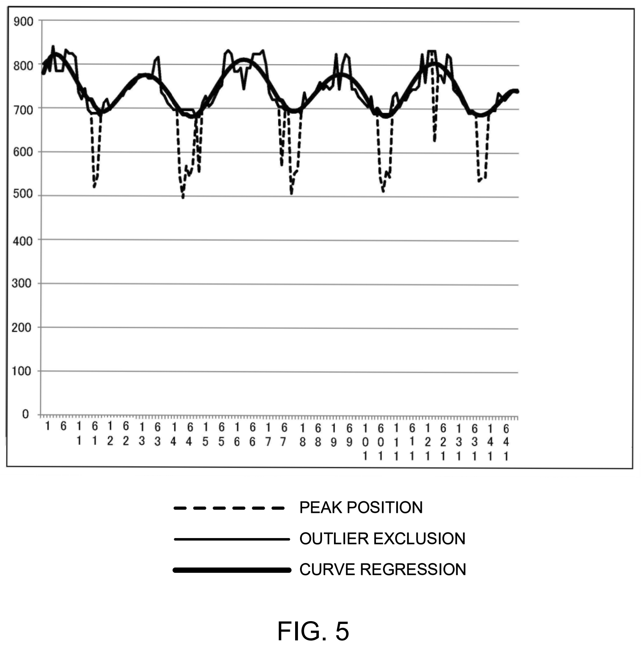

When the positions of the diaphragm were detected across all images using this method, the "peak positions" were identified as shown in FIG. 5. By correcting these detected values, diaphragm motion may be estimated. First, values whose differences exceed a predetermined threshold are regarded as outliers and excluded (thin solid line in FIG. 5). The data, after excluding the outliers, was divided into clusters, and a quartic curve regression was performed on each cluster, and the results were connected (thick solid line in Figure 5). Although regression analysis was used in this analysis, the present invention does not limit the analysis to this; various interpolation methods such as spline interpolation may also be used.

### [Refinement of Dynamic Region Detection]

The contrast along dynamic regions may not always be uniform along a line. When the contrast is not uniform, adjusting the noise-removal threshold and repeating the detection process multiple times may allow more accurate detection of the shape of the dynamic region. For example, in the left lung, the contrast of the diaphragm line tends to diminish toward the interior of the body. In FIG. 4B, only the right half of the diaphragm was detected. By adjusting the threshold to be used for noise removal, the remaining left half of the diaphragm may be detected. Repeating this process multiple times may enable the detection of the entire diaphragm shape. This method allows not only the detection of diaphragm position but also quantification of shape-related changes such as line and surface deformation rates, contributing to new diagnostic insights.

The detected position or shape of the diaphragm may be utilized for diagnostic purposes. Specifically, according to the present invention, diaphragm coordinates are graphed, and the thoracic cage and diaphragm coordinates are calculated using the computed curves (or surfaces) or straight lines described above. Moreover, elements such as cardiac pulsation, vascular pulsation, and lung field density may be graphed as positions and coordinates corresponding to their respective cycles. Such techniques are also applicable to other dynamic regions that move in coordination with respiration.

Through this method, not only the Hz during inhalation and exhalation, but also in frequency bands corresponding to changes in the frequency (Hz) of the diaphragm or dynamic regions associated with respiration may be measured. When extracting the spectrum of a band pass filter (BPF), the BPF may be set within a certain range according to the state of each respiration, allowing the axis of the BPF position to fluctuate during the "reconstructionphase" of each respiration, resulting in an optimal state, and a variable BPF that matches the state may be created. As a result, even if the respiratory rhythm changes, such as during slowed or arrested breathing (Hz = 0), images adapted to those changes may be provided.

Optionally, the overall frequency of exhalation or inhalation may be calculated based on the proportion that the respiratory element occupies within the entire exhalation or inhalation phase. Optionally, for detecting the diaphragm, multiple detections may be performed, and the most stable signal or waveform may be selected. As above, at least one frequency of the respiratory element may be calculated based on the detected diaphragm position or shape, or on the position or shape of dynamic regions associated with respiration. Once the diaphragm or the position or the shape of the dynamic region is identified, the frequency of the respiratory element may be determined. According to this method, even if a part of the waveform is segmented, subsequent waveforms may still be tracked. Therefore, even if the respiratory frequency changes midway, the original respiratory element may still be tracked. Similarly, sudden changes such as cardiac pulsation may also be handled in the same way for cardiovascular components. Next, operation of each module according to the present embodiment is explained.

### [Respiratory Function Analysis]

First, respiratory function analysis is described. FIG. 6A is a flowchart showing an outline of respiratory function analysis according to the present embodiment. The basic module 1 extracts DICOM images from the database 15 (Step S1). At this stage, at least multiple frame images within a single respiratory cycle are obtained. Next, for each acquired frame image, a cycle of the respiratory element is identified using the density (density/intensity) in at least a specific region within the lung field (Step S2). The identified respiratory cycle and the waveform derived from this cycle may be utilized in the subsequent steps described below.

The respiratory element cycle may be determined using the motions of the diaphragm and the thoracic cage. Optionally, data obtained from other measurement methods, such as volumes characterized by "density"/"intensity" in the region where the X-ray transmissive rate is high, or spirometry, may also be used. Furthermore, optionally, the frequency inherent to each organ (in this case, lungs) may be identified in advance, and the "density"/"intensity" corresponding to the identified frequency may be extracted.

Next, in FIG. 6A, the lung fields are automatically detected (Step S3). The lung contour changes continuously; therefore, once the maximum and minimum shapes are detected, the intermediate shapes may be interpolated through calculation. Based on the cycle of the respiratory element identified in Step S2, the lung contour for each frame image is determined by interpolating between frames. Optionally, pattern matching as shown in FIGS. 2E-2H may be performed to detect the lung fields. Optionally, noise may be removed by cutoff from the detected lung fields. Next, the detected lung fields are divided into multiple block regions (Step S4). Then, changes in each block region in each frame image are calculated (Step S5). At this stage, values of change within each block region are averaged and represented as a single data value.

Optionally, noise may be removed by cutoff from the change values within each block region. Next, Fourier analysis or coherence analysis is applied to the "density"/"intensity" values and their variations in each block region, based on the respiratory element cycle described above (Step S6).

Next, noise is removed from the results obtained through Fourier or coherence analysis (Step S7). Here, cutoff processing and artifact removal as described above may be applied. The steps from S5 to S7 are performed at least once, and whether the process is completed is determined (Step S8). Regarding the features to be displayed on the screen, due to the mixture of composite waves and other waves, it may not be possible to display frequency synchrony images of highly pure elements, such as respiratory elements, blood flow elements, and other elements, with a single spectral extraction. In such cases, the displayed feature values may be used as pixel data to repeat part or all of the analysis multiple times. Through this operation, the higher-purity images regarding the coherence and consistency of elements such as respiratory and blood flow components may be obtained. This operation may be performed manually by an operator while visually observing the images on the display, or may be performed automatically by extracting spectra from the output result and recalculating its distribution ratio. Moreover, after the calculations, noise-cut processing, filling (interpolation) using the least squares method, or corrections using surrounding "density" may also be applied as needed.

If the process is not completed in Step S8, the flow returns to Step S5. If the process is completed, the results of Fourier or coherence analysis are displayed on the display as pseudo-color images (Step S9). Optionally, images may be displayed in monochrome. By repeating the cycle multiple times in this manner, accuracy of the data may be improved. As such, a desired video may be displayed. Optionally, the displayed image may be modified to obtain a desired video.

According to the present embodiment, a desired frequency or frequency band is calculated computationally; however, when viewed as an actual image, a preferable image may not always be obtained. Therefore, the following methods may also be employed. (1) A method in which several frequency bands are presented and selected manually by a human operator. (2) A method in which several frequency bands are presented and an appropriate image is extracted using Al-based pattern recognition. (3) A method based on HISTGRAM trends and shapes, that is, the central value of the Histgram of the resulting signal tends to increase, and the "histgram" values fluctuate depending on motion; therefore, frequency bands may be selected based on the HISTGRAM trend and shape.

### [Pulmonary Blood Flow Analysis]

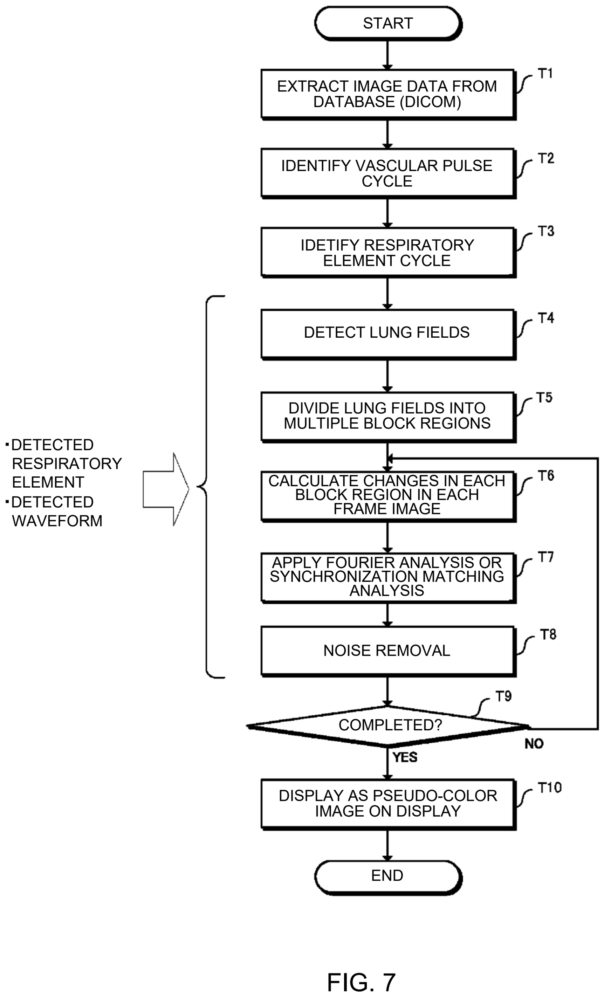

Next, pulmonary blood flow analysis is described. FIG. 7 is a flowchart showing an outline of pulmonary blood flow analysis according to the present embodiment. The basic module 1 extracts DICOM images from the database 15 (Step T1). At this stage, multiple frame images contained within at least a single heartbeat are obtained. Next, based on the obtained frame images, a cycle of vascular pulse is identified (Step T2). The identified vascular pulse cycle and the waveform determined from the vascular pulse cycle may be used in the subsequent steps. As described above, the vascular pulse cycle may be analyzed using measurements from other modalities, such as electrocardiograms or pulse meters, or variations in "density"/"intensity" at arbitrary sites such as the heart, hilum, or major blood vessels. Optionally, the frequency inherent to each organ (in this case, pulmonary blood flow) may be identified in advance, and the "density"/"intensity" corresponding to the identified frequency may be extracted.

Next, in FIG. 7, the respiratory element cycle is identified by the above method (Step T3), and the lung fields are automatically detected using that respiratory element cycle (Step T4). In automatic detection of lung contours, variation may occur among frame images, but by interpolating each frame image based on the respiratory element cycle identified in Step T3, the lung contour in each frame may be determined. Optionally, pattern matching as shown in FIGS. 2E-2H may also be used to detect the lung fields. Optionally, noise may be removed by cutoff from the detected lung fields. Next, the detected lung fields are divided into multiple block regions (Step T5). Then, changes in each block region in each frame image are calculated (Step T6). Here, the change values within each block region are averaged and represented as a single data value. Optionally, noise may be removed by cutoff from these change values. Next, Fourier analysis or coherence analysis is applied to the "density"/"intensity" values and their variations in each block region, based on the vascular pulse cycle (Step T7).

Next, noise is removed from the results obtained through Fourier or coherence analysis (Step T8). Here, cutoff processing and artifact removal as described above may be applied. The steps from T6 to T8 are performed at least once, and whether the process is completed is determined (Step T9). Regarding the features to be displayed on the screen, due to the mixture of composite waves and other waves, it may not be possible to display frequency synchrony images of highly pure elements, such as respiratory elements, blood flow elements, and other elements, with a single spectral extraction. In such cases, the displayed feature values may be used as pixel data to repeat part or all of the analysis multiple times. Through this operation, the higher-purity images regarding the coherence and consistency of elements such as respiratory and blood flow components may be obtained. This operation may be performed manually by an operator while visually observing the images on the display, or may be performed automatically by extracting spectra from the output result and recalculating its distribution ratio. Moreover, after the calculations, noise-cut processing, interpolation using the least squares method, or corrections using surrounding "density" may also be applied as needed.

If the process is not completed in Step T9, the flow returns to Step T6. If the process is completed, the results of Fourier or coherence analysis are displayed on the display as pseudo-color images (Step T10). Optionally, images may be displayed in monochrome. As such, accuracy of the data may be improved. Optionally, the displayed image may be modified to obtain a desired video.

According to the present embodiment, a desired frequency or frequency band is calculated computationally; however, when viewed as an actual image, a preferable image may not always be obtained. Therefore, the following methods may also be employed. (1) A method in which several frequency bands are presented and selected manually by a human operator. (2) A method in which several frequency bands are presented and an appropriate image is extracted using AI-based pattern recognition. (3) A method based on HISTGRAM trends and shapes, that is, the central value of the Histgram of the resulting signal tends to increase, and the "histgram" values fluctuate depending on motion; therefore, frequency bands may be selected based on the HISTGRAM trend and shape.

### [Other Blood Flow Analysis]

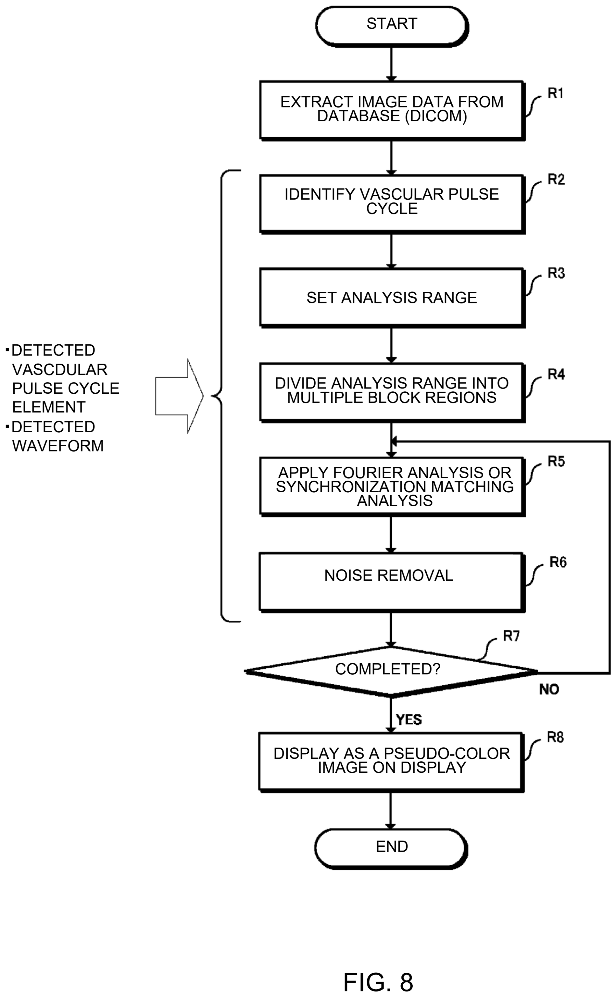

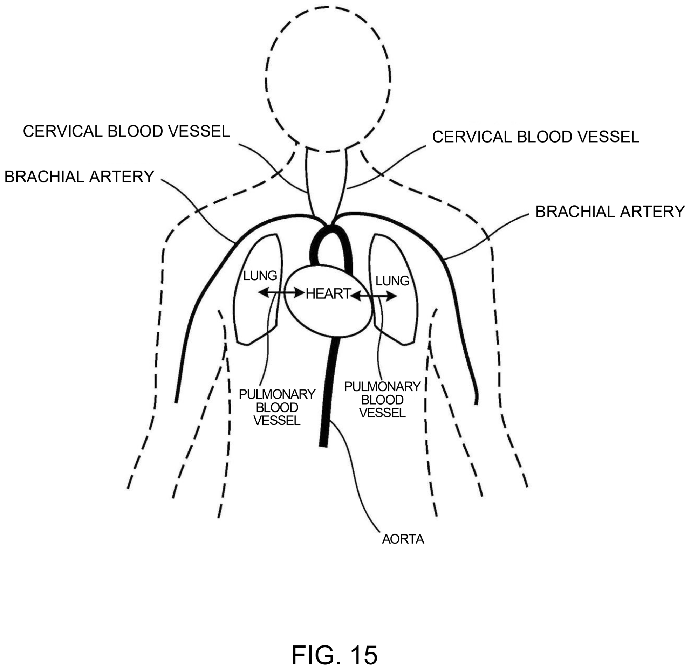

Next, other types of blood flow analysis is described. One aspect of the present invention is applicable to analysis of blood flow in heart, aorta, pulmonary vessels, brachial arteries, cervical vessels, and the like, as shown in FIG. 15. Furthermore, blood flow analysis may similarly be applied to abdominal vessels and peripheral vessels which are not illustrated. FIG. 8 is a flowchart showing an outline of other blood flow analysis according to the present embodiment. The basic module 1 extracts DICOM images from the database 15 (Step R1). At this stage, multiple frame images contained within at least a single heartbeat cycle are obtained. Next, based on the obtained frame images, a cycle of vascular pulse is identified (Step R2). The identified vascular pulse cycle and the waveform determined from the vascular pulse cycle may be used in the subsequent steps. As described above, the vascular pulse cycle may be analyzed using measurements from other modalities, such as electrocardiograms or pulse meters, or variations in "density"/"intensity" at arbitrary sites such as the heart, hilum, or major blood vessels. Optionally, the frequency inherent to each organ (e.g., major blood vessels) may be identified in advance, and the "density"/"intensity" corresponding to the identified frequency may be extracted.

Next, an analysis region is set (Step R3), and the set analysis region is divided into multiple block regions (Step R4). Then, values of change within each block region are averaged and represented as a single data value. Optionally, noise may be removed by cutoff from the change values within each block region. Next, Fourier analysis or coherence analysis is applied to the "density"/"intensity" values and their variations in each block region, based on the vascular pulse cycle described above (Step R5).

Next, noise is removed from the results obtained through Fourier or coherence analysis (Step R6). Here, cutoff processing and artifact removal as described above may be applied. The steps from R5 to R6 are performed at least once, and whether the process is completed is determined (Step R7). Regarding the features to be displayed on the screen, due to the mixture of composite waves and other waves, it may not be possible to display frequency synchrony images of highly pure elements, such as respiratory elements, blood flow elements, and other elements, with a single spectral extraction. In such cases, the displayed feature values may be used as pixel data to repeat part or all of the analysis multiple times. Through this operation, the higher-purity images regarding the coherence and consistency of elements such as respiratory and blood flow components may be obtained. This operation may be performed manually by an operator while visually observing the images on the display, or may be performed automatically by extracting spectra from the output result and recalculating its distribution ratio. Moreover, after the calculations, noise-cut processing, interpolation using the least squares method, or corrections using surrounding "density" may also be applied as needed.

If the process is not completed in Step R7, the flow returns to Step R5. If the process is completed, the results of Fourier or coherence analysis are displayed on the display as pseudo-color images (Step R8). Optionally, images may be displayed in monochrome. As such, accuracy of the data may be improved. Optionally, the displayed image may be modified to obtain a desired video.

According to the present embodiment, a desired frequency or frequency band is calculated computationally; however, when viewed as an actual image, a preferable image may not always be obtained. Therefore, the following methods may also be employed. (1) A method in which several frequency bands are presented and selected manually by a human operator. (2) A method in which several frequency bands are presented and an appropriate image is extracted using AI-based pattern recognition. (3) A method based on HISTGRAM trends and shapes, that is, the central value of the Histgram of the resulting signal tends to increase, and the "histgram" values fluctuate depending on motion; therefore, frequency bands may be selected based on the HISTGRAM trend and shape.

When images are analyzed in 3D, by measuring the respiratory volume, cardiac output, and central blood flow using other devices, the respiratory volume, cardiac output, and central blood flow for each block region may be calculated from the Fourier analysis results, which are expressed as relative values. That is, in respiratory function analysis, the respiratory volume may be used to estimate lung ventilation volume; in pulmonary blood flow analysis, pulmonary blood flow volume may be estimated from cardiac (pulmonary vascular) output; and in other blood flow analysis, the blood flow volume (ratio) of branch vessels may be estimated from the central blood flow (ratio).

As described above, although a more accurate determination may be achieved if the entire acquired database is processed, computational analysis may sometimes require considerable time. Therefore, only a selected number of images (e.g., corresponding to a specific phase) may be extracted for analysis. This approach may shorten analysis time and allow exclusion of irregular sections observed, for example, at the beginning of respiration. Moreover, when displaying the analysis results, a specific range may be displayed selectively. For example, by displaying a range from a transition of "exhale/inhale" to a transition of "inhale/exhale," the images may be played back repeatedly in so-called "endless playback," allowing physicians to make diagnosis easily.

As described above, according to the present embodiment, human body images may be evaluated using an X-ray video apparatus. Once digital data are acquired, the calculations may be performed preferably with existing equipment at low implementation cost. For example, with an X-ray video apparatus using a flat panel detector, a subject may be examined easily. Moreover, pulmonary blood flow may be screened for pulmonary thromboembolism. For example, with an X-ray video apparatus using a flat panel detector, unnecessary examinations may be eliminated by executing the diagnosis assistance program of the present embodiment before CT image-capturing. Moreover, the examination is simple and easy; therefore, highly urgent diseases may be detected early and treated with priority. Although current CT and MRI imaging methods have several limitations, resolving those issues would enable detailed diagnosis in each region.

The embodiment is also applicable to screening for various vascular conditions, such as stenosis of cervical blood flow, as well as to the evaluation and screening of major blood vessels. Moreover, lung respiration data may be effectively used as a partial lung function test and employed as a pulmonary function test. Moreover, diseases such as COPD and pulmonary emphysema may also be identified. Furthermore, the embodiment may be applied to observation of preoperative and postoperative conditions. Furthermore, by applying Fourier analysis to respiratory and blood flow cycles and removing respiratory and blood flow waveforms from abdominal X-ray images, abnormalities in other physiological movements, such as intestinal ileus, may be observed.

When the initially acquired images are in relatively high resolution, the large number of pixels may result in longer computation times. In such a case, the images may be reduced to a certain number of pixels in advance to the calculations. For example, reducing an image from 4096 by 4096 pixels to 1024 by 1024 pixels before computation may reduce the processing time.

### [More Examples]

When capturing X-ray images, predictive algorithms such as the AR method (Autoregressive Moving Average Model) may be employed. Once at least one frequency of a respiratory element is identified, the X-ray imaging apparatus may be controlled to adjust the exposure interval in accordance with the frequency. For example, if a frequency of a respiratory element is low (long cycle), the number of times of X-ray exposures may be reduced. This may reduce radiation dose to the human body. In cases of tachypnea or tachycardia, where the respiratory or cardiovascular pulsation frequency is high (short cycle), the exposure frequency may be increased to obtain optimal images.

As for a storage format of DICOM data, preferably, the data may be stored in an uncompressed format, since compression may degrade image quality. Optionally, the computation method may be varied according to the compression format of the data.

### [Knowing Lung Respiratory Motion]

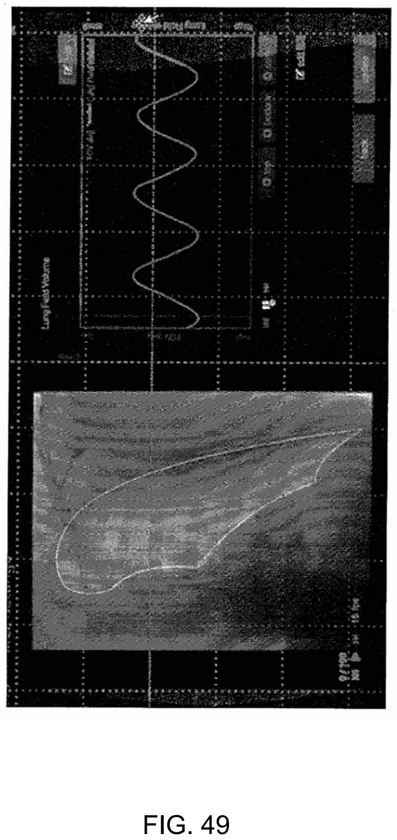

FIG. 49 is a diagram showing a graph indicating a change of density in a static image of a lung and a dynamic image of the lung. As shown in FIG. 49, by the waveform representing density changes in the dynamic lung image, the phase of expiration and inspiration may be identified. By extracting, for example, a 50% point of the density change, the lung condition may be inferred from the corresponding signal value.

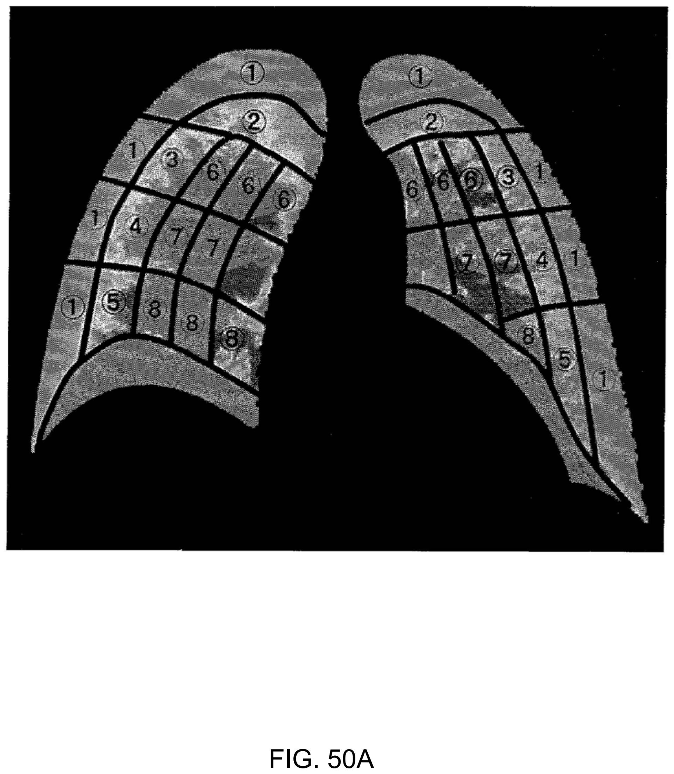

Here, as shown in FIG. 50A, the lung field region is divided into multiple regions. For example, the lung field region may be divided into an outer layer, a middle layer, an inner layer, an upper lung field, an apex, and a lower lung field. In the present embodiment, the lung field region is divided into smaller 15 regions, including an apex, a subapical area, an upper lung field outer layer, an upper lung field middle layer outer side, an upper lung field middle layer inner side, an upper lung field inner layer outer side, and an upper lung field inner layer inner side, and others. Each divided region is scaled to a trapezoid shape, and a score is assigned to each region based on its pixel values. For example, by using the pixel value of the least mobile part of the apex as a reference value, each region may be expressed in relative values. For another example, the outer portion of the lung may be used as the reference. As a result, the extent of signal movement relative to the reference value may be shown, thereby indicating the variation range of signal values in each region.

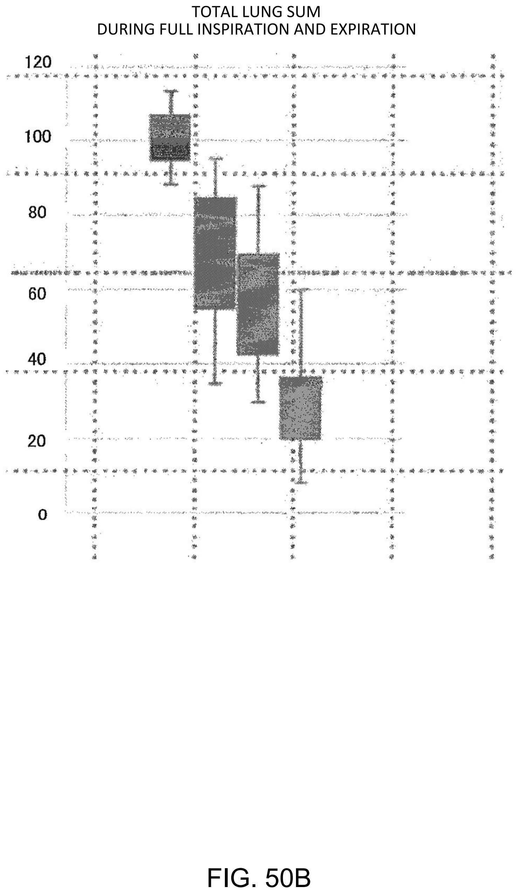

FIG. 50B is a diagram showing a variation range of a signal value relating to a movement of lungs. It shows how much the signal decreases in diseased cases compared to the value of 100 for a healthy individual. For example, if a value decreasing by 80% is classified as grade 1. A value decreasing to a range between 60% and 45% is classified as grade 2. By quantifying the variation in this manner, the degree of change in each region may be recognized, allowing severity of disease to be estimated. In other words, since the range of variation differs depending on the disease, for example, in the "upper lung field/inner side" region, a normal decrease is down to -1.5, whereas in COPD, if the value decreasing only to "-1.3" may be judged as stage 1, "-1.1" as stage 2, and the value decreasing only to 0.85 may be judged as stage 3.

Optionally, inspiration and expiration may be scored separately. Moreover, the right lung or left lung may be scored individually, or the entire lungs may be scored collectively. Preferably, artifacts such as ribs should be manually excluded by an operator.

For example, expected signal characteristics of the "subapical" region are statistically known. When 100 represents the value for a healthy individual, the signal value may be recorded with scores such as score 1 or score 2 corresponding to abnormal levels. For another example, a severity level of COPD may be expressed numerically to indicate the disease stage.

The calculations may be made for each region individually, or the overall signal value may be calculated. For the overall calculation, for example, the degree of deviation with respect to a healthy average value of 100 may be quantified for diseased states. Thereby, normality and abnormality may be distinct, providing a useful index for disease. Such quantification may enable the stage to be identified for each region. For example, if the value in the apex only moves by 0.5 in a normal case, a coefficient of 0.5 may be applied for multiplication to diseased values for that region. In other words, when values may be obtained for multiple regions across the lung field, a reference region is selected and other regions are calculated by ratio. In this process, the normal value serves as the coefficient to compute the abnormal value of each region.

Meanwhile, the posterior side of the heart (toward the back) is not represented in pixel values; therefore, the approximate lung volume excluding the heart may be estimated from, for example, the right lung signal and then multiplied by a coefficient. More specifically, applying a coefficient of approximately 60% to the lung volume may be used to estimate the motion behind the heart.

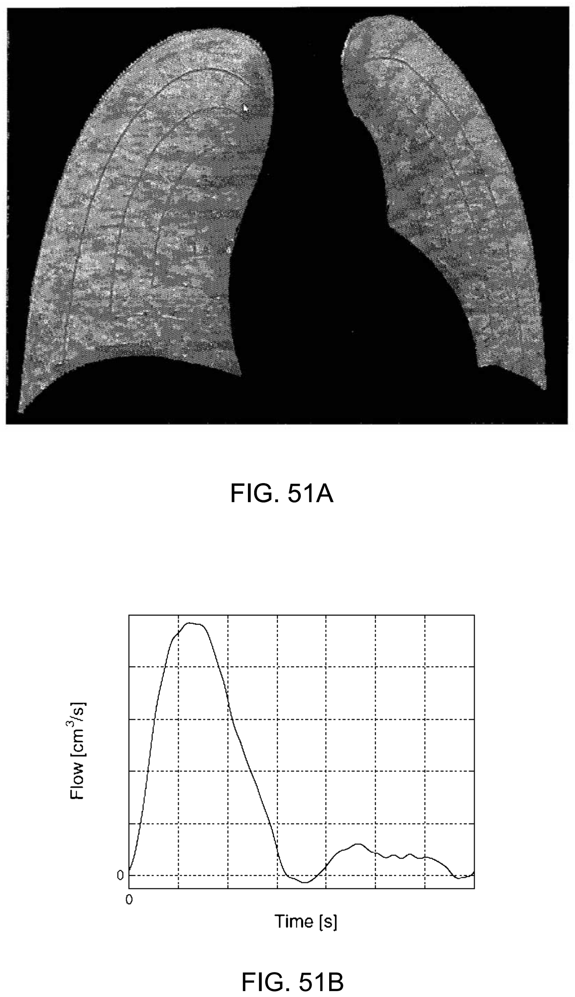

Pulmonary blood flow may be processed in the same way as the lung. In this case, since the signal variation in the pulmonary hilum is small, the signal value at the hilum is preferably used as the reference. In other words, regions with low signal are used as reference. FIG. 51A is a diagram showing a state in which a region of pulmonary blood flow is divided. As shown in FIG. 51A, the lungs are divided into fan-shaped regions centered on the pulmonary hilum. Then, the signal values for the inner layer, the middle layer, and the outer layer (approximately) are measured.

FIG. 51B is a diagram showing an outline of a waveform of the signal value of the pulmonary blood flow. In each divided region, the highest signal value within the waveform is tracked. Thereby, the state of the "branches of the pulmonary vessels" is known. Further, the average value of each divided region is tracked. Thereby, the state of the "spaces between the branches of the pulmonary vessels" is observed. Here, the apical region (where flow is minimal) and the outer region are used as denominators, and the signal values of each region are used as numerators for quantification.

By knowing how much the signal value in each divided region differs from its surrounding regions and how much it decreases compared to a healthy individual, presence or absence of disease may be predicted. For example, regions where signal values are missing in specific divided regions may indicate the possibility of pulmonary thromboembolism.

For example, in a case of pulmonary thromboembolism, vascular signal values do not appear in each divided region. Moreover, the signal values vary largely among the divided regions. Furthermore, in a case of PAH (pulmonary arterial hypertension), the lung are stiff; therefore, signal values of the pulmonary vessel branches may appear, whereas signal values indicating blood flow between branches do not. In other words, when represented by the average signal value for each divided region, the signal values are almost absent. In a case of heart failure, due to the inherently weak pumping power, the degree of increase in signal values is generally low. There may be no significant differences between divided regions, but the overall signal level may be lowered.

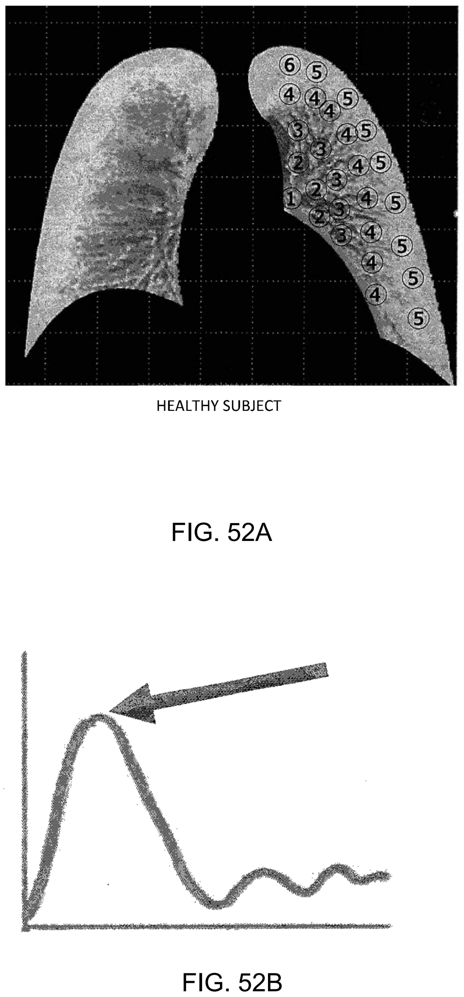

FIG. 52A is a diagram showing a state of pulmonary blood flow of a normal person (a healthy subject), and FIG. 52B is a diagram showing an outline of a waveform of a signal value of pulmonary blood flow. Here, the lung field is divided as shown in FIG. 51A, and the signal values of each region are detected. In FIG. 52A, the region where the signal is highest has the "signal value = 100," and a region without signal variation has the "signal value = 0," and each region displays a relative signal. In FIG. 52A, average signal values for the respective regions were calculated as follows:
(1) Central-side vessels (R1): around 90
(2) Peri-central region (R2): 70-80
(3) Intermediate level (R3): 40-50
(4) Peripheral level (R4): 20-30
(5) Outer layer (R5): 3-8 (Apical region: 3-5)

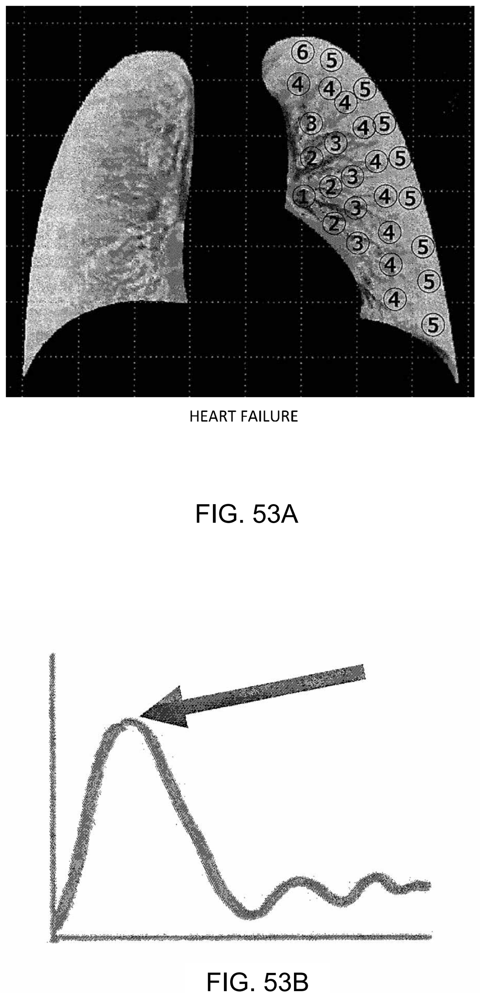

FIG. 53A is a diagram showing a state of pulmonary blood flow of a patient with heart failure, and FIG. 53B is a diagram showing an outline of a waveform of a signal value of pulmonary blood flow. Here, the lung field is divided as shown in FIG. 51A, and the signal values of each region are detected. In FIG. 53A, the region where the signal is highest has the "signal value = 100," and a region without signal variation has the "signal value = 0," and each region displays a relative signal. In FIG. 53A, average signal values for the respective regions were calculated as follows:
(1) Central-side vessels (R1): around 90
(2) Peri-central region (R2): 30-50
(3) Intermediate level (R3): 20-40
(4) Peripheral level (R4): 10-25
(5) Outer layer (R5): 3-8 (Apical region: 3-5)

Comparing the normal person (healthy individual) and the patient with heart failure reveals no significant differences in the central-side vessels (R1) and the outer layer (R5), but a large decrease is observed in the peri-central (R2), the intermediate level (R3), and the peripheral level (R4) in the heart failure patient with respect to the normal person (healthy individual). This indicates impaired blood flow dynamics in the patient with heart failure. As such, by dividing the lung field and quantifying each region, the lung motion may be effectively assessed.

Such signal value patterns may be analyzed more efficient by implementing an AI. The AI may be trained on case-based images to build case models and perform automated diagnosis.

The waveform processing method of the present invention may also be applied to stethoscopes. Conventionally, techniques for acquiring audio signals from a stethoscope and recording the signals in a database have been known. For example, a website identified by the following URL discloses a technique for visualizing and evaluating cardiac sound waveforms obtained from a stethoscope: (https://bigamart.com/product/3m-littmann-core-digital-stethoscope-8572-high-polish-rainbow-chestpiece-black-tube-stem-and-headset-69-cm/?gad=1&gelid=CjwKCAjw6eWnBhAKEiwADpnw9viM_3rqpgXoLW8PF_LB8D7Mj82r GOUd31ZF6AQiSbKHR6NT7hcFhxoCuSoQAvD_BwE). According to the present invention, Fourier transformation may be applied to audio waveforms obtained from a stethoscope. By extracting only the frequencies corresponding to respiration or blood flow from the transformed data and performing inverse Fourier transformation, a desired respiratory or blood flow waveform may be obtained. As a result, diseases may be classified based on the audio waveforms obtained from the stethoscope. Deep learning with an AI may also be applied to the audio waveforms after the inverse Fourier return to construct disease-specific models.

Generally, respiratory sounds may be classified into "normal sounds (breath sounds)" and "adventitious sounds (pathological noises)." In "normal sounds (breath sounds)," bronchial sounds, tracheal sounds, and vesicular breath sounds are detected. On the other hand, adventitious sounds exhibit phenomena such as attenuation, disappearance, prolongation, or bronchial sounding.

According to the present disclosure, respiratory element cycle-synchronized signals obtained from a stethoscope are further recognized as periodic inspiratory and expiratory phases. By capturing periodic features from these cyclic signals, characteristics of auscultation sounds may be classified based on auscultatory measurements. For example, when evaluating respiratory conditions excluding cardiac activity, not much information is given from multiple external factors, the phase remains constant within each repeated cycle, allowing nearly identical periodic signals to be obtained.

Specifically, each respiratory sound is classified into inspiratory and expiratory phases. At the beginning of inspiration and expiration, respiratory sounds begin to occur first, and at the end of inspiration and expiration, the sounds cease. Respiratory sounds tend to be slightly louder at the beginning of inspiration than at the beginning of expiration, enabling these rounds to be distinguished. Optionally, this distinction may be recognized using an AI. Optionally, inspiratory and expiratory phases may be recognized in synchronization with external or input signals. Based on these results, inspiratory and expiratory auscultation sounds are classified.

As a result of classifying each respiratory sound into inspiratory and expiratory phases, "normal sounds (breath sounds)" and "adventitious sounds (pathological noises)" are identified as described above. Meanwhile, the sounds may contain noise unrelated to respiration. Therefore, "frequency tunable data" processing described in the present disclosure is applied. Moreover, respiratory pathologies are identified as repeating noises at the same phase within the respiratory sounds; therefore, abnormalities may be detected by verifying whether the noise is of this repetitive type.

As examples of "adventitious sounds (pathological noises)," crackles are recognized in the early inspiratory phase, typically at 500-1,000 Hz and around 5 msec in duration, become more pronounced during deep inspiration, and are observed during inspiration (becoming stronger toward a latter half of inspiration). Moreover, bubbling sounds occur at 250-500 Hz with a duration of around 15 msec and are heard during an early or early-to-middle inspiratory phase as well as throughout inspiration, and may also be heard during expiration. Whistling sounds consist of prolonged sounds of 400 Hz or higher, polyphonic, and are mainly detected in a late expiratory phase. Snoring sounds occur at 200-250 Hz or lower and are heard during both inspiration and expiration. In conditions such as COPD and asthma, the relative duration of inspiratory and expiratory phases may repetitively vary.

As described above, by recognizing data matched to each cycle of "frequency tunable data" or respiration, and combining the "frequency tunable data" with conditions such as length of inspiration and expiration, ratio, signal intensity, scale and length of the so-called adventitious noise, and phase (beginning of inspiration, middle end of inspiration, end of expiration), abnormalities may be identified. Furthermore, by measuring the degree of these, whether the disease is worsened or recovering may be compared; and by objectively depicting and displaying measurement data using these numerical values, traditionally subjective auscultation-based diagnoses may be led to objective data-based recognition and diagnostic standard. As a result, diagnosis or progression of disease, or changes such as worsening or remission become recognizable.

Although respiratory sounds are associated with certain frequency bands, both "normal sounds (breath sounds)" and "adventitious sounds (pathological noises)" may also be recognized as characteristic waveforms outside those frequency bands. Accordingly, for "normal sounds" and "adventitious sounds," optionally, the characteristics of each adventitious sound may be computed using "wave form tunable data," by varying phase and signal intensity within a certain range, applying logarithmic transformation to the computed results, and recognizing the signal. Furthermore, by using the "wave form tunable data" as a basis to detect changes in phase, signal values (pixel values), and respiratory states, and by converting these into data, measurement data may be extracted objectively, and the results may be displayed to assist diagnosis.

In the presence of abnormalities, periodic changes such as variations in frequency, waveform, amplitude, signal values (pixel values), or phase duration may be measured, and by recognizing such abnormalities early, physicians, nurses, or caregivers may be alerted promptly as an abnormality arises. Accordingly, the abnormality may be treated or prevented in an early stage. In this case, in addition to the above-mentioned changes, calculations using "wavelet transformation" for frequency may also serve as a valid indicator. Moreover, the above-described information may be applied to deep learning, model construction, and automated diagnosis using an AI.

While how "frequency tunable data" in relation to respiration may be handled has been described, the present invention is not limited thereto and may similarly be applied to physiological data such as blood flow. That is, a similar approach may be applied to various physiological data, including electrocardiogram or electromyogram signals, electroencephalograms, and other periodically varying biological information (biological signal). Furthermore, this method may also be applied to dynamic images or other digital data that exhibit periodic repetition, not limited to biological data.

For example, measured electrocardiogram data may be analyzed based on the original data by classifying according to heartbeat or rhythm type centered on the strongest peak, i.e., the R-wave, comparing characteristics along different ECG directions, superimposing waveforms, and converting each detail into structured data for automated diagnosis. Moreover, such data analysis may have been feasible only when the subject is lying at rest without external stimuli; however, by applying the "frequency tunable data" of the present invention, it is not necessary for the subject to be in a resting state. Such data processing may similarly be applied to respiratory data.

Wristband-formed sensor watch may have only one sensor; therefore, detailed electrocardiogram (ECG) analysis may be difficult. However, by attaching an auxiliary wristband to the opposite arm or to one of the ankles, ECG waveforms suitable for clinical evaluation may be obtained. Even this alone makes it possible to grasp the condition of the patient and pathological changes to a certain extent.

In the present embodiment, based on the obtained respiratory-synchronized periodic signals, inspiratory and expiratory cycles are identified, and by capturing periodic features from the corresponding images again, auscultatory sound characteristics may be classified more suitably than by conventional auscultation measurements. When recognizing respiratory states excluding cardiac activity, no information such as external factors is involved, allowing repeated acquisition of similar signals with the same phase within each cycle. Specifically, respiratory sounds are classified into inspiratory and expiratory phases. Specifically, inspiration and expiration are classified. At the beginning of inspiration and expiration, breath sounds start to appear, while at the end of inspiration and expiration, the sounds cease. Inspiratory sounds tend to be slightly louder at their onset than expiratory sounds, allowing for recognition of differences between the two phases. This distinction may be recognized using an AI. Optionally, inspiratory and expiratory phases may be identified by linking to external or input signals. Based on these, inspiratory and expiratory auscultatory sounds may be classified.

As a result of such classification, "normal sounds (breath sounds)" and "adventitious sounds (pathological noises)" are identified. Meanwhile, the sounds may contain noise unrelated to respiration. Therefore, "frequency tunable data" processing described in the present disclosure is applied., or, by recognizing whether the noise is repetitive (respiratory pathologies are identified as repeating noises at the same phase within the respiratory sounds), abnormalities may be detected. As examples of "adventitious sounds (pathological noises)," crackles are recognized in the early inspiratory phase, typically at 500-1,000 Hz and around 5 msec in duration, become more pronounced during deep inspiration, and are observed during inspiration (becoming stronger toward a latter half of inspiration). Moreover, bubbling sounds occur at 250-500 Hz with a duration of around 15 msec and are heard during an early or early-to-middle inspiratory phase as well as throughout inspiration, and may also be heard during expiration. Whistling sounds consist of prolonged sounds of 400 Hz or higher, polyphonic, and are mainly detected in a late expiratory phase. Snoring sounds occur at 200-250 Hz or lower and are heard during both inspiration and expiration. Conditions such as COPD and asthma, where the relative duration of inspiratory and expiratory phases repetitively vary, may be recognized thereby.

As described above, by recognizing data matched to each cycle of "frequency tunable data" or respiration, and combining the "frequency tunable data" with conditions such as length of inspiration and expiration, ratio, signal intensity, scale and length of the so-called adventitious noise, and phase (beginning of inspiration, middle end of inspiration, end of expiration), abnormalities may be identified. Furthermore, by measuring the degree of these, whether the disease is worsened or recovering may be compared; and by objectively depicting and displaying measurement data using these numerical values, traditionally subjective auscultation-based diagnoses may be led to objective data-based recognition and diagnostic standard. As a result, diagnosis or progression of disease, or changes such as worsening or remission become recognizable. Although these sounds are associated with certain frequency bands, disease-related or auscultatory sounds may also be recognized as characteristic waveforms outside those bands. For this purpose, using "wave form tunable data," the characteristics of each adventitious sound may be computed by varying phase or signal to a certain degree, and the data may be extracted through logarithmic transformation to recognize the signals.

Furthermore, by using the "wave form tunable data" as a basis to detect changes in phase, signal values (pixel values), and respiratory states, and by converting these into data, measurement data may be extracted objectively, and the results may be displayed to assist diagnosis. In the presence of abnormalities, periodic changes such as variations in frequency, waveform, amplitude, signal values (pixel values), or phase duration may be measured, and by recognizing such abnormalities early, physicians, nurses, or caregivers may be alerted promptly as an abnormality arises. Accordingly, the abnormality may be treated or prevented in an early stage. In this case, in addition to the above-mentioned changes, calculations using "wavelet transformation" for frequency may also serve as a valid indicator. Optionally, the above processing may be performed using an AI.

In the present embodiment, to distinguish between heart sounds, breath sounds, and other biological sounds such as intestinal sounds, frequencies specific to living body are filtered in advance to separate the respiratory and cardiac sounds. By processing with filters in advance for heart sounds centered around 0.7-2 Hz and for respiratory sounds in the 0.15-0.4 Hz range, "frequency tunable data" may be more easily obtained. Moreover, the waveforms of repeated respiration or heartbeats may be used to calculate waveform consistency and waveform synchronization, which may then be used to determine respiratory or blood flow frequencies or waveforms in advance.

For example, heart sounds may be processed as follows:
(1) A computer identifies the first heart sound S1 and second heart sound S2 and recognizes their relationship. The first heart sound S1 and the second heart sound S2 are the two heart sounds that may be heard by placing ear against chest. Specifically, in the characteristic "lub-dub" sound of heartbeat, "lub" corresponds to S1, and "dub" corresponds to S2, which includes the aortic (IIA) and pulmonary (IIP) valve components occurring in quick succession. Although the third heart sound S3 or the fourth heat sound S4 may sometimes be heard between the second heart sound S2 and the first heart sound S1, in normal heart sounds these are typically not heard. The first heart sound S1 tends to have a larger amplitude and lower frequency, whereas the second heart sound S2 tends to be smaller and higher in frequency than the first heart sound S1. If the first heart sound S1 and the second heart sound S2 overlap with abnormal sounds, the position of the largest sound may be treated as S1 for overall evaluation.
(2) Systolic murmurs following the first heart sound S1 and diastolic murmurs following the second heart sound S2 are detected. After identifying the first heart sound S1 and the second heart sound S2, the waveform patterns are computed based on their phase and the corresponding waveform and frequency components. The computer evaluates the degree of waveform and frequency matching during heart sound acquisition. By capturing and categorizing frequency components that occur at specific phases (high frequency, low frequency), those components may be correlated with pathological findings.

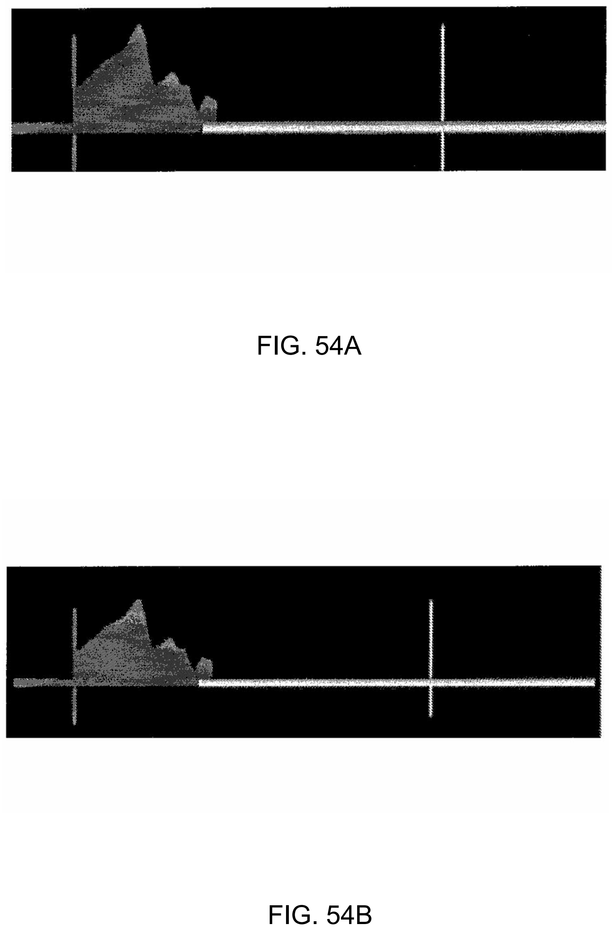

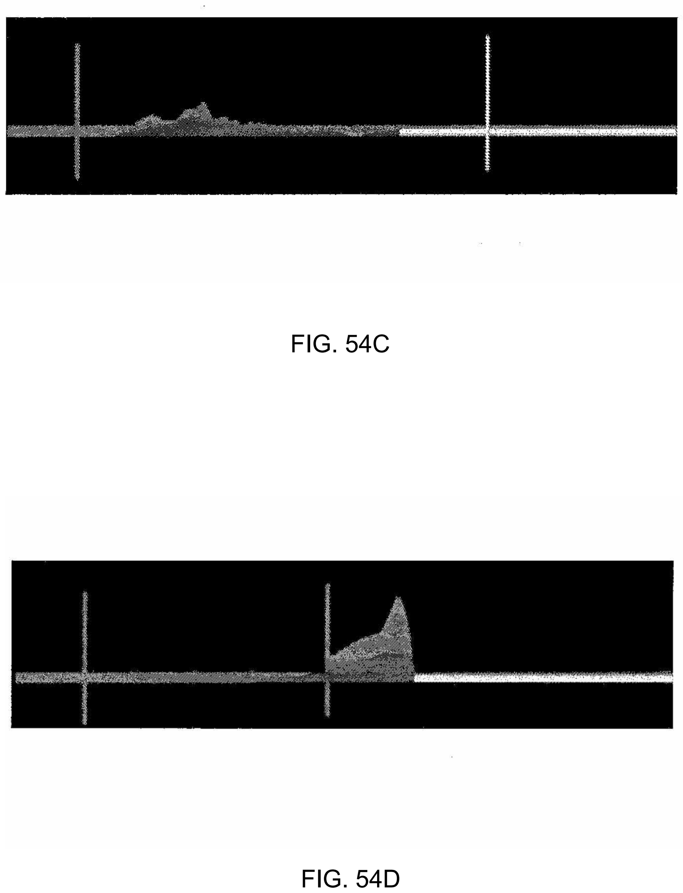

Specifically, for example, as shown in FIG. 54A, in aortic stenosis, a strong ejection sound appears simultaneously with or immediately after the first heart sound S1, and a relatively high-frequency sound range occurs. Moreover, for example, in mitral regurgitation, not only ejection murmurs but also regurgitant murmurs occur from immediately after the first heart sound S1 up to just before the second heart sound S2, resulting in mixed noise that persists over a long interval between the first heart sound S1 and the second heart sound S2. Compared with the murmur immediately following the first heart sound S1, the subsequent sound tends to have lower intensity and lower frequency. FIG. 54B illustrates the early ejection period immediately following the first heart sound S1, and FIG. 54C illustrates the late ejection period after the first heart sound S1.

Moreover, as shown in FIG. 54D, in aortic regurgitation, a sound including relatively high frequencies is heard immediately after the second heart sound S2, whereas the sound is not heard as an abnormal sound in the late S2 phase. For another example, in mitral regurgitation, the sound becomes louder from immediately after the second heart sound S2 to mid-phase, then weaker in the late phase, and a relatively low frequency noise is heard. As such, by recognizing ejection murmurs and regurgitant murmurs associated with the first heart sound S1, room-number valvular murmurs and regurgitant murmurs associated with the second heart sound S2, and their degrees relative to the baseline of the first heart sound S1 or the second heart sound S2, and by analyzing frequency and phase-frequency waveforms, diseases may be classified and their variations identified. Thus, the heart sound murmurs may be recognized. These classifications may be computed analytically, or optionally, learned by AI-based recognition.

Furthermore, the normal second heart sound S2 consists of an aortic component (IIA) and a pulmonary component (IIP). By analyzing variations in the closure timing of these two valve components, patterns relative to rhythm and the first heart sound S1, as well as overall delays, may be grasped, even by focusing solely on the second heart sound S2. Specifically, during inspiration, the normal interval between IIA and IIP is approximately 0.02 seconds, but in cases of atrial septal defect, this interval widens to around 0.05 seconds, and in right bundle branch block or pulmonary stenosis, it may widen to around 0.12 seconds. In left bundle branch block or aortic stenosis, the normal timing is reversed or altered: the IIa sound, which normally precedes, may occur after IIp or overlap IIp. As such, changes in the second heart sound S2 may also be analyzed computationally, enabling calculation and display of heart sound classifications. Accordingly, detection of abnormalities, monitoring of disease progression or sudden reduction over various time intervals (minutes, hours, days, or months) become possible, and classification of data such as phase timing, duration, intensity, frequency, and waveform characteristics becomes possible, allowing for detailed understanding of pathological conditions. Optionally, an AI may be trained to learn these.

### [Blood Flow-Related Peak Projection (BPP)]

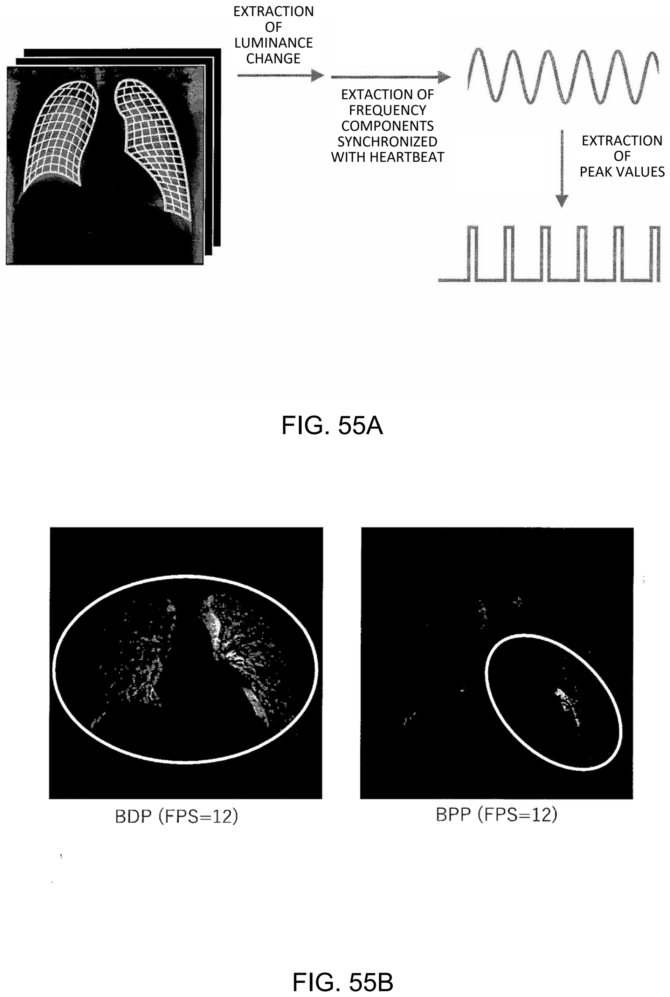

FIG. 55A is a diagram showing an outline of BPP (Blood flow-related Peak Projection). According to the present disclosure, as shown in FIG. 55A, when luminance variations are extracted from an image (here, a lung image), and frequency components synchronized with heartbeats are further extracted, the resulting signal exhibits a sinusoidal waveform, which has the property of expressing states of increase or decrease. Due to this property, images with a characteristic "fading away" appearance in peripheral regions may be generated, providing good correlation with contrast-enhanced CT.

On the other hand, because the signal intensity in the peripheral regions of the lung tends to be lower, the signal is easily buried in noise, making it difficult to determine whether blood flow has propagated all the way to the lung periphery. For example, the "Forrester classification," which is used for assessing the severity of heart failure, employs the value of pulmonary capillary wedge pressure (PCWP). In cases of heart failure, blood flow stagnates, causing an increase in pressure, and as a result, the propagation velocity of blood is expected to decrease.

In the present invention, blood propagation velocity is considered to be represented as signal propagation velocity. To focus on signal propagation velocity as an expression of signal increase and decrease, it is necessary to remove "mixed noise." To achieve this, an image is generated by mapping only peak values (maximum amplitude values). This image enables low-noise representation of the propagation of peak values, i.e., the manner in which blood propagates.

Moreover, when attempting Al-based diagnosis, excessive information in the image may hinder effective machine learning. Therefore, this approach is also valuable as a method for reducing the amount of information by one stage.

Accordingly, the present disclosure proposes "BPP (Blood flow-related Peak Projection)." FIG. 55B shows, on the left, an image of BDP (Blood flow-related Differential Projection) and, on the right, an image of BPP (Blood flow-related Peak Projection). In the area enclosed by a white circle on the left side of FIG. 55B, an image based on signals represented by BDP is shown. On the other hand, in the area enclosed by a white circle on the right side, a sharp dynamic image focusing only on peak values was successfully displayed using BPP.

### [Application Example 1]

According to the present disclosure, based on the generated dynamic images, two or more images representing respiratory phases such as inspiration and expiration, i.e., a smaller number of images than usual, may be used to calculate their differences and to analyze correlations with AI models or the original data. This allows lung function and blood flow function to be computed using a smaller number of images. This method may also be applied to deformative movement of contours, ribs, and thoracic cage, and diaphragmatic movement.

### [Application Example 2]

Based on the images processed according to the present invention, estimating "Swan-Ganz catheter measurement values" using an Al may be considered. As described above, by applying "supervised learning" to images processed by this invention, an AI model may be constructed to estimate "Swan-Ganz catheter measurement values." Here, the inventors of the present invention have found that by training an AI to learn "DICOM video" obtained from fluoroscopy equipment, "Swan-Ganz catheter measurement values" are accurately estimated.

In Application Example 2, when capturing X-ray cine images using an X-ray fluoroscopic apparatus or an angiographic apparatus, the images should be captured under the conditions applied in the present invention, rather than under those typically used in clinical practice. More specifically, no image filters such as a gamma filter are used. Furthermore, the X-ray exposure dose is fixed while images are being captured. Normally, while a subject is breathing, X-ray transmissivity varies depending on the expansion and contraction of the lungs. Therefore, in standard practice, the X-ray exposure dose is adjusted in accordance with the degree of transmissivity. However, in Application Example 2, by fixing the X-ray exposure dose, subtle image variations of the lungs and blood flow may be captured. Optionally, in Application Example 2, the DICOM video itself obtained by the fluoroscopic equipment may be used.

The outline of the operation in Application Example 2 is as follows. Specifically, the input image is a "medical chest X-ray image" or a "series of medical chest X-ray images." The AI-based decision algorithm is set as a "convolutional neural network (CNN: Convolutional Neural Network)."

For generating the AI model, a transfer learning method (for example, VGG-16) may be used. In this transfer learning, the initial layers of the CNN adopt a pre-trained model with pre-determined parameters. In the later layers, new layers are added, and their parameters are learned and determined. Moreover, because this method may not achieve sufficient accuracy, a fine-tuning technique is applied. That is, "updating parameter" is applied to the "pre-trained model" to improve accuracy. In transfer learning based on VGG-16, the "classification layer following the convolution layers," which learns shapes, is replaced with a "regression layer." For sequential images, an "RNN layer" for handling time-series data is placed after the "convolution layers, followed by a regression layer. The output of Application Example 2 is the "Swan-Ganz catheter measurement value (predicted value), obtained from the predicted numerical value output of the regression layer." This output may be derived from either "simple regression" or "multiple regression."

In this Application Example 2, it was confirmed that the correlation between the "Swan-Ganz catheter measurement values" obtained using conventional methods and the measurement values (estimated values) obtained by the AI was high. As a result, by using an AI model, Swan-Ganz catheter measurement values may be predicted from DICOM cine images obtained under appropriate conditions, enabling non-invasive acquisition of diagnostic results.

### [Application Example 3]

In the blood flow analysis images (BDP) of the present invention, "embolisms" may be visualized. "Embolisms" may be broadly categorized into two types: "occlusion" and "stenosis." Each type appears differently in the BDP (Blood flow-related Differential Projection) images. In "occlusion," a "wedge-shaped void" appears in the signal propagation pattern of the image. In contrast, in "stenosis", because the blood flow slows down, a "phase shift (relative to surrounding regions)" appears in the signal propagation pattern.

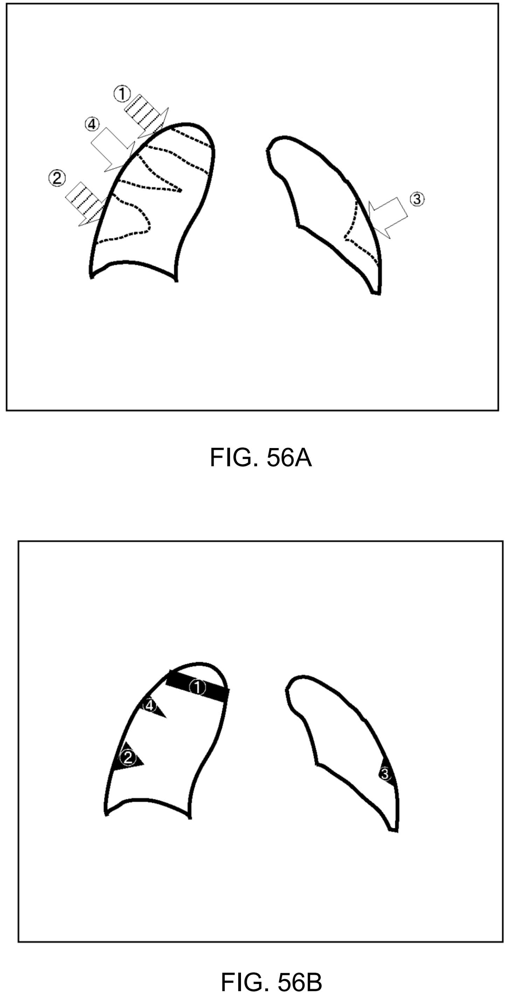

Based on these findings, the inventors have conceived a method for displaying the characteristic images of these "embolisms" in a single image. First, "occlusion (obstruction)," is visualized using MBDP (Maximum Blood-flow-related Differential Projection). FIG. 56A shows a lung image in which the areas labeled (1) and (2) are considered to be defect regions. The areas labeled (3) and (4) are suspected defect regions. FIG. 56B shows an example in which these defect areas are explicitly indicated. As shown in FIG. 56B, the sites where "occlusion (obstruction)" is predictably occurring may be clearly indicated, for example, by displaying them using hollow rectangles or wedge shapes.

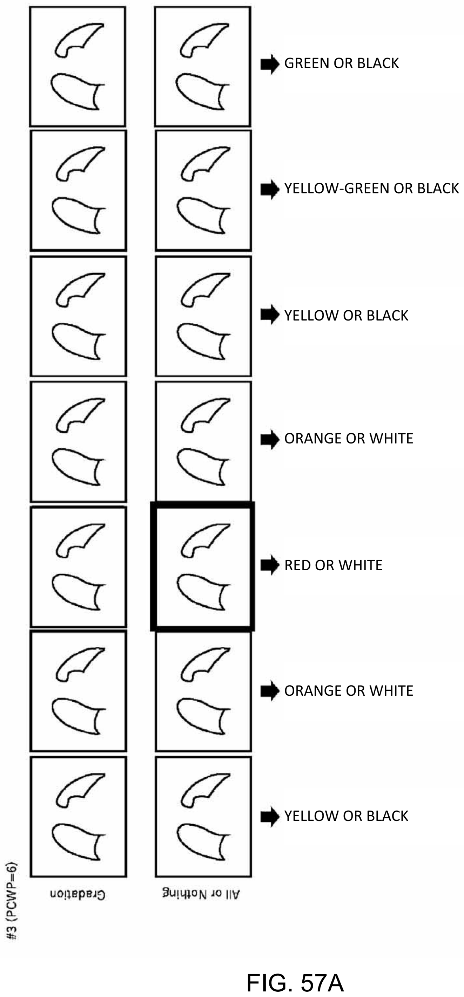

On the other hand, "stenosis (coarctation)" is visualized using BPTP (Blood flow-related Peak Timing Projection). That is, the regions where blood flow timing deviates occur are visualized. FIG. 57A is a diagram schematically showing an outline of BPTP. Here, a reference frame (for example, the frame enclosed by a black rectangle in FIG. 57A) is selected, and the degree of deviation of the peak pixel values from this reference frame is displayed using selected colors (the drawing is in monochrome). The reference frame may be defined as the "frame containing the largest number of pixels with peak values."

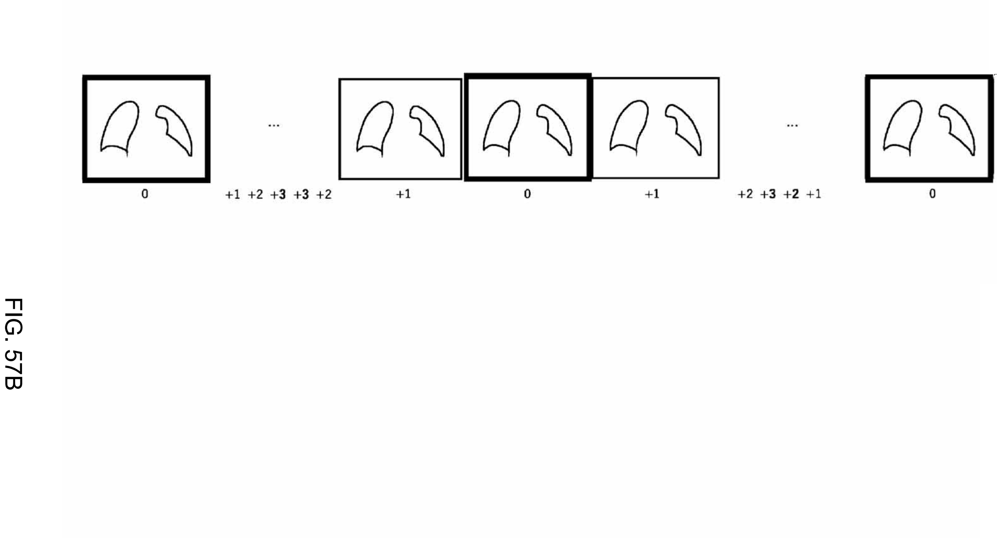

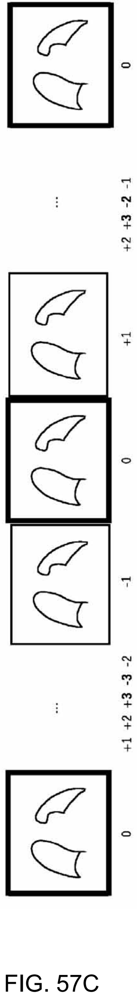

FIGS. 57B and 57C are diagrams schematically showing mapping examples of BPTP. FIG. 57B shows an example where deviation from the reference frame is expressed in absolute values. In this example, "+1" and "-1" may be distinguished, but the direction of the deviation is inexpressible. FIG. 57C shows an example in which positive and negative concepts are provided to groups. In this case, while "+1" and "-1" may be distinguished, when mapped with positive and negative values, large changes in index values may occur between adjacent frames, such as from "-3" to "+3." Therefore, by using a color set such as "-100 red - 0 white - +100 red," the visualization may express absolute values while preserving the "directionality of deviation" of the actual pixel values. Furthermore, by adopting a color set such as "-100 black - blue - 0 white - red - +100 black," displaying images that match the theory and that change at the same timing in the same color tones is possible.

Based on the above considerations, the inventors adopted a method of assigning positive and negative concepts to groups in the BPTP mapping, by which images are displayed preferably. Specifically, the reference frame is displayed in white, and as the deviation from the reference frame increases, colors are assigned in the order of "white - yellow - red - black," thereby enhancing the visibility of the deviated regions. Furthermore, while the reference frame is displayed in white, "white - yellow - red" is assigned to regions whose timing is delayed relative to the reference frame (displayed in white), and "white - cyan - blue - black" is assigned to regions whose timing is earlier than the reference frame, thereby enhancing the visibility of deviated regions.

As described above, according to the present embodiment, the overall respiratory state and vascular dynamics of the subject human body may be grasped, and images showing actual movements may be displayed based on the waveform or frequency, or waveform and frequency, of the respiration, heart, blood vessels or blood flow in the hilum, or based on the tendency of changes in the images.

### DESCRIPTION OF REFERENCE NUMERALS

- 1:: Basic module
- 3:: Respiratory function analyzing unit
- 5:: Pulmonary blood flow analyzing unit
- 7:: Other blood flow analyzing unit
- 9:: Fourier analyzing unit
- 10:: Waveform analyzing unit
- 11:: Visualization/quantification unit
- 13:: Input interface
- 15:: Database
- 17:: Output interface
- 19:: Display

## Claims

1. A diagnosis assistance program for analyzing an image of a living body and displaying an analysis result, the program being configured to cause a computer to execute:
a process for acquiring a plurality of frame images;
a process for acquiring a periodic image signal that varies at a predetermined cycle from the plurality of frame images;
a process for extracting all or a part of a waveform of the periodic image signal; and
a process for matching the extracted waveform with a model waveform acquired in advance and detecting a correlation therebetween.

2. The diagnosis assistance program according to claim 1, further including a process for complementing the periodic image signal, the periodic image signal being discrete, and forming a continuous waveform.

3. The diagnosis assistance program according to claim 1, wherein the correlation is detected based on at least one of a phase, an amplitude, or a frequency of all or a part of the waveform of the periodic image signal.

4. The diagnosis assistance program according to claim 1, configured to cause the computer to execute a process for visualizing the extracted waveform based on the detected correlation.

5. A diagnosis assistance program for analyzing an image of a living body and displaying an analysis result, the program being configured to cause a computer to execute:
a process for acquiring a plurality of frame images;
a process for acquiring a periodic image signal that varies at a predetermined cycle from the plurality of frame images;
a process for extracting all or a part of a frequency of the periodic image signal;
a process for matching the extracted frequency with a model frequency acquired in advance and detecting a correlation therebetween.

6. The diagnosis assistance program according to claim 5, further including a process for identifying a waveform based on a phase, an amplitude, and the frequency of all or a part of the periodic image signal.

7. The diagnosis assistance program according to claim 6, configured to cause the computer to execute a process for visualizing the identified waveform.

8. A diagnosis assistance program for analyzing an image of a living body and displaying an analysis result, the program being configured to cause a computer to execute:
a process for acquiring a plurality of frame images;
a process for acquiring a periodic image signal that varies at a predetermined cycle from the plurality of frame images; and
a process for creating a model by training an AI (Artificial Intelligence) to learn all or a part of the periodic image signal.

9. The diagnosis assistance program according to claim 8, further comprising a process for detecting a correlation between the model and the periodic image signal acquired from the plurality of frame images.

10. The diagnosis assistance program according to claim 1, further including a process for extracting a signal having a maximum amplitude among the extracted waveform,
wherein the signal having the maximum amplitude is visualized based on the detected correlation.

11. The diagnosis assistance program according to claim 1 or claim 5, further including a process for applying logarithmic transformation to the periodic image signal acquired from the plurality of frame images,
wherein each of the processes is executed with the periodic image signal after the logarithmic transformation.

12. The diagnosis assistance program according to claim 1 or claim 5, further including:
a process for applying Fourier transformation to the periodic image signal;
a process for extracting a signal having the correlation from the signal after the Fourier transformation; and
a process for applying inverse Fourier transformation to the extracted signal.

13. The diagnosis assistance program according to claim 1, further including a process for interpolating the waveform using a waveform representing a movement of a standard model created by training an AI (Artificial Intelligence) to learn an image of a standard organ.

14. The diagnosis assistance program according to claim 5, further including a process, using a frequency of a movement of a standard model created by training an AI (Artificial Intelligence) to learn an image of a standard organ, for extracting pixels, of which pixel values vary over time according to the frequency of the movement of the standard model.

15. A diagnosis assistance program for analyzing an image of a living body and displaying an analysis result, the program being configured to cause a computer to execute:
a process for acquiring a video composed of a plurality of frame images;
a process for creating a model by training an AI (Artificial Intelligence) to learn the video; and
a process for predicting a measurement value for the living body using the model.

16. A diagnosis assistance program for analyzing an image of a living body and displaying an analysis result, the program being configured to cause a computer to execute:
a process for acquiring a video composed of a plurality of frame images;
a process for acquiring a periodic image signal that varies at a predetermined cycle from the video;
a process for creating a model by training an AI (Artificial Intelligence) to learn the periodic image signal; and
a process for predicting a phenomenon occurring in the living body using the model.

17. A diagnosis assistance system configured to analyze an image of a living body and display an analysis result, comprising:
an input interface configured to acquire a plurality of frame images; and
a basic module configured to:
acquire a periodic signal image that varies at a predetermined cycle from the plurality of frame images;
extract all or a part of a waveform of the periodic image signal; and
match the extracted waveform with a model waveform acquired in advance and detect a correlation therebetween.
